# FASCICULE DE BREVET EUROPEEN

(11) **EP 1 658 373 B1**
(45) Date de publication et mention de la délivrance du brevet: **25.11.2009**
(21) Numéro de dépôt: 04786383.2
(22) Date de dépôt: 19.08.2004
(51) Int. Cl.: C12N 15/85, C12N 15/62, C12N 1/21, C07K 14/18, C12P 21/02, A61K 38/16

(54) **VECTEUR DE CO-EXPRESSION DE DOMAINES MEMBRANAIRES DE PROTEINES D'ENVELOPPE D'UN VIRUS ET UTILISATIONS**
KOEXPRESSIONSVEKTOR FÜR DIE MEMBRANDOMÄNEN VON VIRUSHÜLLPROTEINEN UND VERWENDUNGEN DAVON
CO-EXPRESSION VECTOR FOR THE MEMBRANE DOMAINS OF VIRAL ENVELOPE PROTEINS AND USES THEREOF

(30) Priorité: 28.08.2003 FR 0350470
(43) Date de publication de la demande: 24.05.2006
(73) Titulaire: CENTRE NATIONAL DE LA RECHERCHE SCIENTIFIQUE (CNRS), 75794 Paris Cedex 16 (FR); COMMISSARIAT A L'ENERGIE ATOMIQUE, 75015 Paris (FR)
(72) Inventeur: FALSON, Pierre, F-69110 Sainte Foy les Lyon (FR); MONTIGNY, Cédric, F-91190 Gif sur Yvette (FR); PENIN, François, F-69150 Decines (FR)
(74) Mandataire: Poulin, Gérard
(86) Numéro de dépôt international: PCT/FR2004/050385
(87) Numéro de publication internationale: WO 2005/024031

(56) Documents cités:
- EP-A- 1 304 381
- WO-A-03/051912
- WO-A-2004/027068
- MONTIGNY CEDRIC ET AL: "Overcoming the toxicity of membrane peptide expression in bacteria by upstream insertion of Asp-Pro sequence." BIOCHIMICA ET BIOPHYSICA ACTA, vol. 1660, no. 1-2, 28 janvier 2004 (2004-01-28), pages 53-65, XP004486901 ISSN: 0006-3002 (ISSN print)
- DE BEECK ANNE OP ET AL: "The transmembrane domains of hepatitis C virus envelope glycoproteins E1 and E2 play a major role in heterodimerization" JOURNAL OF BIOLOGICAL CHEMISTRY, vol. 275, no. 40, 6 octobre 2000 (2000-10-06), pages 31428-31437, XP002280044 ISSN: 0021-9258 cité dans la demande
- COCQUEREL L ET AL: "Charged residues in the transmembrane domains of hepatitis C virus glycoproteins play a major role in the processing, subcellular localization, and assembly of these envelope proteins." JOURNAL OF VIROLOGY. UNITED STATES APR 2000, vol. 74, no. 8, avril 2000 (2000-04), pages 3623-3633, XP002234017 ISSN: 0022-538X
- WANG Y ET AL: "A UNIQUE APPROACH FOR HIGH LEVEL EXPRESSION AND PRODUCTION OF A RECOMBINANT COBRA NEUROTOXIN IN ESCHERICHIA COLI" JOURNAL OF BIOTECHNOLOGY, ELSEVIER SCIENCE PUBLISHERS B.V., AMSTERDAM, NL, vol. 94, no. 3, 2002, pages 235-244, XP001159705 ISSN: 0168-1656
- LORENZ IVO C ET AL: "Folding and dimerization of tick-borne encephalitis virus envelope proteins prM and E in the endoplasmic reticulum" JOURNAL OF VIROLOGY, vol. 76, no. 11, juin 2002 (2002-06), pages 5480-5491, XP002282501 ISSN: 0022-538X cité dans la demande

### LISTE DE SEQUENCES

<110> CENTRE NATIONAL DE LA RECHERCHE SCIENTIFIQUE et COMMISSARIAT A L'ENERGIE ATOMIQUE
<120> VECTEUR DE CO-EXPRESSION DE DOMAINES MEMBRANAIRES DE PROTEINES D'ENVELOPPE D'UN VIRUS ET UTILISATIONS
<130> B 14336 EE
<140>
   <141>
<160> 81
<170> PatentIn Ver. 2.1
<210> 1
   <211> 111
   <212> ADN
   <213> Hepatitis C virus
<220>
   <223> ADN codant pour la protéine TME1
<400> 1
   atgatcgctg gtgctcactg gggtgttctg gctggtatcg cttacttctc tatggttggt 60
   aactgggcta aagttctggt tgttctgctg ctgttcgctg gtgttgacgc t          111
<210> 2
   <211> 37
   <212> PRT
   <213> Hepatitis C virus
<220>
   <223> protéine TME1
<400> 2
   Met Ile Ala Gly Ala His Trp Gly Val Leu Ala Gly Ile Ala Tyr Phe
     1               5                  10                  15
   Ser Met Val Gly Asn Trp Ala Lys Val Leu Val Val Leu Leu Leu Phe
                20                  25                  30
   Ala Gly Val Asp Ala
            35
<210> 3
   <211> 42
   <212> ADN
   <213> Séquence artificielle
<220>
   <223> Description de la séquence artificielle: oligonucléotide (+) pour générer la mutation TME1_G354L
<220>
<400> 3
   gtaagcgata ccagccagaa ccagccagtg agcaccagcg at 42
<210> 4
   <211> 42
   <212> ADN
   <213> Séquence artificielle
<220>
   <223> Description de la séquence artificielle: oligonucléotide (-) pour générer la mutation TME1_G354L
<400> 4
   atcgctggtg ctcactggct ggttctggct ggtatcgctt ac 42
<210> 5
   <211> 43
   <212> ADN
   <213> Séquence artificielle
<220>
   <223> Description de la séquence artificielle: oligonucléotide (+) pour générer la mutation TME1_G358L
<400> 5
   caaccataga gaagtaagcg atcagagcca gaacacccca gtg 43
<210> 6
   <211> 43
   <212> ADN
   <213> Séquence artificielle
<220>
   <223> Description de la séquence artificielle: oligonucléotide (-) pour générer la mutation TME1_G358L
<400> 6
   cactggggtg ttctggctct gatcgcttac ttctctatgg ttg 43
<210> 7
   <211> 43
   <212> ADN
   <213> Séquence artificielle
<220>
   <223> Description de la séquence artificielle: oligonucléotide (+) pour générer la mutation TME1_G354&358L
<400> 7
   caaccataga gaagtaagcg atcagagcca gaaccagcca gtg 43
<210> 8
   <211> 43
   <212> ADN
   <213> Séquence artificielle
<220>
   <223> Description de la séquence artificielle: oligonucléotide (-) pour générer la mutation TME1_G354&358L
<400> 8
   cactggctgg ttctggctct gatcgcttac ttctctatgg ttg 43
<210> 9
   <211> 111
   <212> ADN
   <213> Séquence artificielle
<220>
   <223> Description de la séquence artificielle: ADN codant pour TME1_G354L
<400> 9
   atgatcgctg gtgctcactg gctggttctg gctggtatcg cttacttctc tatggttggt 60
   aactgggcta aagttctggt tgttctgctg ctgttcgctg gtgttgacgc t          111
<210> 10
   <211> 37
   <212> PRT
   <213> Séquence artificielle
<220>
   <223> Description de la séquence artificielle: protéine TME1_G354L
<400> 10
   Met Ile Ala Gly Ala His Trp Leu Val Leu Ala Gly Ile Ala Tyr Phe
   1               5                   10                  15
   Ser Met Val Gly Asn Trp Ala Lys Val Leu Val Val Leu Leu Leu Phe
               20                  25                  30
   Ala Gly Val Asp Ala
           35
<210> 11
   <211> 111
   <212> ADN
   <213> Séquence artificielle
<220>
   <223> Description de la séquence artificielle: ADN codant pour TME1_G358L
<400> 11
   atgatcgctg gtgctcactg gggtgttctg gctctgatcg cttacttctc tatggttggt 60
   aactgggcta aagttctggt tgttctgctg ctgttcgctg gtgttgacgc t          111
<210> 12
   <211> 37
   <212> PRT
   <213> Séquence artificielle
<220>
   <223> Description de la séquence artificielle: protéine TME1_G358L
<400> 12
   Met Ile Ala Gly Ala His Trp Gly Val Leu Ala Leu Ile Ala Tyr Phe
    1               5                  10                  15
   Ser Met Val Gly Asn Trp Ala Lys Val Leu Val Val Leu Leu Leu Phe
               20                  25                  30
   Ala Gly Val Asp Ala
           35
<210> 13
   <211> 111
   <212> ADN
   <213> Séquence artificielle
<220>
   <223> Description de la séquence artificielle: ADN codant pour TME1_G354&G358L
<400> 13
   atgatcgctg gtgctcactg gctggttctg gctctgatcg cttacttctc tatggttggt 60
   aactgggcta aagttctggt tgttctgctg ctgttcgctg gtgttgacgc t          111
<210> 14
   <211> 37
   <212> PRT
   <213> Séquence artificielle
<220>
   <223> Description de la séquence artificielle: protéine TME1_G354&G358L
<400> 14
   Met Ile Ala Gly Ala His Trp Leu Val Leu Ala Leu Ile Ala Tyr Phe
    1               5                  10                  15
   Ser Met Val Gly Asn Trp Ala Lys Val Leu Val Val Leu Leu Leu Phe
               20                  25                  30
   Ala Gly Val Asp Ala
           35
<210> 15
   <211> 90
   <212> ADN
   <213> Hepatitis C virus
<220>
   <223> ADN codant pour TME2
<400> 15
   gaatacgttg ttctgctgtt cctgctgctg gctgacgctc gtgtttgctc ttgcctgtgg 60
   atgatgctgc tgatctctca ggctgaagct                                  90
<210> 16
   <211> 30
   <212> PRT
   <213> Hepatitis C virus
<220>
   <223> protéine TME2
<400> 16
   Glu Tyr Val Val Leu Leu Phe Leu Leu Leu Ala Asp Ala Arg Val Cys
    1               5                  10                  15
   Ser Cys Leu Trp Met Met Leu Leu Ile Ser Gln Ala Glu Ala
               20                  25                  30
<210> 17
   <211> 90
   <212> ADN
   <213> Séquence artificielle
<220>
   <223> Description de la séquence artificielle: ADN codant pour TME2_C731&733A
<400> 17
   gaatacgttg ttctgctgtt cctgctgctg gctgacgctc gtgttgcttc tgctctgtgg 60
   atgatgctgc tgatctctca ggctgaagct                                  90
<210> 18
   <211> 66
   <212> ADN
   <213> Séquence artificielle
<220>
   <223> Description de la séquence artificielle: oligonucléotide (+) pour création du fragment TME2_C731&733A
<400> 18
   aaaggatccg acccggaata cgttgttctg ctgttcctgc tgctggctga cgctcgtgtt 60
   gcttct                                                            66
<210> 19
   <211> 69
   <212> ADN
   <213> Séquence artificielle
<220>
   <223> Description de la séquence artificielle: oligonucléotide (-) pour création du fragment TME2_C731&733A
<400> 19
   gggaattcct aagcttcagc ctgagagatc agcagcatca tccacagagc agaagcaaca 60
   cgagcgtca                                                         69
<210> 20
   <211> 18
   <212> ADN
   <213> Séquence artificielle
<220>
   <223> Description de la séquence artificielle: oligonucléotide (+) pour amplification du fragment TME2_C731&733A
<400> 20
   aaaggatccg acccggaa 18
<210> 21
   <211> 21
   <212> ADN
   <213> Séquence artificielle
<220>
   <223> Description de la séquence artificielle: oligonucléotide (-) pour amplification du fragment TME2_C731&733A
<400> 21
   gggaattcct aagcttcagc c 21
<210> 22
   <211> 30
   <212> PRT
   <213> Séquence artificielle
<220>
   <223> Description de la séquence artificielle: protéine TME2_C731&733A
<400> 22
   Glu Tyr Val Val Leu Leu Phe Leu Leu Leu Ala Asp Ala Arg Val Ala
    1               5                  10                  15
   Ser Ala Leu Trp Met Met Leu Leu Ile Ser Gln Ala Glu Ala
               20                  25                  30
<210> 23
   <211> 4969
   <212> ADN
   <213> Séquence artificielle
<220>
   <223> Description de la séquence artificielle: plasmide d'expression pGEXKT
<400> 23
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccccgg 960
   gaattcatcg tgactgactg acgatctgcc tcgcgcgttt cggtgatgac ggtgaaaacc 1020
   tctgacacat gcagctcccg gagacggtca cagcttgtct gtaagcggat gccgggagca 1080
   gacaagcccg tcagggcgcg tcagcgggtg ttggcgggtg tcggggcgca gccatgaccc 1140
   agtcacgtag cgatagcgga gtgtataatt cttgaagacg aaagggcctc gtgatacgcc 1200
   tatttttata ggttaatgtc atgataataa tggtttctta gacgtcaggt ggcacttttc 1260
   ggggaaatgt gcgcggaacc cctatttgtt tatttttcta aatacattca aatatgtatc 1320
   cgctcatgag acaataaccc tgataaatgc ttcaataata ttgaaaaagg aagagtatga 1380
   gtattcaaca tttccgtgtc gcccttattc ccttttttgc ggcattttgc cttcctgttt 1440
   ttgctcaccc agaaacgctg gtgaaagtaa aagatgctga agatcagttg ggtgcacgag 1500
   tgggttacat cgaactggat ctcaacagcg gtaagatcct tgagagtttt cgccccgaag 1560
   aacgttttcc aatgatgagc acttttaaag ttctgctatg tggcgcggta ttatcccgtg 1620
   ttgacgccgg gcaagagcaa ctcggtcgcc gcatacacta ttctcagaat gacttggttg 1680
   agtactcacc agtcacagaa aagcatctta cggatggcat gacagtaaga gaattatgca 1740
   gtgctgccat aaccatgagt gataacactg cggccaactt acttctgaca acgatcggag 1800
   gaccgaagga gctaaccgct tttttgcaca acatggggga tcatgtaact cgccttgatc 1860
   gttgggaacc ggagctgaat gaagccatac caaacgacga gcgtgacacc acgatgcctg 1920
   cagcaatggc aacaacgttg cgcaaactat taactggcga actacttact ctagcttccc 1980
   ggcaacaatt aatagactgg atggaggcgg ataaagttgc aggaccactt ctgcgctcgg 2040
   cccttccggc tggctggttt attgctgata aatctggagc cggtgagcgt gggtctcgcg 2100
   gtatcattgc agcactgggg ccagatggta agccctcccg tatcgtagtt atctacacga 2160
   cggggagtca ggcaactatg gatgaacgaa atagacagat cgctgagata ggtgcctcac 2220
   tgattaagca ttggtaactg tcagaccaag tttactcata tatactttag attgatttaa 2280
   aacttcattt ttaatttaaa aggatctagg tgaagatcct ttttgataat ctcatgacca 2340
   aaatccctta acgtgagttt tcgttccact gagcgtcaga ccccgtagaa aagatcaaag 2400
   gatcttcttg agatcctttt tttctgcgcg taatctgctg cttgcaaaca aaaaaaccac 2460
   cgctaccagc ggtggtttgt ttgccggatc aagagctacc aactcttttt ccgaaggtaa 2520
   ctggcttcag cagagcgcag ataccaaata ctgtccttct agtgtagccg tagttaggcc 2580
   accacttcaa gaactctgta gcaccgccta catacctcgc tctgctaatc ctgttaccag 2640
   tggctgctgc cagtggcgat aagtcgtgtc ttaccgggtt ggactcaaga cgatagttac 2700
   cggataaggc gcagcggtcg ggctgaacgg ggggttcgtg cacacagccc agcttggagc 2760
   gaacgaccta caccgaactg agatacctac agcgtgagct atgagaaagc gccacgcttc 2820
   ccgaagggag aaaggcggac aggtatccgg taagcggcag ggtcggaaca ggagagcgca 2880
   cgagggagct tccaggggga aacgcctggt atctttatag tcctgtcggg tttcgccacc 2940
   tctgacttga gcgtcgattt ttgtgatgct cgtcaggggg gcggagccta tggaaaaacg 3000
   ccagcaacgc ggccttttta cggttcctgg ccttttgctg gccttttgct cacatgttct 3060
   ttcctgcgtt atcccctgat tctgtggata accgtattac cgcctttgag tgagctgata 3120
   ccgctcgccg cagccgaacg accgagcgca gcgagtcagt gagcgaggaa gcggaagagc 3180
   gcctgatgcg gtattttctc cttacgcatc tgtgcggtat ttcacaccgc ataaattccg 3240
   acaccatcga atggtgcaaa acctttcgcg gtatggcatg atagcgcccg gaagagagtc 3300
   aattcagggt ggtgaatgtg aaaccagtaa cgttatacga tgtcgcagag tatgccggtg 3360
   tctcttatca gaccgtttcc cgcgtggtga accaggccag ccacgtttct gcgaaaacgc 3420
   gggaaaaagt ggaagcggcg atggcggagc tgaattacat tcccaaccgc gtggcacaac 3480
   aactggcggg caaacagtcg ttgctgattg gcgttgccac ctccagtctg gccctgcacg 3540
   cgccgtcgca aattgtcgcg gcgattaaat ctcgcgccga tcaactgggt gccagcgtgg 3600
   tggtgtcgat ggtagaacga agcggcgtcg aagcctgtaa agcggcggtg cacaatcttc 3660
   tcgcgcaacg cgtcagtggg ctgatcatta actatccgct ggatgaccag gatgccattg 3720
   ctgtggaagc tgcctgcact aatgttccgg cgttatttct tgatgtctct gaccagacac 3780
   ccatcaacag tattattttc tcccatgaag acggtacgcg actgggcgtg gagcatctgg 3840
   tcgcattggg tcaccagcaa atcgcgctgt tagcgggccc attaagttct gtctcggcgc 3900
   gtctgcgtct ggctggctgg cataaatatc tcactcgcaa tcaaattcag ccgatagcgg 3960
   aacgggaagg cgactggagt gccatgtccg gttttcaaca aaccatgcaa atgctgaatg 4020
   agggcatcgt tcccactgcg atgctggttg ccaacgatca gatggcgctg ggcgcaatgc 4080
   gcgccattac cgagtccggg ctgcgcgttg gtgcggatat ctcggtagtg ggatacgacg 4140
   ataccgaaga cagctcatgt tatatcccgc cgttaaccac catcaaacag gattttcgcc 4200
   tgctggggca aaccagcgtg gaccgcttgc tgcaactctc tcagggccag gcggtgaagg 4260
   gcaatcagct gttgcccgtc tcactggtga aaagaaaaac caccctggcg cccaatacgc 4320
   aaaccgcctc tccccgcgcg ttggccgatt cattaatgca gctggcacga caggtttccc 4380
   gactggaaag cgggcagtga gcgcaacgca attaatgtga gttagctcac tcattaggca 4440
   ccccaggctt tacactttat gcttccggct cgtatgttgt gtggaattgt gagcggataa 4500
   caatttcaca caggaaacag ctatgaccat gattacggat tcactggccg tcgttttaca 4560
   acgtcgtgac tgggaaaacc ctggcgttac ccaacttaat cgccttgcag cacatccccc 4620
   tttcgccagc tggcgtaata gcgaagaggc ccgcaccgat cgcccttccc aacagttgcg 4680
   cagcctgaat ggcgaatggc gctttgcctg gtttccggca ccagaagcgg tgccggaaag 4740
   ctggctggag tgcgatcttc ctgaggccga tactgtcgtc gtcccctcaa actggcagat 4800
   gcacggttac gatgcgccca tctacaccaa cgtaacctat cccattacgg tcaatccgcc 4860
   gtttgttccc acggagaatc cgacgggttg ttactcgctc acatttaatg ttgatgaaag 4920
   ctggctacag gaaggccaga cgcgaattat ttttgatggc gttggaatt           4969
<210> 24
   <211> 717
   <212> ADN
   <213> Séquence artificielle
<220>
   <223> Description de la séquence artificielle: ADN codant pour la GST dans le plasmide pGEXKT
<400> 24
   atgtccccta tactaggtta ttggaaaatt aagggccttg tgcaacccac tcgacttctt 60
   ttggaatatc ttgaagaaaa atatgaagag catttgtatg agcgcgatga aggtgataaa 120
   tggcgaaaca aaaagtttga attgggtttg gagtttccca atcttcctta ttatattgat 180
   ggtgatgtta aattaacaca gtctatggcc atcatacgtt atatagctga caagcacaac 240
   atgttgggtg gttgtccaaa agagcgtgca gagatttcaa tgcttgaagg agcggttttg 300
   gatattagat acggtgtttc gagaattgca tatagtaaag actttgaaac tctcaaagtt 360
   gattttctta gcaagctacc tgaaatgctg aaaatgttcg aagatcgttt atgtcataaa 420
   acatatttaa atggtgatca tgtaacccat cctgacttca tgttgtatga cgctcttgat 480
   gttgttttat acatggaccc aatgtgcctg gatgcgttcc caaaattagt ttgttttaaa 540
   aaacgtattg aagctatccc acaaattgat aagtacttga aatccagcaa gtatatagca 600
   tggcctttgc agggctggca agccacgttt ggtggtggcg accatcctcc aaaatcggat 660
   ctgtctggtg gtggtggtgg tctggttccg cgtggatccc cgggaattca tcgtgac    717
<210> 25
   <211> 239
   <212> PRT
   <213> Séquence artificielle
<220>
   <223> Description de la séquence artificielle: protéine GST codée dans le plasmide pGEXKT
<400> 25
   Met Ser Pro Ile Leu Gly Tyr Trp Lys Ile Lys Gly Leu Val Gln Pro
    1               5                  10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Leu Ser Gly Gly
       210                 215                 220
   Gly Gly Gly Leu Val Pro Arg Gly Ser Pro Gly Ile His Arg Asp
   225                 230                 235
<210> 26
   <211> 5082
   <212> ADN
   <213> Séquence artificielle
<220>
   <223> Description de la séquence artificielle: vecteur pGEXKT-DP-TME1
<400> 26
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cgatcgctgg tgctcactgg ggtgttctgg ctggtatcgc ttacttctct atggttggta 1020
   actgggctaa agttctggtt gttctgctgc tgttcgctgg tgttgacgct taggaattca 1080
   tcgtgactga ctgacgatct gcctcgcgcg tttcggtgat gacggtgaaa acctctgaca 1140
   catgcagctc ccggagacgg tcacagcttg tctgtaagcg gatgccggga gcagacaagc 1200
   ccgtcagggc gcgtcagcgg gtgttggcgg gtgtcggggc gcagccatga cccagtcacg 1260
   tagcgatagc ggagtgtata attcttgaag acgaaagggc ctcgtgatac gcctattttt 1320
   ataggttaat gtcatgataa taatggtttc ttagacgtca ggtggcactt ttcggggaaa 1380
   tgtgcgcgga acccctattt gtttattttt ctaaatacat tcaaatatgt atccgctcat 1440
   gagacaataa ccctgataaa tgcttcaata atattgaaaa aggaagagta tgagtattca 1500
   acatttccgt gtcgccctta ttcccttttt tgcggcattt tgccttcctg tttttgctca 1560
   cccagaaacg ctggtgaaag taaaagatgc tgaagatcag ttgggtgcac gagtgggtta 1620
   catcgaactg gatctcaaca gcggtaagat ccttgagagt tttcgccccg aagaacgttt 1680
   tccaatgatg agcactttta aagttctgct atgtggcgcg gtattatccc gtgttgacgc 1740
   cgggcaagag caactcggtc gccgcataca ctattctcag aatgacttgg ttgagtactc 1800
   accagtcaca gaaaagcatc ttacggatgg catgacagta agagaattat gcagtgctgc 1860
   cataaccatg agtgataaca ctgcggccaa cttacttctg acaacgatcg gaggaccgaa 1920
   ggagctaacc gcttttttgc acaacatggg ggatcatgta actcgccttg atcgttggga 1980
   accggagctg aatgaagcca taccaaacga cgagcgtgac accacgatgc ctgcagcaat 2040
   ggcaacaacg ttgcgcaaac tattaactgg cgaactactt actctagctt cccggcaaca 2100
   attaatagac tggatggagg cggataaagt tgcaggacca cttctgcgct cggcccttcc 2160
   ggctggctgg tttattgctg ataaatctgg agccggtgag cgtgggtctc gcggtatcat 2220
   tgcagcactg gggccagatg gtaagccctc ccgtatcgta gttatctaca cgacggggag 2280
   tcaggcaact atggatgaac gaaatagaca gatcgctgag ataggtgcct cactgattaa 2340
   gcattggtaa ctgtcagacc aagtttactc atatatactt tagattgatt taaaacttca 2400
   tttttaattt aaaaggatct aggtgaagat cctttttgat aatctcatga ccaaaatccc 2460
   ttaacgtgag ttttcgttcc actgagcgtc agaccccgta gaaaagatca aaggatcttc 2520
   ttgagatcct ttttttctgc gcgtaatctg ctgcttgcaa acaaaaaaac caccgctacc 2580
   agcggtggtt tgtttgccgg atcaagagct accaactctt tttccgaagg taactggctt 2640
   cagcagagcg cagataccaa atactgtcct tctagtgtag ccgtagttag gccaccactt 2700
   caagaactct gtagcaccgc ctacatacct cgctctgcta atcctgttac cagtggctgc 2760
   tgccagtggc gataagtcgt gtcttaccgg gttggactca agacgatagt taccggataa 2820
   ggcgcagcgg tcgggctgaa cggggggttc gtgcacacag cccagcttgg agcgaacgac 2880
   ctacaccgaa ctgagatacc tacagcgtga gctatgagaa agcgccacgc ttcccgaagg 2940
   gagaaaggcg gacaggtatc cggtaagcgg cagggtcgga acaggagagc gcacgaggga 3000
   gcttccaggg ggaaacgcct ggtatcttta tagtcctgtc gggtttcgcc acctctgact 3060
   tgagcgtcga tttttgtgat gctcgtcagg ggggcggagc ctatggaaaa acgccagcaa 3120
   cgcggccttt ttacggttcc tggccttttg ctggcctttt gctcacatgt tctttcctgc 3180
   gttatcccct gattctgtgg ataaccgtat taccgccttt gagtgagctg ataccgctcg 3240
   ccgcagccga acgaccgagc gcagcgagtc agtgagcgag gaagcggaag agcgcctgat 3300
   gcggtatttt ctccttacgc atctgtgcgg tatttcacac cgcataaatt ccgacaccat 3360
   cgaatggtgc aaaacctttc gcggtatggc atgatagcgc ccggaagaga gtcaattcag 3420
   ggtggtgaat gtgaaaccag taacgttata cgatgtcgca gagtatgccg gtgtctctta 3480
   tcagaccgtt tcccgcgtgg tgaaccaggc cagccacgtt tctgcgaaaa cgcgggaaaa 3540
   agtggaagcg gcgatggcgg agctgaatta cattcccaac cgcgtggcac aacaactggc 3600
   gggcaaacag tcgttgctga ttggcgttgc cacctccagt ctggccctgc acgcgccgtc 3660
   gcaaattgtc gcggcgatta aatctcgcgc cgatcaactg ggtgccagcg tggtggtgtc 3720
   gatggtagaa cgaagcggcg tcgaagcctg taaagcggcg gtgcacaatc ttctcgcgca 3780
   acgcgtcagt gggctgatca ttaactatcc gctggatgac caggatgcca ttgctgtgga 3840
   agctgcctgc actaatgttc cggcgttatt tcttgatgtc tctgaccaga cacccatcaa 3900
   cagtattatt ttctcccatg aagacggtac gcgactgggc gtggagcatc tggtcgcatt 3960
   gggtcaccag caaatcgcgc tgttagcggg cccattaagt tctgtctcgg cgcgtctgcg 4020
   tctggctggc tggcataaat atctcactcg caatcaaatt cagccgatag cggaacggga 4080
   aggcgactgg agtgccatgt ccggttttca acaaaccatg caaatgctga atgagggcat 4140
   cgttcccact gcgatgctgg ttgccaacga tcagatggcg ctgggcgcaa tgcgcgccat 4200
   taccgagtcc gggctgcgcg ttggtgcgga tatctcggta gtgggatacg acgataccga 4260
   agacagctca tgttatatcc cgccgttaac caccatcaaa caggattttc gcctgctggg 4320
   gcaaaccagc gtggaccgct tgctgcaact ctctcagggc caggcggtga agggcaatca 4380
   gctgttgccc gtctcactgg tgaaaagaaa aaccaccctg gcgcccaata cgcaaaccgc 4440
   ctctccccgc gcgttggccg attcattaat gcagctggca cgacaggttt cccgactgga 4500
   aagcgggcag tgagcgcaac gcaattaatg tgagttagct cactcattag gcaccccagg 4560
   ctttacactt tatgcttccg gctcgtatgt tgtgtggaat tgtgagcgga taacaatttc 4620
   acacaggaaa cagctatgac catgattacg gattcactgg ccgtcgtttt acaacgtcgt 4680
   gactgggaaa accctggcgt tacccaactt aatcgccttg cagcacatcc ccctttcgcc 4740
   agctggcgta atagcgaaga ggcccgcacc gatcgccctt cccaacagtt gcgcagcctg 4800
   aatggcgaat ggcgctttgc ctggtttccg gcaccagaag cggtgccgga aagctggctg 4860
   gagtgcgatc ttcctgaggc cgatactgtc gtcgtcccct caaactggca gatgcacggt 4920
   tacgatgcgc ccatctacac caacgtaacc tatcccatta cggtcaatcc gccgtttgtt 4980
   cccacggaga atccgacggg ttgttactcg ctcacattta atgttgatga aagctggcta 5040
   caggaaggcc agacgcgaat tatttttgat ggcgttggaa tt                    5082
<210> 27
   <211> 813
   <212> ADN
   <213> Séquence artificielle
<220>
   <223> Description de la séquence artificielle: ADN codant pour la protéine de fusion GST-DP-TME1 dans le vecteur pGEXKT-DP-TME1
<400> 27
   atgtccccta tactaggtta ttggaaaatt aagggccttg tgcaacccac tcgacttctt 60
   ttggaatatc ttgaagaaaa atatgaagag catttgtatg agcgcgatga aggtgataaa 120
   tggcgaaaca aaaagtttga attgggtttg gagtttccca atcttcctta ttatattgat 180
   ggtgatgtta aattaacaca gtctatggcc atcatacgtt atatagctga caagcacaac 240
   atgttgggtg gttgtccaaa agagcgtgca gagatttcaa tgcttgaagg agcggttttg 300
   gatattagat acggtgtttc gagaattgca tatagtaaag actttgaaac tctcaaagtt 360
   gattttctta gcaagctacc tgaaatgctg aaaatgttcg aagatcgttt atgtcataaa 420
   acatatttaa atggtgatca tgtaacccat cctgacttca tgttgtatga cgctcttgat 480
   gttgttttat acatggaccc aatgtgcctg gatgcgttcc caaaattagt ttgttttaaa 540
   aaacgtattg aagctatccc acaaattgat aagtacttga aatccagcaa gtatatagca 600
   tggcctttgc agggctggca agccacgttt ggtggtggcg accatcctcc aaaatcggat 660
   ctgtctggtg gtggtggtgg tctggttccg cgtggatccg acccgatcgc tggtgctcac 720
   tggggtgttc tggctggtat cgcttacttc tctatggttg gtaactgggc taaagttctg 780
   gttgttctgc tgctgttcgc tggtgttgac gct                              813
<210> 28
   <211> 271
   <212> PRT
   <213> Séquence artificielle
<220>
   <223> Description de la séquence artificielle: protéine GST-DP-TME1 codée par le vecteur pGEXKT-DP-TME1
<400> 28
   Met Ser Pro Ile Leu Gly Tyr Trp Lys Ile Lys Gly Leu Val Gln Pro
    1               5                  10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Leu Ser Gly Gly
       210                 215                 220
   Gly Gly Gly Leu Val Pro Arg Gly Ser Asp Pro Ile Ala Gly Ala His
   225                 230                 235                 240
   Trp Gly Val Leu Ala Gly Ile Ala Tyr Phe Ser Met Val Gly Asn Trp
                   245                 250                 255
   Ala Lys Val Leu Val Val Leu Leu Leu Phe Ala Gly Val Asp Ala
               260                 265                 270
<210> 29
   <211> 5064
   <212> ADN
   <213> Séquence artificielle
<220>
   <223> Description de la séquence artificielle: vecteur pGEXKT-DP-TME2
<400> 29
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cggaatacgt tgttctgctg ttcctgctgc tggctgacgc tcgtgtttgc tcttgcctgt 1020
   ggatgatgct gctgatctct caggctgaag cttaggaatt catcgtgact gactgacgat 1080
   ctgcctcgcg cgtttcggtg atgacggtga aaacctctga cacatgcagc tcccggagac 1140
   ggtcacagct tgtctgtaag cggatgccgg gagcagacaa gcccgtcagg gcgcgtcagc 1200
   gggtgttggc gggtgtcggg gcgcagccat gacccagtca cgtagcgata gcggagtgta 1260
   taattcttga agacgaaagg gcctcgtgat acgcctattt ttataggtta atgtcatgat 1320
   aataatggtt tcttagacgt caggtggcac ttttcgggga aatgtgcgcg gaacccctat 1380
   ttgtttattt ttctaaatac attcaaatat gtatccgctc atgagacaat aaccctgata 1440
   aatgcttcaa taatattgaa aaaggaagag tatgagtatt caacatttcc gtgtcgccct 1500
   tattcccttt tttgcggcat tttgccttcc tgtttttgct cacccagaaa cgctggtgaa 1560
   agtaaaagat gctgaagatc agttgggtgc acgagtgggt tacatcgaac tggatctcaa 1620
   cagcggtaag atccttgaga gttttcgccc cgaagaacgt tttccaatga tgagcacttt 1680
   taaagttctg ctatgtggcg cggtattatc ccgtgttgac gccgggcaag agcaactcgg 1740
   tcgccgcata cactattctc agaatgactt ggttgagtac tcaccagtca cagaaaagca 1800
   tcttacggat ggcatgacag taagagaatt atgcagtgct gccataacca tgagtgataa 1860
   cactgcggcc aacttacttc tgacaacgat cggaggaccg aaggagctaa ccgctttttt 1920
   gcacaacatg ggggatcatg taactcgcct tgatcgttgg gaaccggagc tgaatgaagc 1980
   cataccaaac gacgagcgtg acaccacgat gcctgcagca atggcaacaa cgttgcgcaa 2040
   actattaact ggcgaactac ttactctagc ttcccggcaa caattaatag actggatgga 2100
   ggcggataaa gttgcaggac cacttctgcg ctcggccctt ccggctggct ggtttattgc 2160
   tgataaatct ggagccggtg agcgtgggtc tcgcggtatc attgcagcac tggggccaga 2220
   tggtaagccc tcccgtatcg tagttatcta cacgacgggg agtcaggcaa ctatggatga 2280
   acgaaataga cagatcgctg agataggtgc ctcactgatt aagcattggt aactgtcaga 2340
   ccaagtttac tcatatatac tttagattga tttaaaactt catttttaat ttaaaaggat 2400
   ctaggtgaag atcctttttg ataatctcat gaccaaaatc ccttaacgtg agttttcgtt 2460
   ccactgagcg tcagaccccg tagaaaagat caaaggatct tcttgagatc ctttttttct 2520
   gcgcgtaatc tgctgcttgc aaacaaaaaa accaccgcta ccagcggtgg tttgtttgcc 2580
   ggatcaagag ctaccaactc tttttccgaa ggtaactggc ttcagcagag cgcagatacc 2640
   aaatactgtc cttctagtgt agccgtagtt aggccaccac ttcaagaact ctgtagcacc 2700
   gcctacatac ctcgctctgc taatcctgtt accagtggct gctgccagtg gcgataagtc 2760
   gtgtcttacc gggttggact caagacgata gttaccggat aaggcgcagc ggtcgggctg 2820
   aacggggggt tcgtgcacac agcccagctt ggagcgaacg acctacaccg aactgagata 2880
   cctacagcgt gagctatgag aaagcgccac gcttcccgaa gggagaaagg cggacaggta 2940
   tccggtaagc ggcagggtcg gaacaggaga gcgcacgagg gagcttccag ggggaaacgc 3000
   ctggtatctt tatagtcctg tcgggtttcg ccacctctga cttgagcgtc gatttttgtg 3060
   atgctcgtca ggggggcgga gcctatggaa aaacgccagc aacgcggcct ttttacggtt 3120
   cctggccttt tgctggcctt ttgctcacat gttctttcct gcgttatccc ctgattctgt 3180
   ggataaccgt attaccgcct ttgagtgagc tgataccgct cgccgcagcc gaacgaccga 3240
   gcgcagcgag tcagtgagcg aggaagcgga agagcgcctg atgcggtatt ttctccttac 3300
   gcatctgtgc ggtatttcac accgcataaa ttccgacacc atcgaatggt gcaaaacctt 3360
   tcgcggtatg gcatgatagc gcccggaaga gagtcaattc agggtggtga atgtgaaacc 3420
   agtaacgtta tacgatgtcg cagagtatgc cggtgtctct tatcagaccg tttcccgcgt 3480
   ggtgaaccag gccagccacg tttctgcgaa aacgcgggaa aaagtggaag cggcgatggc 3540
   ggagctgaat tacattccca accgcgtggc acaacaactg gcgggcaaac agtcgttgct 3600
   gattggcgtt gccacctcca gtctggccct gcacgcgccg tcgcaaattg tcgcggcgat 3660
   taaatctcgc gccgatcaac tgggtgccag cgtggtggtg tcgatggtag aacgaagcgg 3720
   cgtcgaagcc tgtaaagcgg cggtgcacaa tcttctcgcg caacgcgtca gtgggctgat 3780
   cattaactat ccgctggatg accaggatgc cattgctgtg gaagctgcct gcactaatgt 3840
   tccggcgtta tttcttgatg tctctgacca gacacccatc aacagtatta ttttctccca 3900
   tgaagacggt acgcgactgg gcgtggagca tctggtcgca ttgggtcacc agcaaatcgc 3960
   gctgttagcg ggcccattaa gttctgtctc ggcgcgtctg cgtctggctg gctggcataa 4020
   atatctcact cgcaatcaaa ttcagccgat agcggaacgg gaaggcgact ggagtgccat 4080
   gtccggtttt caacaaacca tgcaaatgct gaatgagggc atcgttccca ctgcgatgct 4140
   ggttgccaac gatcagatgg cgctgggcgc aatgcgcgcc attaccgagt ccgggctgcg 4200
   cgttggtgcg gatatctcgg tagtgggata cgacgatacc gaagacagct catgttatat 4260
   cccgccgtta accaccatca aacaggattt tcgcctgctg gggcaaacca gcgtggaccg 4320
   cttgctgcaa ctctctcagg gccaggcggt gaagggcaat cagctgttgc ccgtctcact 4380
   ggtgaaaaga aaaaccaccc tggcgcccaa tacgcaaacc gcctctcccc gcgcgttggc 4440
   cgattcatta atgcagctgg cacgacaggt ttcccgactg gaaagcgggc agtgagcgca 4500
   acgcaattaa tgtgagttag ctcactcatt aggcacccca ggctttacac tttatgcttc 4560
   cggctcgtat gttgtgtgga attgtgagcg gataacaatt tcacacagga aacagctatg 4620
   accatgatta cggattcact ggccgtcgtt ttacaacgtc gtgactggga aaaccctggc 4680
   gttacccaac ttaatcgcct tgcagcacat ccccctttcg ccagctggcg taatagcgaa 4740
   gaggcccgca ccgatcgccc ttcccaacag ttgcgcagcc tgaatggcga atggcgcttt 4800
   gcctggtttc cggcaccaga agcggtgccg gaaagctggc tggagtgcga tcttcctgag 4860
   gccgatactg tcgtcgtccc ctcaaactgg cagatgcacg gttacgatgc gcccatctac 4920
   accaacgtaa cctatcccat tacggtcaat ccgccgtttg ttcccacgga gaatccgacg 4980
   ggttgttact cgctcacatt taatgttgat gaaagctggc tacaggaagg ccagacgcga 5040
   attatttttg atggcgttgg aatt                                        5064
<210> 30
   <211> 795
   <212> ADN
   <213> Séquence artificielle
<220>
   <223> Description de la séquence artificielle: ADN codant pour la protéine de fusion GST-DP-TME2 dans le vecteur pGEXKT-DP-TME2
<400> 30
   atgtccccta tactaggtta ttggaaaatt aagggccttg tgcaacccac tcgacttctt 60
   ttggaatatc ttgaagaaaa atatgaagag catttgtatg agcgcgatga aggtgataaa 120
   tggcgaaaca aaaagtttga attgggtttg gagtttccca atcttcctta ttatattgat 180
   ggtgatgtta aattaacaca gtctatggcc atcatacgtt atatagctga caagcacaac 240
   atgttgggtg gttgtccaaa agagcgtgca gagatttcaa tgcttgaagg agcggttttg 300
   gatattagat acggtgtttc gagaattgca tatagtaaag actttgaaac tctcaaagtt 360
   gattttctta gcaagctacc tgaaatgctg aaaatgttcg aagatcgttt atgtcataaa 420
   acatatttaa atggtgatca tgtaacccat cctgacttca tgttgtatga cgctcttgat 480
   gttgttttat acatggaccc aatgtgcctg gatgcgttcc caaaattagt ttgttttaaa 540
   aaacgtattg aagctatccc acaaattgat aagtacttga aatccagcaa gtatatagca 600
   tggcctttgc agggctggca agccacgttt ggtggtggcg accatcctcc aaaatcggat 660
   ctgtctggtg gtggtggtgg tctggttccg cgtggatccg acccggaata cgttgttctg 720
   ctgttcctgc tgctggctga cgctcgtgtt tgctcttgcc tgtggatgat gctgctgatc 780
   tctcaggctg aagct                                                  795
<210> 31
   <211> 265
   <212> PRT
   <213> Séquence artificielle
<220>
   <223> Description de la séquence artificielle: protéine GST-DP-TME2 codée par le vecteur pGEXKT-DP-TME2
<400> 31
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Leu Ser Gly Gly
       210                 215                 220
   Gly Gly Gly Leu Val Pro Arg Gly Ser Asp Pro Glu Tyr Val Val Leu
   225                 230                 235                 240
   Leu Phe Leu Leu Leu Ala Asp Ala Arg Val Cys Ser Cys Leu Trp Met
                   245                 250                 255
   Met Leu Leu Ile Ser Gln Ala Glu Ala
               260                 265
<210> 32
   <211> 5064
   <212> ADN
   <213> Séquence artificielle
<220>
   <223> Description de la séquence artificielle: vecteur pGEXKT-DP-TME2_C731&C733A
<400> 32
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cggaatacgt tgttctgctg ttcctgctgc tggctgacgc tcgtgttgct tctgctctgt 1020
   ggatgatgct gctgatctct caggctgaag cttaggaatt catcgtgact gactgacgat 1080
   ctgcctcgcg cgtttcggtg atgacggtga aaacctctga cacatgcagc tcccggagac 1140
   ggtcacagct tgtctgtaag cggatgccgg gagcagacaa gcccgtcagg gcgcgtcagc 1200
   gggtgttggc gggtgtcggg gcgcagccat gacccagtca cgtagcgata gcggagtgta 1260
   taattcttga agacgaaagg gcctcgtgat acgcctattt ttataggtta atgtcatgat 1320
   aataatggtt tcttagacgt caggtggcac ttttcgggga aatgtgcgcg gaacccctat 1380
   ttgtttattt ttctaaatac attcaaatat gtatccgctc atgagacaat aaccctgata 1440
   aatgcttcaa taatattgaa aaaggaagag tatgagtatt caacatttcc gtgtcgccct 1500
   tattcccttt tttgcggcat tttgccttcc tgtttttgct cacccagaaa cgctggtgaa 1560
   agtaaaagat gctgaagatc agttgggtgc acgagtgggt tacatcgaac tggatctcaa 1620
   cagcggtaag atccttgaga gttttcgccc cgaagaacgt tttccaatga tgagcacttt 1680
   taaagttctg ctatgtggcg cggtattatc ccgtgttgac gccgggcaag agcaactcgg 1740
   tcgccgcata cactattctc agaatgactt ggttgagtac tcaccagtca cagaaaagca 1800
   tcttacggat ggcatgacag taagagaatt atgcagtgct gccataacca tgagtgataa 1860
   cactgcggcc aacttacttc tgacaacgat cggaggaccg aaggagctaa ccgctttttt 1920
   gcacaacatg ggggatcatg taactcgcct tgatcgttgg gaaccggagc tgaatgaagc 1980
   cataccaaac gacgagcgtg acaccacgat gcctgcagca atggcaacaa cgttgcgcaa 2040
   actattaact ggcgaactac ttactctagc ttcccggcaa caattaatag actggatgga 2100
   ggcggataaa gttgcaggac cacttctgcg ctcggccctt ccggctggct ggtttattgc 2160
   tgataaatct ggagccggtg agcgtgggtc tcgcggtatc attgcagcac tggggccaga 2220
   tggtaagccc tcccgtatcg tagttatcta cacgacgggg agtcaggcaa ctatggatga 2280
   acgaaataga cagatcgctg agataggtgc ctcactgatt aagcattggt aactgtcaga 2340
   ccaagtttac tcatatatac tttagattga tttaaaactt catttttaat ttaaaaggat 2400
   ctaggtgaag atcctttttg ataatctcat gaccaaaatc ccttaacgtg agttttcgtt 2460
   ccactgagcg tcagaccccg tagaaaagat caaaggatct tcttgagatc ctttttttct 2520
   gcgcgtaatc tgctgcttgc aaacaaaaaa accaccgcta ccagcggtgg tttgtttgcc 2580
   ggatcaagag ctaccaactc tttttccgaa ggtaactggc ttcagcagag cgcagatacc 2640
   aaatactgtc cttctagtgt agccgtagtt aggccaccac ttcaagaact ctgtagcacc 2700
   gcctacatac ctcgctctgc taatcctgtt accagtggct gctgccagtg gcgataagtc 2760
   gtgtcttacc gggttggact caagacgata gttaccggat aaggcgcagc ggtcgggctg 2820
   aacggggggt tcgtgcacac agcccagctt ggagcgaacg acctacaccg aactgagata 2880
   cctacagcgt gagctatgag aaagcgccac gcttcccgaa gggagaaagg cggacaggta 2940
   tccggtaagc ggcagggtcg gaacaggaga gcgcacgagg gagcttccag ggggaaacgc 3000
   ctggtatctt tatagtcctg tcgggtttcg ccacctctga cttgagcgtc gatttttgtg 3060
   atgctcgtca ggggggcgga gcctatggaa aaacgccagc aacgcggcct ttttacggtt 3120
   cctggccttt tgctggcctt ttgctcacat gttctttcct gcgttatccc ctgattctgt 3180
   ggataaccgt attaccgcct ttgagtgagc tgataccgct cgccgcagcc gaacgaccga 3240
   gcgcagcgag tcagtgagcg aggaagcgga agagcgcctg atgcggtatt ttctccttac 3300
   gcatctgtgc ggtatttcac accgcataaa ttccgacacc atcgaatggt gcaaaacctt 3360
   tcgcggtatg gcatgatagc gcccggaaga gagtcaattc agggtggtga atgtgaaacc 3420
   agtaacgtta tacgatgtcg cagagtatgc cggtgtctct tatcagaccg tttcccgcgt 3480
   ggtgaaccag gccagccacg tttctgcgaa aacgcgggaa aaagtggaag cggcgatggc 3540
   ggagctgaat tacattccca accgcgtggc acaacaactg gcgggcaaac agtcgttgct 3600
   gattggcgtt gccacctcca gtctggccct gcacgcgccg tcgcaaattg tcgcggcgat 3660
   taaatctcgc gccgatcaac tgggtgccag cgtggtggtg tcgatggtag aacgaagcgg 3720
   cgtcgaagcc tgtaaagcgg cggtgcacaa tcttctcgcg caacgcgtca gtgggctgat 3780
   cattaactat ccgctggatg accaggatgc cattgctgtg gaagctgcct gcactaatgt 3840
   tccggcgtta tttcttgatg tctctgacca gacacccatc aacagtatta ttttctccca 3900
   tgaagacggt acgcgactgg gcgtggagca tctggtcgca ttgggtcacc agcaaatcgc 3960
   gctgttagcg ggcccattaa gttctgtctc ggcgcgtctg cgtctggctg gctggcataa 4020
   atatctcact cgcaatcaaa ttcagccgat agcggaacgg gaaggcgact ggagtgccat 4080
   gtccggtttt caacaaacca tgcaaatgct gaatgagggc atcgttccca ctgcgatgct 4140
   ggttgccaac gatcagatgg cgctgggcgc aatgcgcgcc attaccgagt ccgggctgcg 4200
   cgttggtgcg gatatctcgg tagtgggata cgacgatacc gaagacagct catgttatat 4260
   cccgccgtta accaccatca aacaggattt tcgcctgctg gggcaaacca gcgtggaccg 4320
   cttgctgcaa ctctctcagg gccaggcggt gaagggcaat cagctgttgc ccgtctcact 4380
   ggtgaaaaga aaaaccaccc tggcgcccaa tacgcaaacc gcctctcccc gcgcgttggc 4440
   cgattcatta atgcagctgg cacgacaggt ttcccgactg gaaagcgggc agtgagcgca 4500
   acgcaattaa tgtgagttag ctcactcatt aggcacccca ggctttacac tttatgcttc 4560
   cggctcgtat gttgtgtgga attgtgagcg gataacaatt tcacacagga aacagctatg 4620
   accatgatta cggattcact ggccgtcgtt ttacaacgtc gtgactggga aaaccctggc 4680
   gttacccaac ttaatcgcct tgcagcacat ccccctttcg ccagctggcg taatagcgaa 4740
   gaggcccgca ccgatcgccc ttcccaacag ttgcgcagcc tgaatggcga atggcgcttt 4800
   gcctggtttc cggcaccaga agcggtgccg gaaagctggc tggagtgcga tcttcctgag 4860
   gccgatactg tcgtcgtccc ctcaaactgg cagatgcacg gttacgatgc gcccatctac 4920
   accaacgtaa cctatcccat tacggtcaat ccgccgtttg ttcccacgga gaatccgacg 4980
   ggttgttact cgctcacatt taatgttgat gaaagctggc tacaggaagg ccagacgcga 5040
   attatttttg atggcgttgg aatt                                        5064
<210> 33
   <211> 795
   <212> ADN
   <213> Séquence artificielle
<220>
   <223> Description de la séquence artificielle: ADN codant pour la protéine de fusion GST-DP-TME2_C731&C733A dans le vevteur pGEXKT-DP-TME2
<400> 33
   atgtccccta tactaggtta ttggaaaatt aagggccttg tgcaacccac tcgacttctt 60
   ttggaatatc ttgaagaaaa atatgaagag catttgtatg agcgcgatga aggtgataaa 120
   tggcgaaaca aaaagtttga attgggtttg gagtttccca atcttcctta ttatattgat 180
   ggtgatgtta aattaacaca gtctatggcc atcatacgtt atatagctga caagcacaac 240
   atgttgggtg gttgtccaaa agagcgtgca gagatttcaa tgcttgaagg agcggttttg 300
   gatattagat acggtgtttc gagaattgca tatagtaaag actttgaaac tctcaaagtt 360
   gattttctta gcaagctacc tgaaatgctg aaaatgttcg aagatcgttt atgtcataaa 420
   acatatttaa atggtgatca tgtaacccat cctgacttca tgttgtatga cgctcttgat 480
   gttgttttat acatggaccc aatgtgcctg gatgcgttcc caaaattagt ttgttttaaa 540
   aaacgtattg aagctatccc acaaattgat aagtacttga aatccagcaa gtatatagca 600
   tggcctttgc agggctggca agccacgttt ggtggtggcg accatcctcc aaaatcggat 660
   ctgtctggtg gtggtggtgg tctggttccg cgtggatccg acccggaata cgttgttctg 720
   ctgttcctgc tgctggctga cgctcgtgtt gcttctgctc tgtggatgat gctgctgatc 780
   tctcaggctg aagct                                                  795
<210> 34
   <211> 265
   <212> PRT
   <213> Séquence artificielle
<220>
   <223> Description de la séquence artificielle: protéine GST-DP-TME2_C731&C733A codée par le vecteur pGEXKT-DP-TME2
<400> 34
   Met Ser Pro Ile Leu Gly Tyr Trp Lys Ile Lys Gly Leu Val Gln Pro
    1               5                  10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Leu Ser Gly Gly
       210                 215                 220
   Gly Gly Gly Leu Val Pro Arg Gly Ser Asp Pro Glu Tyr Val Val Leu
   225                 230                 235                 240
   Leu Phe Leu Leu Leu Ala Asp Ala Arg Val Ala Ser Ala Leu Trp Met
                   245                 250                 255
   Met Leu Leu Ile Ser Gln Ala Glu Ala
               260                 265
<210> 35
   <211> 11800
   <212> ADN
   <213> Séquence artificielle
<220>
   <223> Description de la séquence artificielle: plasmide d'expression pET32a+
<400> 35
   atccggatat agttcctcct ttcagcaaaa aacccctcaa gacccgttta gaggccccaa 60
   ggggttatgc tagttattgc tcagcggtgg cagcagccaa taggcctata tcaaggagga 120
   aagtcgtttt ttggggagtt ctgggcaaat ctccggggtt ccccaatacg atcaataacg 180
   agtcgccacc gtcgtcggtt ctcagcttcc tttcgggctt tgttagcagc cggatctcag 240
   tggtggtggt ggtggtgctc gagtgcggcc gcaagcttgt cgacggagct cgaattcgga 300
   gagtcgaagg aaagcccgaa acaatcgtcg gcctagagtc accaccacca ccaccacgag 360
   ctcacgccgg cgttcgaaca gctgcctcga gcttaagcct tccgatatca gccatggcct 420
   tgtcgtcgtc gtcggtaccc agatctgggc tgtccatgtg ctggcgttcg aatttagcag 480
   cagcggtttc tttcatacca aggctatagt cggtaccgga acagcagcag cagccatggg 540
   tctagacccg acaggtacac gaccgcaagc ttaaatcgtc gtcgccaaag aaagtatggt 600
   gaaccgcgtg gcaccagacc agaagaatga tgatgatgat ggtgcatatg gccagaacca 660
   gaaccggcca ggttagcgtc gaggaactct ttcaactgac cttggcgcac cgtggtctgg 720
   tcttcttact actactacta ccacgtatac cggtcttggt cttggccggt ccaatcgcag 780
   ctccttgaga aagttgactg ctttagacag tgcacccact ttggttgccg ccacttcacc 840
   gtttttgaac agcagcagag tcgggatacc acggatgcca tatttcggcg cagtgccagg 900
   gaaatctgtc acgtgggtga aaccaacggc ggtgaagtgg caaaaacttg tcgtcgtctc 960
   agccctatgg tgcctacggt ataaagccgc gtcacggtcc gttttgatcg atgttcagtt 1020
   ttgcaacggt cagtttgccc tgatattcgt cagcgatttc atccagaatc ggggcgatca 1080
   ttttgcacgg accgcaccac caaaactagc tacaagtcaa aacgttgcca gtcaaacggg 1140
   actataagca gtcgctaaag taggtcttag ccccgctagt aaaacgtgcc tggcgtggtg 1200
   tctgcccaga aatcgacgag gatcgccccg tccgctttga gtacatccgt gtcaaaactg 1260
   tcgtcagtca ggtgaataat tttatcgctc atatgtatat agacgggtct ttagctgctc 1320
   ctagcggggc aggcgaaact catgtaggca cagttttgac agcagtcagt ccacttatta 1380
   aaatagcgag tatacatata ctccttctta aagttaaaca aaattatttc tagaggggaa 1440
   ttgttatccg ctcacaattc ccctatagtg agtcgtatta atttcgcggg atcgagatcg 1500
   gaggaagaat ttcaatttgt tttaataaag atctcccctt aacaataggc gagtgttaag 1560
   gggatatcac tcagcataat taaagcgccc tagctctagc atctcgatcc tctacgccgg 1620
   acgcatcgtg gccggcatca ccggcgccac aggtgcggtt gctggcgcct atatcgccga 1680
   catcaccgat ggggaagatc tagagctagg agatgcggcc tgcgtagcac cggccgtagt 1740
   ggccgcggtg tccacgccaa cgaccgcgga tatagcggct gtagtggcta ccccttctag 1800
   gggctcgcca cttcgggctc atgagcgctt gtttcggcgt gggtatggtg gcaggccccg 1860
   tggccggggg actgttgggc gccatctcct tgcatgcacc cccgagcggt gaagcccgag 1920
   tactcgcgaa caaagccgca cccataccac cgtccggggc accggccccc tgacaacccg 1980
   cggtagagga acgtacgtgg attccttgcg gcggcggtgc tcaacggcct caacctacta 2040
   ctgggctgct tcctaatgca ggagtcgcat aagggagagc gtcgagatcc cggacaccat 2100
   taaggaacgc cgccgccacg agttgccgga gttggatgat gacccgacga aggattacgt 2160
   cctcagcgta ttccctctcg cagctctagg gcctgtggta cgaatggcgc aaaacctttc 2220
   gcggtatggc atgatagcgc ccggaagaga gtcaattcag ggtggtgaat gtgaaaccag 2280
   taacgttata cgatgtcgca gcttaccgcg ttttggaaag cgccataccg tactatcgcg 2340
   ggccttctct cagttaagtc ccaccactta cactttggtc attgcaatat gctacagcgt 2400
   gagtatgccg gtgtctctta tcagaccgtt tcccgcgtgg tgaaccaggc cagccacgtt 2460
   tctgcgaaaa cgcgggaaaa agtggaagcg gcgatggcgg ctcatacggc cacagagaat 2520
   agtctggcaa agggcgcacc acttggtccg gtcggtgcaa agacgctttt gcgccctttt 2580
   tcaccttcgc cgctaccgcc agctgaatta cattcccaac cgcgtggcac aacaactggc 2640
   gggcaaacag tcgttgctga ttggcgttgc cacctccagt ctggccctgc acgcgccgtc 2700
   tcgacttaat gtaagggttg gcgcaccgtg ttgttgaccg cccgtttgtc agcaacgact 2760
   aaccgcaacg gtggaggtca gaccgggacg tgcgcggcag gcaaattgtc gcggcgatta 2820
   aatctcgcgc cgatcaactg ggtgccagcg tggtggtgtc gatggtagaa cgaagcggcg 2880
   tcgaagcctg taaagcggcg cgtttaacag cgccgctaat ttagagcgcg gctagttgac 2940
   ccacggtcgc accaccacag ctaccatctt gcttcgccgc agcttcggac atttcgccgc 3000
   gtgcacaatc ttctcgcgca acgcgtcagt gggctgatca ttaactatcc gctggatgac 3060
   caggatgcca ttgctgtgga agctgcctgc actaatgttc cacgtgttag aagagcgcgt 3120
   tgcgcagtca cccgactagt aattgatagg cgacctactg gtcctacggt aacgacacct 3180
   tcgacggacg tgattacaag cggcgttatt tcttgatgtc tctgaccaga cacccatcaa 3240
   cagtattatt ttctcccatg aagacggtac gcgactgggc gtggagcatc tggtcgcatt 3300
   gccgcaataa agaactacag agactggtct gtgggtagtt gtcataataa aagagggtac 3360
   ttctgccatg cgctgacccg cacctcgtag accagcgtaa gggtcaccag caaatcgcgc 3420
   tgttagcggg cccattaagt tctgtctcgg cgcgtctgcg tctggctggc tggcataaat 3480
   atctcactcg caatcaaatt cccagtggtc gtttagcgcg acaatcgccc gggtaattca 3540
   agacagagcc gcgcagacgc agaccgaccg accgtattta tagagtgagc gttagtttaa 3600
   cagccgatag cggaacggga aggcgactgg agtgccatgt ccggttttca acaaaccatg 3660
   caaatgctga atgagggcat cgttcccact gcgatgctgg gtcggctatc gccttgccct 3720
   tccgctgacc tcacggtaca ggccaaaagt tgtttggtac gtttacgact tactcccgta 3780
   gcaagggtga cgctacgacc ttgccaacga tcagatggcg ctgggcgcaa tgcgcgccat 3840
   taccgagtcc gggctgcgcg ttggtgcgga catctcggta gtgggatacg acgataccga 3900
   aacggttgct agtctaccgc gacccgcgtt acgcgcggta atggctcagg cccgacgcgc 3960
   aaccacgcct gtagagccat caccctatgc tgctatggct agacagctca tgttatatcc 4020
   cgccgttaac caccatcaaa caggattttc gcctgctggg gcaaaccagc gtggaccgct 4080
   tgctgcaact ctctcagggc tctgtcgagt acaatatagg gcggcaattg gtggtagttt 4140
   gtcctaaaag cggacgaccc cgtttggtcg cacctggcga acgacgttga gagagtcccg 4200
   caggcggtga agggcaatca gctgttgccc gtctcactgg tgaaaagaaa aaccaccctg 4260
   gcgcccaata cgcaaaccgc ctctccccgc gcgttggccg gtccgccact tcccgttagt 4320
   cgacaacggg cagagtgacc acttttcttt ttggtgggac cgcgggttat gcgtttggcg 4380
   gagaggggcg cgcaaccggc attcattaat gcagctggca cgacaggttt cccgactgga 4440
   aagcgggcag tgagcgcaac gcaattaatg taagttagct cactcattag gcaccgggat 4500
   taagtaatta cgtcgaccgt gctgtccaaa gggctgacct ttcgcccgtc actcgcgttg 4560
   cgttaattac attcaatcga gtgagtaatc cgtggcccta ctcgaccgat gcccttgaga 4620
   gccttcaacc cagtcagctc cttccggtgg gcgcggggca tgactatcgt cgccgcactt 4680
   atgactgtct tctttatcat gagctggcta cgggaactct cggaagttgg gtcagtcgag 4740
   gaaggccacc cgcgccccgt actgatagca gcggcgtgaa tactgacaga agaaatagta 4800
   gcaactcgta ggacaggtgc cggcagcgct ctgggtcatt ttcggcgagg accgctttcg 4860
   ctggagcgcg acgatgatcg gcctgtcgct tgcggtattc cgttgagcat cctgtccacg 4920
   gccgtcgcga gacccagtaa aagccgctcc tggcgaaagc gacctcgcgc tgctactagc 4980
   cggacagcga acgccataag ggaatcttgc acgccctcgc tcaagccttc gtcactggtc 5040
   ccgccaccaa acgtttcggc gagaagcagg ccattatcgc cggcatggcg gccccacggg 5100
   ccttagaacg tgcgggagcg agttcggaag cagtgaccag ggcggtggtt tgcaaagccg 5160
   ctcttcgtcc ggtaatagcg gccgtaccgc cggggtgccc tgcgcatgat cgtgctcctg 5220
   tcgttgagga cccggctagg ctggcggggt tgccttactg gttagcagaa tgaatcaccg 5280
   atacgcgagc gaacgtgaag acgcgtacta gcacgaggac agcaactcct gggccgatcc 5340
   gaccgcccca acggaatgac caatcgtctt acttagtggc tatgcgctcg cttgcacttc 5400
   cgactgctgc tgcaaaacgt ctgcgacctg agcaacaaca tgaatggtct tcggtttccg 5460
   tgtttcgtaa agtctggaaa cgcggaagtc agcgccctgc gctgacgacg acgttttgca 5520
   gacgctggac tcgttgttgt acttaccaga agccaaaggc acaaagcatt tcagaccttt 5580
   gcgccttcag tcgcgggacg accattatgt tccggatctg catcgcagga tgctgctggc 5640
   taccctgtgg aacacctaca tctgtattaa cgaagcgctg gcattgaccc tgagtgattt 5700
   tggtaataca aggcctagac gtagcgtcct acgacgaccg atgggacacc ttgtggatgt 5760
   agacataatt gcttcgcgac cgtaactggg actcactaaa ttctctggtc ccgccgcatc 5820
   cataccgcca gttgtttacc ctcacaacgt tccagtaacc gggcatgttc atcatcagta 5880
   acccgtatcg tgagcatcct aagagaccag ggcggcgtag gtatggcggt caacaaatgg 5940
   gagtgttgca aggtcattgg cccgtacaag tagtagtcat tgggcatagc actcgtagga 6000
   ctctcgtttc atcggtatca ttacccccat gaacagaaat cccccttaca cggaggcatc 6060
   agtgaccaaa caggaaaaaa ccgcccttaa catggcccgc gagagcaaag tagccatagt 6120
   aatgggggta cttgtcttta gggggaatgt gcctccgtag tcactggttt gtcctttttt 6180
   ggcgggaatt gtaccgggcg tttatcagaa gccagacatt aacgcttctg gagaaactca 6240
   acgagctgga cgcggatgaa caggcagaca tctgtgaatc gcttcacgac cacgctgatg 6300
   aaatagtctt cggtctgtaa ttgcgaagac ctctttgagt tgctcgacct gcgcctactt 6360
   gtccgtctgt agacacttag cgaagtgctg gtgcgactac agctttaccg cagctgcctc 6420
   gcgcgtttcg gtgatgacgg tgaaaacctc tgacacatgc agctcccgga gacggtcaca 6480
   gcttgtctgt aagcggatgc tcgaaatggc gtcgacggag cgcgcaaagc cactactgcc 6540
   acttttggag actgtgtacg tcgagggcct ctgccagtgt cgaacagaca ttcgcctacg 6600
   cgggagcaga caagcccgtc agggcgcgtc agcgggtgtt ggcgggtgtc ggggcgcagc 6660
   catgacccag tcacgtagcg atagcggagt gtatactggc gccctcgtct gttcgggcag 6720
   tcccgcgcag tcgcccacaa ccgcccacag ccccgcgtcg gtactgggtc agtgcatcgc 6780
   tatcgcctca catatgaccg ttaactatgc ggcatcagag cagattgtac tgagagtgca 6840
   ccatatatgc ggtgtgaaat accgcacaga tgcgtaagga gaaaataccg catcaggcgc 6900
   aattgatacg ccgtagtctc gtctaacatg actctcacgt ggtatatacg ccacacttta 6960
   tggcgtgtct acgcattcct cttttatggc gtagtccgcg tcttccgctt cctcgctcac 7020
   tgactcgctg cgctcggtcg ttcggctgcg gcgagcggta tcagctcact caaaggcggt 7080
   aatacggtta tccacagaat agaaggcgaa ggagcgagtg actgagcgac gcgagccagc 7140
   aagccgacgc cgctcgccat agtcgagtga gtttccgcca ttatgccaat aggtgtctta 7200
   caggggataa cgcaggaaag aacatgtgag caaaaggcca gcaaaaggcc aggaaccgta 7260
   aaaaggccgc gttgctggcg tttttccata ggctccgccc gtcccctatt gcgtcctttc 7320
   ttgtacactc gttttccggt cgttttccgg tccttggcat ttttccggcg caacgaccgc 7380
   aaaaaggtat ccgaggcggg ccctgacgag catcacaaaa atcgacgctc aagtcagagg 7440
   tggcgaaacc cgacaggact ataaagatac caggcgtttc cccctggaag ctccctcgtg 7500
   gggactgctc gtagtgtttt tagctgcgag ttcagtctcc accgctttgg gctgtcctga 7560
   tatttctatg gtccgcaaag ggggaccttc gagggagcac cgctctcctg ttccgaccct 7620
   gccgcttacc ggatacctgt ccgcctttct cccttcggga agcgtggcgc tttctcatag 7680
   ctcacgctgt aggtatctca gcgagaggac aaggctggga cggcgaatgg cctatggaca 7740
   ggcggaaaga gggaagccct tcgcaccgcg aaagagtatc gagtgcgaca tccatagagt 7800
   gttcggtgta ggtcgttcgc tccaagctgg gctgtgtgca cgaacccccc gttcagcccg 7860
   accgctgcgc cttatccggt aactatcgtc ttgagtccaa caagccacat ccagcaagcg 7920
   aggttcgacc cgacacacgt gcttgggggg caagtcgggc tggcgacgcg gaataggcca 7980
   ttgatagcag aactcaggtt cccggtaaga cacgacttat cgccactggc agcagccact 8040
   ggtaacagga ttagcagagc gaggtatgta ggcggtgcta cagagttctt gaagtggtgg 8100
   gggccattct gtgctgaata gcggtgaccg tcgtcggtga ccattgtcct aatcgtctcg 8160
   ctccatacat ccgccacgat gtctcaagaa cttcaccacc cctaactacg gctacactag 8220
   aaggacagta tttggtatct gcgctctgct gaagccagtt accttcggaa aaagagttgg 8280
   tagctcttga tccggcaaac ggattgatgc cgatgtgatc ttcctgtcat aaaccataga 8340
   cgcgagacga cttcggtcaa tggaagcctt tttctcaacc atcgagaact aggccgtttg 8400
   aaaccaccgc tggtagcggt ggtttttttg tttgcaagca gcagattacg cgcagaaaaa 8460
   aaggatctca agaagatcct ttgatctttt ctacggggtc tttggtggcg accatcgcca 8520
   ccaaaaaaac aaacgttcgt cgtctaatgc gcgtcttttt ttcctagagt tcttctagga 8580
   aactagaaaa gatgccccag tgacgctcag tggaacgaaa actcacgtta agggattttg 8640
   gtcatgagat tatcaaaaag gatcttcacc tagatccttt taaattaaaa atgaagtttt 8700
   actgcgagtc accttgcttt tgagtgcaat tccctaaaac cagtactcta atagtttttc 8760
   ctagaagtgg atctaggaaa atttaatttt tacttcaaaa aaatcaatct aaagtatata 8820
   tgagtaaact tggtctgaca gttaccaatg cttaatcagt gaggcaccta tctcagcgat 8880
   ctgtctattt cgttcatcca tttagttaga tttcatatat actcatttga accagactgt 8940
   caatggttac gaattagtca ctccgtggat agagtcgcta gacagataaa gcaagtaggt 9000
   tagttgcctg actccccgtc gtgtagataa ctacgatacg ggagggctta ccatctggcc 9060
   ccagtgctgc aatgataccg cgagacccac gctcaccggc atcaacggac tgaggggcag 9120
   cacatctatt gatgctatgc cctcccgaat ggtagaccgg ggtcacgacg ttactatggc 9180
   gctctgggtg cgagtggccg tccagattta tcagcaataa accagccagc cggaagggcc 9240
   gagcgcagaa gtggtcctgc aactttatcc gcctccatcc agtctattaa ttgttgccgg 9300
   aggtctaaat agtcgttatt tggtcggtcg gccttcccgg ctcgcgtctt caccaggacg 9360
   ttgaaatagg cggaggtagg tcagataatt aacaacggcc gaagctagag taagtagttc 9420
   gccagttaat agtttgcgca acgttgttgc cattgctgca ggcatcgtgg tgtcacgctc 9480
   gtcgtttggt atggcttcat cttcgatctc attcatcaag cggtcaatta tcaaacgcgt 9540
   tgcaacaacg gtaacgacgt ccgtagcacc acagtgcgag cagcaaacca taccgaagta 9600
   tcagctccgg ttcccaacga tcaaggcgag ttacatgatc ccccatgttg tgcaaaaaag 9660
   cggttagctc cttcggtcct ccgatcgttg tcagaagtaa agtcgaggcc aagggttgct 9720
   agttccgctc aatgtactag ggggtacaac acgttttttc gccaatcgag gaagccagga 9780
   ggctagcaac agtcttcatt gttggccgca gtgttatcac tcatggttat ggcagcactg 9840
   cataattctc ttactgtcat gccatccgta agatgctttt ctgtgactgg tgagtactca 9900
   caaccggcgt cacaatagtg agtaccaata ccgtcgtgac gtattaagag aatgacagta 9960
   cggtaggcat tctacgaaaa gacactgacc actcatgagt accaagtcat tctgagaata 10020
   gtgtatgcgg cgaccgagtt gctcttgccc ggcgtcaata cgggataata ccgcgccaca 10080
   tagcagaact ttaaaagtgc tggttcagta agactcttat cacatacgcc gctggctcaa 10140
   cgagaacggg ccgcagttat gccctattat ggcgcggtgt atcgtcttga aattttcacg 10200
   tcatcattgg aaaacgttct tcggggcgaa aactctcaag gatcttaccg ctgttgagat 10260
   ccagttcgat gtaacccact cgtgcaccca actgatcttc agtagtaacc ttttgcaaga 10320
   agccccgctt ttgagagttc ctagaatggc gacaactcta ggtcaagcta cattgggtga 10380
   gcacgtgggt tgactagaag agcatctttt actttcacca gcgtttctgg gtgagcaaaa 10440
   acaggaaggc aaaatgccgc aaaaaaggga ataagggcga cacggaaatg ttgaatactc 10500
   tcgtagaaaa tgaaagtggt cgcaaagacc cactcgtttt tgtccttccg ttttacggcg 10560
   ttttttccct tattcccgct gtgcctttac aacttatgag atactcttcc tttttcaata 10620
   ttattgaagc atttatcagg gttattgtct catgagcgga tacatatttg aatgtattta 10680
   gaaaaataaa caaatagggg tatgagaagg aaaaagttat aataacttcg taaatagtcc 10740
   caataacaga gtactcgcct atgtataaac ttacataaat ctttttattt gtttatcccc 10800
   ttccgcgcac atttccccga aaagtgccac ctgaaattgt aaacgttaat attttgttaa 10860
   aattcgcgtt aaatttttgt taaatcagct cattttttaa aaggcgcgtg taaaggggct 10920
   tttcacggtg gactttaaca tttgcaatta taaaacaatt ttaagcgcaa tttaaaaaca 10980
   atttagtcga gtaaaaaatt ccaataggcc gaaatcggca aaatccctta taaatcaaaa 11040
   gaatagaccg agatagggtt gagtgttgtt ccagtttgga acaagagtcc actattaaag 11100
   ggttatccgg ctttagccgt tttagggaat atttagtttt cttatctggc tctatcccaa 11160
   ctcacaacaa ggtcaaacct tgttctcagg tgataatttc aacgtggact ccaacgtcaa 11220
   agggcgaaaa accgtctatc agggcgatgg cccactacgt gaaccatcac cctaatcaag 11280
   ttttttgggg tcgaggtgcc ttgcacctga ggttgcagtt tcccgctttt tggcagatag 11340
   tcccgctacc gggtgatgca cttggtagtg ggattagttc aaaaaacccc agctccacgg 11400
   gtaaagcact aaatcggaac cctaaaggga gcccccgatt tagagcttga cggggaaagc 11460
   cggcgaacgt ggcgagaaag gaagggaaga aagcgaaagg catttcgtga tttagccttg 11520
   ggatttccct cgggggctaa atctcgaact gcccctttcg gccgcttgca ccgctctttc 11580
   cttcccttct ttcgctttcc agcgggcgct agggcgctgg caagtgtagc ggtcacgctg 11640
   cgcgtaacca ccacacccgc cgcgcttaat gcgccgctac agggcgcgtc ccattcgcca 11700
   tcgcccgcga tcccgcgacc gttcacatcg ccagtgcgac gcgcattggt ggtgtgggcg 11760
   gcgcgaatta cgcggcgatg tcccgcgcag ggtaagcggt                      11800
<210> 36
   <211> 327
   <212> ADN
   <213> Séquence artificielle
<220>
   <223> Description de la séquence artificielle: ADN codant pour la thiorédoxine dans le plasmide pET32a+
<400> 36
   atgagcgata aaattattca cctgactgac gacagttttg acacggatgt actcaaagcg 60
   gacggggcga tcctcgtcga tttctgggca gagtggtgcg gtccgtgcaa aatgatcgcc 120
   ccgattctgg atgaaatcgc tgacgaatat cagggcaaac tgaccgttgc aaaactgaac 180
   atcgatcaaa accctggcac tgcgccgaaa tatggcatcc gtggtatccc gactctgctg 240
   ctgttcaaaa acggtgaagt ggcggcaacc aaagtgggtg cactgtctaa aggtcagttg 300
   aaagagttcc tcgacgctaa cctggcc                                    327
<210> 37
   <211> 109
   <212> PRT
   <213> Séquence artificielle
<220>
   <223> Description de la séquence artificielle: protéine thiorédoxine codée par le plasmide pET32a+
<400> 37
   Met Ser Asp Lys Ile Ile His Leu Thr Asp Asp Ser Phe Asp Thr Asp
    1               5                  10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala
               100                 105
<210> 38
   <211> 27
   <212> ADN
   <213> Séquence artificielle
<220>
   <223> Description de la séquence artificielle: oligonucléotide amont pour amplification des ADNs codant pour TME1 et TME2 à partir des constructions réalisées dans pGEXKT, et insertion dans pET32a+
<400> 38
   gtgatatctg atctgtctgg tggtggt 27
<210> 39
   <211> 23
   <212> ADN
   <213> Séquence artificielle
<220>
   <223> Description de la séquence artificielle: oligonucléotide aval pour amplification de l'ADN codant pour TME1 à partir de la construction réalisée dans pGEXKT, et insertion dans pET32a+
<400> 39
   gaattcctaa gcttcagcct gag 23
<210> 40
   <211> 30
   <212> ADN
   <213> Séquence artificielle
<220>
   <223> Description de la séquence artificielle: oligonucléotide aval pour amplification des ADNs codant pour TME2 sauvage et mutants à partir des constructions réalisées dans pGEXKT, et insertion dans pET32a
<400> 40
   gaattcttaa gcttcagcct gagagatcag 30
<210> 41
   <211> 5918
   <212> ADN
   <213> Séquence artificielle
<220>
   <223> Description de la séquence artificielle: vecteur pET32a-DP-TME1 (brin complémentaire)
<400> 41
   atccggatat agttcctcct ttcagcaaaa aacccctcaa gacccgttta gaggccccaa 60
   ggggttatgc tagttattgc tcagcggtgg cagcagccaa ctcagcttcc tttcgggctt 120
   tgttagcagc cggatctcag tggtggtggt ggtggtgctc gagtgcggcc gcaagcttgt 180
   cgacggagct cgaattccta agcgtcaaca ccagcgaaca gcagcagaac aaccagaact 240
   ttagcccagt taccaaccat agagaagtaa gcgataccag ccagaacacc ccagtgagca 300
   ccagcgatcg ggtcggatcc acgcggaacc agaccaccac caccaccaga cagatccgat 360
   tttggagatc cagaaccaga accggccagg ttagcgtcga ggaactcttt caactgacct 420
   ttagacagtg cacccacttt ggttgccgcc acttcaccgt ttttgaacag cagcagagtc 480
   gggataccac ggatgccata tttcggcgca gtgccagggt tttgatcgat gttcagtttt 540
   gcaacggtca gtttgccctg atattcgtca gcgatttcat ccagaatcgg ggcgatcatt 600
   ttgcacggac cgcaccactc tgcccagaaa tcgacgagga tcgccccgtc cgctttgagt 660
   acatccgtgt caaaactgtc gtcagtcagg tgaataattt tatcgctcat atgtatatct 720
   ccttcttaaa gttaaacaaa attatttcta gaggggaatt gttatccgct cacaattccc 780
   ctatagtgag tcgtattaat ttcgcgggat cgagatcgat ctcgatcctc tacgccggac 840
   gcatcgtggc cggcatcacc ggcgccacag gtgcggttgc tggcgcctat atcgccgaca 900
   tcaccgatgg ggaagatcgg gctcgccact tcgggctcat gagcgcttgt ttcggcgtgg 960
   gtatggtggc aggccccgtg gccgggggac tgttgggcgc catctccttg catgcaccat 1020
   tccttgcggc ggcggtgctc aacggcctca acctactact gggctgcttc ctaatgcagg 1080
   agtcgcataa gggagagcgt cgagatcccg gacaccatcg aatggcgcaa aacctttcgc 1140
   ggtatggcat gatagcgccc ggaagagagt caattcaggg tggtgaatgt gaaaccagta 1200
   acgttatacg atgtcgcaga gtatgccggt gtctcttatc agaccgtttc ccgcgtggtg 1260
   aaccaggcca gccacgtttc tgcgaaaacg cgggaaaaag tggaagcggc gatggcggag 1320
   ctgaattaca ttcccaaccg cgtggcacaa caactggcgg gcaaacagtc gttgctgatt 1380
   ggcgttgcca cctccagtct ggccctgcac gcgccgtcgc aaattgtcgc ggcgattaaa 1440
   tctcgcgccg atcaactggg tgccagcgtg gtggtgtcga tggtagaacg aagcggcgtc 1500
   gaagcctgta aagcggcggt gcacaatctt ctcgcgcaac gcgtcagtgg gctgatcatt 1560
   aactatccgc tggatgacca ggatgccatt gctgtggaag ctgcctgcac taatgttccg 1620
   gcgttatttc ttgatgtctc tgaccagaca cccatcaaca gtattatttt ctcccatgaa 1680
   gacggtacgc gactgggcgt ggagcatctg gtcgcattgg gtcaccagca aatcgcgctg 1740
   ttagcgggcc cattaagttc tgtctcggcg cgtctgcgtc tggctggctg gcataaatat 1800
   ctcactcgca atcaaattca gccgatagcg gaacgggaag gcgactggag tgccatgtcc 1860
   ggttttcaac aaaccatgca aatgctgaat gagggcatcg ttcccactgc gatgctggtt 1920
   gccaacgatc agatggcgct gggcgcaatg cgcgccatta ccgagtccgg gctgcgcgtt 1980
   ggtgcggaca tctcggtagt gggatacgac gataccgaag acagctcatg ttatatcccg 2040
   ccgttaacca ccatcaaaca ggattttcgc ctgctggggc aaaccagcgt ggaccgcttg 2100
   ctgcaactct ctcagggcca ggcggtgaag ggcaatcagc tgttgcccgt ctcactggtg 2160
   aaaagaaaaa ccaccctggc gcccaatacg caaaccgcct ctccccgcgc gttggccgat 2220
   tcattaatgc agctggcacg acaggtttcc cgactggaaa gcgggcagtg agcgcaacgc 2280
   aattaatgta agttagctca ctcattaggc accgggatct cgaccgatgc ccttgagagc 2340
   cttcaaccca gtcagctcct tccggtgggc gcggggcatg actatcgtcg ccgcacttat 2400
   gactgtcttc tttatcatgc aactcgtagg acaggtgccg gcagcgctct gggtcatttt 2460
   cggcgaggac cgctttcgct ggagcgcgac gatgatcggc ctgtcgcttg cggtattcgg 2520
   aatcttgcac gccctcgctc aagccttcgt cactggtccc gccaccaaac gtttcggcga 2580
   gaagcaggcc attatcgccg gcatggcggc cccacgggtg cgcatgatcg tgctcctgtc 2640
   gttgaggacc cggctaggct ggcggggttg ccttactggt tagcagaatg aatcaccgat 2700
   acgcgagcga acgtgaagcg actgctgctg caaaacgtct gcgacctgag caacaacatg 2760
   aatggtcttc ggtttccgtg tttcgtaaag tctggaaacg cggaagtcag cgccctgcac 2820
   cattatgttc cggatctgca tcgcaggatg ctgctggcta ccctgtggaa cacctacatc 2880
   tgtattaacg aagcgctggc attgaccctg agtgattttt ctctggtccc gccgcatcca 2940
   taccgccagt tgtttaccct cacaacgttc cagtaaccgg gcatgttcat catcagtaac 3000
   ccgtatcgtg agcatcctct ctcgtttcat cggtatcatt acccccatga acagaaatcc 3060
   cccttacacg gaggcatcag tgaccaaaca ggaaaaaacc gcccttaaca tggcccgctt 3120
   tatcagaagc cagacattaa cgcttctgga gaaactcaac gagctggacg cggatgaaca 3180
   ggcagacatc tgtgaatcgc ttcacgacca cgctgatgag ctttaccgca gctgcctcgc 3240
   gcgtttcggt gatgacggtg aaaacctctg acacatgcag ctcccggaga cggtcacagc 3300
   ttgtctgtaa gcggatgccg ggagcagaca agcccgtcag ggcgcgtcag cgggtgttgg 3360
   cgggtgtcgg ggcgcagcca tgacccagtc acgtagcgat agcggagtgt atactggctt 3420
   aactatgcgg catcagagca gattgtactg agagtgcacc atatatgcgg tgtgaaatac 3480
   cgcacagatg cgtaaggaga aaataccgca tcaggcgctc ttccgcttcc tcgctcactg 3540
   actcgctgcg ctcggtcgtt cggctgcggc gagcggtatc agctcactca aaggcggtaa 3600
   tacggttatc cacagaatca ggggataacg caggaaagaa catgtgagca aaaggccagc 3660
   aaaaggccag gaaccgtaaa aaggccgcgt tgctggcgtt tttccatagg ctccgccccc 3720
   ctgacgagca tcacaaaaat cgacgctcaa gtcagaggtg gcgaaacccg acaggactat 3780
   aaagatacca ggcgtttccc cctggaagct ccctcgtgcg ctctcctgtt ccgaccctgc 3840
   cgcttaccgg atacctgtcc gcctttctcc cttcgggaag cgtggcgctt tctcatagct 3900
   cacgctgtag gtatctcagt tcggtgtagg tcgttcgctc caagctgggc tgtgtgcacg 3960
   aaccccccgt tcagcccgac cgctgcgcct tatccggtaa ctatcgtctt gagtccaacc 4020
   cggtaagaca cgacttatcg ccactggcag cagccactgg taacaggatt agcagagcga 4080
   ggtatgtagg cggtgctaca gagttcttga agtggtggcc taactacggc tacactagaa 4140
   ggacagtatt tggtatctgc gctctgctga agccagttac cttcggaaaa agagttggta 4200
   gctcttgatc cggcaaacaa accaccgctg gtagcggtgg tttttttgtt tgcaagcagc 4260
   agattacgcg cagaaaaaaa ggatctcaag aagatccttt gatcttttct acggggtctg 4320
   acgctcagtg gaacgaaaac tcacgttaag ggattttggt catgagatta tcaaaaagga 4380
   tcttcaccta gatcctttta aattaaaaat gaagttttaa atcaatctaa agtatatatg 4440
   agtaaacttg gtctgacagt taccaatgct taatcagtga ggcacctatc tcagcgatct 4500
   gtctatttcg ttcatccata gttgcctgac tccccgtcgt gtagataact acgatacggg 4560
   agggcttacc atctggcccc agtgctgcaa tgataccgcg agacccacgc tcaccggctc 4620
   cagatttatc agcaataaac cagccagccg gaagggccga gcgcagaagt ggtcctgcaa 4680
   ctttatccgc ctccatccag tctattaatt gttgccggga agctagagta agtagttcgc 4740
   cagttaatag tttgcgcaac gttgttgcca ttgctgcagg catcgtggtg tcacgctcgt 4800
   cgtttggtat ggcttcattc agctccggtt cccaacgatc aaggcgagtt acatgatccc 4860
   ccatgttgtg caaaaaagcg gttagctcct tcggtcctcc gatcgttgtc agaagtaagt 4920
   tggccgcagt gttatcactc atggttatgg cagcactgca taattctctt actgtcatgc 4980
   catccgtaag atgcttttct gtgactggtg agtactcaac caagtcattc tgagaatagt 5040
   gtatgcggcg accgagttgc tcttgcccgg cgtcaatacg ggataatacc gcgccacata 5100
   gcagaacttt aaaagtgctc atcattggaa aacgttcttc ggggcgaaaa ctctcaagga 5160
   tcttaccgct gttgagatcc agttcgatgt aacccactcg tgcacccaac tgatcttcag 5220
   catcttttac tttcaccagc gtttctgggt gagcaaaaac aggaaggcaa aatgccgcaa 5280
   aaaagggaat aagggcgaca cggaaatgtt gaatactcat actcttcctt tttcaatatt 5340
   attgaagcat ttatcagggt tattgtctca tgagcggata catatttgaa tgtatttaga 5400
   aaaataaaca aataggggtt ccgcgcacat ttccccgaaa agtgccacct gaaattgtaa 5460
   acgttaatat tttgttaaaa ttcgcgttaa atttttgtta aatcagctca ttttttaacc 5520
   aataggccga aatcggcaaa atcccttata aatcaaaaga atagaccgag atagggttga 5580
   gtgttgttcc agtttggaac aagagtccac tattaaagaa cgtggactcc aacgtcaaag 5640
   ggcgaaaaac cgtctatcag ggcgatggcc cactacgtga accatcaccc taatcaagtt 5700
   ttttggggtc gaggtgccgt aaagcactaa atcggaaccc taaagggagc ccccgattta 5760
   gagcttgacg gggaaagccg gcgaacgtgg cgagaaagga agggaagaaa gcgaaaggag 5820
   cgggcgctag ggcgctggca agtgtagcgg tcacgctgcg cgtaaccacc acacccgccg 5880
   cgcttaatgc gccgctacag ggcgcgtccc attcgcca                        5918
<210> 42
   <211> 513
   <212> ADN
   <213> Séquence artificielle
<220>
   <223> Description de la séquence artificielle: ADN codant pour la protéine de fusion TrX-DP-TME1 dans le vecteur pET32a-DP-TME1
<400> 42
   atgagcgata aaattattca cctgactgac gacagttttg acacggatgt actcaaagcg 60
   gacggggcga tcctcgtcga tttctgggca gagtggtgcg gtccgtgcaa aatgatcgcc 120
   ccgattctgg atgaaatcgc tgacgaatat cagggcaaac tgaccgttgc aaaactgaac 180
   atcgatcaaa accctggcac tgcgccgaaa tatggcatcc gtggtatccc gactctgctg 240
   ctgttcaaaa acggtgaagt ggcggcaacc aaagtgggtg cactgtctaa aggtcagttg 300
   aaagagttcc tcgacgctaa cctggccggt tctggttctg gatctccaaa atcggatctg 360
   tctggtggtg gtggtggtct ggttccgcgt ggatccgacc cgatcgctgg tgctcactgg 420
   ggtgttctgg ctggtatcgc ttacttctct atggttggta actgggctaa agttctggtt 480
   gttctgctgc tgttcgctgg tgttgacgct tag                             513
<210> 43
   <211> 170
   <212> PRT
   <213> Séquence artificielle
<220>
   <223> Description de la séquence artificielle: protéine de fusion TrX-DP-TME1 codée par le vecteur pET32a-DP-TME1
<400> 43
   Met Ser Asp Lys Ile Ile His Leu Thr Asp Asp Ser Phe Asp Thr Asp
    1               5                  10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly Ser Pro Lys Ser Asp Leu Ser Gly Gly Gly Gly Gly Leu Val
           115                 120                 125
   Pro Arg Gly Ser Asp Pro Ile Ala Gly Ala His Trp Gly Val Leu Ala
       130                 135                 140
   Gly Ile Ala Tyr Phe Ser Met Val Gly Asn Trp Ala Lys Val Leu Val
   145                 150                 155                 160
   Val Leu Leu Leu Phe Ala Gly Val Asp Ala
                   165                 170
<210> 44
   <211> 5918
   <212> ADN
   <213> Séquence artificielle
<220>
   <223> Description de la séquence artificielle: vecteur pET32a-DP-TME1_G354L (brin complémentaire)
<400> 44
   atccggatat agttcctcct ttcagcaaaa aacccctcaa gacccgttta gaggccccaa 60
   ggggttatgc tagttattgc tcagcggtgg cagcagccaa ctcagcttcc tttcgggctt 120
   tgttagcagc cggatctcag tggtggtggt ggtggtgctc gagtgcggcc gcaagcttgt 180
   cgacggagct cgaattccta agcgtcaaca ccagcgaaca gcagcagaac aaccagaact 240
   ttagcccagt taccaaccat agagaagtaa gcgataccag ccagaaccag ccagtgagca 300
   ccagcgatcg ggtcggatcc acgcggaacc agaccaccac caccaccaga cagatccgat 360
   tttggagatc cagaaccaga accggccagg ttagcgtcga ggaactcttt caactgacct 420
   ttagacagtg cacccacttt ggttgccgcc acttcaccgt ttttgaacag cagcagagtc 480
   gggataccac ggatgccata tttcggcgca gtgccagggt tttgatcgat gttcagtttt 540
   gcaacggtca gtttgccctg atattcgtca gcgatttcat ccagaatcgg ggcgatcatt 600
   ttgcacggac cgcaccactc tgcccagaaa tcgacgagga tcgccccgtc cgctttgagt 660
   acatccgtgt caaaactgtc gtcagtcagg tgaataattt tatcgctcat atgtatatct 720
   ccttcttaaa gttaaacaaa attatttcta gaggggaatt gttatccgct cacaattccc 780
   ctatagtgag tcgtattaat ttcgcgggat cgagatcgat ctcgatcctc tacgccggac 840
   gcatcgtggc cggcatcacc ggcgccacag gtgcggttgc tggcgcctat atcgccgaca 900
   tcaccgatgg ggaagatcgg gctcgccact tcgggctcat gagcgcttgt ttcggcgtgg 960
   gtatggtggc aggccccgtg gccgggggac tgttgggcgc catctccttg catgcaccat 1020
   tccttgcggc ggcggtgctc aacggcctca acctactact gggctgcttc ctaatgcagg 1080
   agtcgcataa gggagagcgt cgagatcccg gacaccatcg aatggcgcaa aacctttcgc 1140
   ggtatggcat gatagcgccc ggaagagagt caattcaggg tggtgaatgt gaaaccagta 1200
   acgttatacg atgtcgcaga gtatgccggt gtctcttatc agaccgtttc ccgcgtggtg 1260
   aaccaggcca gccacgtttc tgcgaaaacg cgggaaaaag tggaagcggc gatggcggag 1320
   ctgaattaca ttcccaaccg cgtggcacaa caactggcgg gcaaacagtc gttgctgatt 1380
   ggcgttgcca cctccagtct ggccctgcac gcgccgtcgc aaattgtcgc ggcgattaaa 1440
   tctcgcgccg atcaactggg tgccagcgtg gtggtgtcga tggtagaacg aagcggcgtc 1500
   gaagcctgta aagcggcggt gcacaatctt ctcgcgcaac gcgtcagtgg gctgatcatt 1560
   aactatccgc tggatgacca ggatgccatt gctgtggaag ctgcctgcac taatgttccg 1620
   gcgttatttc ttgatgtctc tgaccagaca cccatcaaca gtattatttt ctcccatgaa 1680
   gacggtacgc gactgggcgt ggagcatctg gtcgcattgg gtcaccagca aatcgcgctg 1740
   ttagcgggcc cattaagttc tgtctcggcg cgtctgcgtc tggctggctg gcataaatat 1800
   ctcactcgca atcaaattca gccgatagcg gaacgggaag gcgactggag tgccatgtcc 1860
   ggttttcaac aaaccatgca aatgctgaat gagggcatcg ttcccactgc gatgctggtt 1920
   gccaacgatc agatggcgct gggcgcaatg cgcgccatta ccgagtccgg gctgcgcgtt 1980
   ggtgcggaca tctcggtagt gggatacgac gataccgaag acagctcatg ttatatcccg 2040
   ccgttaacca ccatcaaaca ggattttcgc ctgctggggc aaaccagcgt ggaccgcttg 2100
   ctgcaactct ctcagggcca ggcggtgaag ggcaatcagc tgttgcccgt ctcactggtg 2160
   aaaagaaaaa ccaccctggc gcccaatacg caaaccgcct ctccccgcgc gttggccgat 2220
   tcattaatgc agctggcacg acaggtttcc cgactggaaa gcgggcagtg agcgcaacgc 2280
   aattaatgta agttagctca ctcattaggc accgggatct cgaccgatgc ccttgagagc 2340
   cttcaaccca gtcagctcct tccggtgggc gcggggcatg actatcgtcg ccgcacttat 2400
   gactgtcttc tttatcatgc aactcgtagg acaggtgccg gcagcgctct gggtcatttt 2460
   cggcgaggac cgctttcgct ggagcgcgac gatgatcggc ctgtcgcttg cggtattcgg 2520
   aatcttgcac gccctcgctc aagccttcgt cactggtccc gccaccaaac gtttcggcga 2580
   gaagcaggcc attatcgccg gcatggcggc cccacgggtg cgcatgatcg tgctcctgtc 2640
   gttgaggacc cggctaggct ggcggggttg ccttactggt tagcagaatg aatcaccgat 2700
   acgcgagcga acgtgaagcg actgctgctg caaaacgtct gcgacctgag caacaacatg 2760
   aatggtcttc ggtttccgtg tttcgtaaag tctggaaacg cggaagtcag cgccctgcac 2820
   cattatgttc cggatctgca tcgcaggatg ctgctggcta ccctgtggaa cacctacatc 2880
   tgtattaacg aagcgctggc attgaccctg agtgattttt ctctggtccc gccgcatcca 2940
   taccgccagt tgtttaccct cacaacgttc cagtaaccgg gcatgttcat catcagtaac 3000
   ccgtatcgtg agcatcctct ctcgtttcat cggtatcatt acccccatga acagaaatcc 3060
   cccttacacg gaggcatcag tgaccaaaca ggaaaaaacc gcccttaaca tggcccgctt 3120
   tatcagaagc cagacattaa cgcttctgga gaaactcaac gagctggacg cggatgaaca 3180
   ggcagacatc tgtgaatcgc ttcacgacca cgctgatgag ctttaccgca gctgcctcgc 3240
   gcgtttcggt gatgacggtg aaaacctctg acacatgcag ctcccggaga cggtcacagc 3300
   ttgtctgtaa gcggatgccg ggagcagaca agcccgtcag ggcgcgtcag cgggtgttgg 3360
   cgggtgtcgg ggcgcagcca tgacccagtc acgtagcgat agcggagtgt atactggctt 3420
   aactatgcgg catcagagca gattgtactg agagtgcacc atatatgcgg tgtgaaatac 3480
   cgcacagatg cgtaaggaga aaataccgca tcaggcgctc ttccgcttcc tcgctcactg 3540
   actcgctgcg ctcggtcgtt cggctgcggc gagcggtatc agctcactca aaggcggtaa 3600
   tacggttatc cacagaatca ggggataacg caggaaagaa catgtgagca aaaggccagc 3660
   aaaaggccag gaaccgtaaa aaggccgcgt tgctggcgtt tttccatagg ctccgccccc 3720
   ctgacgagca tcacaaaaat cgacgctcaa gtcagaggtg gcgaaacccg acaggactat 3780
   aaagatacca ggcgtttccc cctggaagct ccctcgtgcg ctctcctgtt ccgaccctgc 3840
   cgcttaccgg atacctgtcc gcctttctcc cttcgggaag cgtggcgctt tctcatagct 3900
   cacgctgtag gtatctcagt tcggtgtagg tcgttcgctc caagctgggc tgtgtgcacg 3960
   aaccccccgt tcagcccgac cgctgcgcct tatccggtaa ctatcgtctt gagtccaacc 4020
   cggtaagaca cgacttatcg ccactggcag cagccactgg taacaggatt agcagagcga 4080
   ggtatgtagg cggtgctaca gagttcttga agtggtggcc taactacggc tacactagaa 4140
   ggacagtatt tggtatctgc gctctgctga agccagttac cttcggaaaa agagttggta 4200
   gctcttgatc cggcaaacaa accaccgctg gtagcggtgg tttttttgtt tgcaagcagc 4260
   agattacgcg cagaaaaaaa ggatctcaag aagatccttt gatcttttct acggggtctg 4320
   acgctcagtg gaacgaaaac tcacgttaag ggattttggt catgagatta tcaaaaagga 4380
   tcttcaccta gatcctttta aattaaaaat gaagttttaa atcaatctaa agtatatatg 4440
   agtaaacttg gtctgacagt taccaatgct taatcagtga ggcacctatc tcagcgatct 4500
   gtctatttcg ttcatccata gttgcctgac tccccgtcgt gtagataact acgatacggg 4560
   agggcttacc atctggcccc agtgctgcaa tgataccgcg agacccacgc tcaccggctc 4620
   cagatttatc agcaataaac cagccagccg gaagggccga gcgcagaagt ggtcctgcaa 4680
   ctttatccgc ctccatccag tctattaatt gttgccggga agctagagta agtagttcgc 4740
   cagttaatag tttgcgcaac gttgttgcca ttgctgcagg catcgtggtg tcacgctcgt 4800
   cgtttggtat ggcttcattc agctccggtt cccaacgatc aaggcgagtt acatgatccc 4860
   ccatgttgtg caaaaaagcg gttagctcct tcggtcctcc gatcgttgtc agaagtaagt 4920
   tggccgcagt gttatcactc atggttatgg cagcactgca taattctctt actgtcatgc 4980
   catccgtaag atgcttttct gtgactggtg agtactcaac caagtcattc tgagaatagt 5040
   gtatgcggcg accgagttgc tcttgcccgg cgtcaatacg ggataatacc gcgccacata 5100
   gcagaacttt aaaagtgctc atcattggaa aacgttcttc ggggcgaaaa ctctcaagga 5160
   tcttaccgct gttgagatcc agttcgatgt aacccactcg tgcacccaac tgatcttcag 5220
   catcttttac tttcaccagc gtttctgggt gagcaaaaac aggaaggcaa aatgccgcaa 5280
   aaaagggaat aagggcgaca cggaaatgtt gaatactcat actcttcctt tttcaatatt 5340
   attgaagcat ttatcagggt tattgtctca tgagcggata catatttgaa tgtatttaga 5400
   aaaataaaca aataggggtt ccgcgcacat ttccccgaaa agtgccacct gaaattgtaa 5460
   acgttaatat tttgttaaaa ttcgcgttaa atttttgtta aatcagctca ttttttaacc 5520
   aataggccga aatcggcaaa atcccttata aatcaaaaga atagaccgag atagggttga 5580
   gtgttgttcc agtttggaac aagagtccac tattaaagaa cgtggactcc aacgtcaaag 5640
   ggcgaaaaac cgtctatcag ggcgatggcc cactacgtga accatcaccc taatcaagtt 5700
   ttttggggtc gaggtgccgt aaagcactaa atcggaaccc taaagggagc cccgatttta 5760
   gagcttgacg gggaaagccg gcgaacgtgg cgagaaagga agggaagaaa gcgaaaggag 5820
   cgggcgctag ggcgctggca agtgtagcgg tcacgctgcg cgtaaccacc acacccgccg 5880
   cgcttaatgc gccgctacag ggcgcgtccc attcgcca                         5918
<210> 45
   <211> 513
   <212> ADN
   <213> Séquence artificielle
<220>
   <223> Description de la séquence artificielle: ADN codant pour la protéine de fusion TrX-DP-TME1_G354L dans le vecteur pET32a-DP-TME1_G354L
<400> 45
   atgagcgata aaattattca cctgactgac gacagttttg acacggatgt actcaaagcg 60
   gacggggcga tcctcgtcga tttctgggca gagtggtgcg gtccgtgcaa aatgatcgcc 120
   ccgattctgg atgaaatcgc tgacgaatat cagggcaaac tgaccgttgc aaaactgaac 180
   atcgatcaaa accctggcac tgcgccgaaa tatggcatcc gtggtatccc gactctgctg 240
   ctgttcaaaa acggtgaagt ggcggcaacc aaagtgggtg cactgtctaa aggtcagttg 300
   aaagagttcc tcgacgctaa cctggccggt tctggttctg gatctccaaa atcggatctg 360
   tctggtggtg gtggtggtct ggttccgcgt ggatccgacc cgatcgctgg tgctcactgg 420
   ctggttctgg ctggtatcgc ttacttctct atggttggta actgggctaa agttctggtt 480
   gttctgctgc tgttcgctgg tgttgacgct tag                               513
<210> 46
   <211> 170
   <212> PRT
   <213> Séquence artificielle
<220>
   <223> Description de la séquence artificielle: protéine de fusion TrX-DP-TME1_G354L codée par le vecteur pET32a-DP-TME1_G354L
<400> 46
   Met Ser Asp Lys Ile Ile His Leu Thr Asp Asp Ser Phe Asp Thr Asp
    1               5                  10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly Ser Pro Lys Ser Asp Leu Ser Gly Gly Gly Gly Gly Leu Val
           115                 120                 125
   Pro Arg Gly Ser Asp Pro Ile Ala Gly Ala His Trp Leu Val Leu Ala
       130                 135                 140
   Gly Ile Ala Tyr Phe Ser Met Val Gly Asn Trp Ala Lys Val Leu Val
   145                 150                 155                 160
   Val Leu Leu Leu Phe Ala Gly Val Asp Ala
                   165                 170
<210> 47
   <211> 5918
   <212> ADN
   <213> Séquence artificielle
<220>
   <223> Description de la séquence artificielle: vecteur pET32a-DP-TME1_358L (brin complémentaire)
<400> 47
   atccggatat agttcctcct ttcagcaaaa aacccctcaa gacccgttta gaggccccaa 60
   ggggttatgc tagttattgc tcagcggtgg cagcagccaa ctcagcttcc tttcgggctt 120
   tgttagcagc cggatctcag tggtggtggt ggtggtgctc gagtgcggcc gcaagcttgt 180
   cgacggagct cgaattccta agcgtcaaca ccagcgaaca gcagcagaac aaccagaact 240
   ttagcccagt taccaaccat agagaagtaa gcgatcagag ccagaacacc ccagtgagca 300
   ccagcgatcg ggtcggatcc acgcggaacc agaccaccac caccaccaga cagatccgat 360
   tttggagatc cagaaccaga accggccagg ttagcgtcga ggaactcttt caactgacct 420
   ttagacagtg cacccacttt ggttgccgcc acttcaccgt ttttgaacag cagcagagtc 480
   gggataccac ggatgccata tttcggcgca gtgccagggt tttgatcgat gttcagtttt 540
   gcaacggtca gtttgccctg atattcgtca gcgatttcat ccagaatcgg ggcgatcatt 600
   ttgcacggac cgcaccactc tgcccagaaa tcgacgagga tcgccccgtc cgctttgagt 660
   acatccgtgt caaaactgtc gtcagtcagg tgaataattt tatcgctcat atgtatatct 720
   ccttcttaaa gttaaacaaa attatttcta gaggggaatt gttatccgct cacaattccc 780
   ctatagtgag tcgtattaat ttcgcgggat cgagatcgat ctcgatcctc tacgccggac 840
   gcatcgtggc cggcatcacc ggcgccacag gtgcggttgc tggcgcctat atcgccgaca 900
   tcaccgatgg ggaagatcgg gctcgccact tcgggctcat gagcgcttgt ttcggcgtgg 960
   gtatggtggc aggccccgtg gccgggggac tgttgggcgc catctccttg catgcaccat 1020
   tccttgcggc ggcggtgctc aacggcctca acctactact gggctgcttc ctaatgcagg 1080
   agtcgcataa gggagagcgt cgagatcccg gacaccatcg aatggcgcaa aacctttcgc 1140
   ggtatggcat gatagcgccc ggaagagagt caattcaggg tggtgaatgt gaaaccagta 1200
   acgttatacg atgtcgcaga gtatgccggt gtctcttatc agaccgtttc ccgcgtggtg 1260
   aaccaggcca gccacgtttc tgcgaaaacg cgggaaaaag tggaagcggc gatggcggag 1320
   ctgaattaca ttcccaaccg cgtggcacaa caactggcgg gcaaacagtc gttgctgatt 1380
   ggcgttgcca cctccagtct ggccctgcac gcgccgtcgc aaattgtcgc ggcgattaaa 1440
   tctcgcgccg atcaactggg tgccagcgtg gtggtgtcga tggtagaacg aagcggcgtc 1500
   gaagcctgta aagcggcggt gcacaatctt ctcgcgcaac gcgtcagtgg gctgatcatt 1560
   aactatccgc tggatgacca ggatgccatt gctgtggaag ctgcctgcac taatgttccg 1620
   gcgttatttc ttgatgtctc tgaccagaca cccatcaaca gtattatttt ctcccatgaa 1680
   gacggtacgc gactgggcgt ggagcatctg gtcgcattgg gtcaccagca aatcgcgctg 1740
   ttagcgggcc cattaagttc tgtctcggcg cgtctgcgtc tggctggctg gcataaatat 1800
   ctcactcgca atcaaattca gccgatagcg gaacgggaag gcgactggag tgccatgtcc 1860
   ggttttcaac aaaccatgca aatgctgaat gagggcatcg ttcccactgc gatgctggtt 1920
   gccaacgatc agatggcgct gggcgcaatg cgcgccatta ccgagtccgg gctgcgcgtt 1980
   ggtgcggaca tctcggtagt gggatacgac gataccgaag acagctcatg ttatatcccg 2040
   ccgttaacca ccatcaaaca ggattttcgc ctgctggggc aaaccagcgt ggaccgcttg 2100
   ctgcaactct ctcagggcca ggcggtgaag ggcaatcagc tgttgcccgt ctcactggtg 2160
   aaaagaaaaa ccaccctggc gcccaatacg caaaccgcct ctccccgcgc gttggccgat 2220
   tcattaatgc agctggcacg acaggtttcc cgactggaaa gcgggcagtg agcgcaacgc 2280
   aattaatgta agttagctca ctcattaggc accgggatct cgaccgatgc ccttgagagc 2340
   cttcaaccca gtcagctcct tccggtgggc gcggggcatg actatcgtcg ccgcacttat 2400
   gactgtcttc tttatcatgc aactcgtagg acaggtgccg gcagcgctct gggtcatttt 2460
   cggcgaggac cgctttcgct ggagcgcgac gatgatcggc ctgtcgcttg cggtattcgg 2520
   aatcttgcac gccctcgctc aagccttcgt cactggtccc gccaccaaac gtttcggcga 2580
   gaagcaggcc attatcgccg gcatggcggc cccacgggtg cgcatgatcg tgctcctgtc 2640
   gttgaggacc cggctaggct ggcggggttg ccttactggt tagcagaatg aatcaccgat 2700
   acgcgagcga acgtgaagcg actgctgctg caaaacgtct gcgacctgag caacaacatg 2760
   aatggtcttc ggtttccgtg tttcgtaaag tctggaaacg cggaagtcag cgccctgcac 2820
   cattatgttc cggatctgca tcgcaggatg ctgctggcta ccctgtggaa cacctacatc 2880
   tgtattaacg aagcgctggc attgaccctg agtgattttt ctctggtccc gccgcatcca 2940
   taccgccagt tgtttaccct cacaacgttc cagtaaccgg gcatgttcat catcagtaac 3000
   ccgtatcgtg agcatcctct ctcgtttcat cggtatcatt acccccatga acagaaatcc 3060
   cccttacacg gaggcatcag tgaccaaaca ggaaaaaacc gcccttaaca tggcccgctt 3120
   tatcagaagc cagacattaa cgcttctgga gaaactcaac gagctggacg cggatgaaca 3180
   ggcagacatc tgtgaatcgc ttcacgacca cgctgatgag ctttaccgca gctgcctcgc 3240
   gcgtttcggt gatgacggtg aaaacctctg acacatgcag ctcccggaga cggtcacagc 3300
   ttgtctgtaa gcggatgccg ggagcagaca agcccgtcag ggcgcgtcag cgggtgttgg 3360
   cgggtgtcgg ggcgcagcca tgacccagtc acgtagcgat agcggagtgt atactggctt 3420
   aactatgcgg catcagagca gattgtactg agagtgcacc atatatgcgg tgtgaaatac 3480
   cgcacagatg cgtaaggaga aaataccgca tcaggcgctc ttccgcttcc tcgctcactg 3540
   actcgctgcg ctcggtcgtt cggctgcggc gagcggtatc agctcactca aaggcggtaa 3600
   tacggttatc cacagaatca ggggataacg caggaaagaa catgtgagca aaaggccagc 3660
   aaaaggccag gaaccgtaaa aaggccgcgt tgctggcgtt tttccatagg ctccgccccc 3720
   ctgacgagca tcacaaaaat cgacgctcaa gtcagaggtg gcgaaacccg acaggactat 3780
   aaagatacca ggcgtttccc cctggaagct ccctcgtgcg ctctcctgtt ccgaccctgc 3840
   cgcttaccgg atacctgtcc gcctttctcc cttcgggaag cgtggcgctt tctcatagct 3900
   cacgctgtag gtatctcagt tcggtgtagg tcgttcgctc caagctgggc tgtgtgcacg 3960
   aaccccccgt tcagcccgac cgctgcgcct tatccggtaa ctatcgtctt gagtccaacc 4020
   cggtaagaca cgacttatcg ccactggcag cagccactgg taacaggatt agcagagcga 4080
   ggtatgtagg cggtgctaca gagttcttga agtggtggcc taactacggc tacactagaa 4140
   ggacagtatt tggtatctgc gctctgctga agccagttac cttcggaaaa agagttggta 4200
   gctcttgatc cggcaaacaa accaccgctg gtagcggtgg tttttttgtt tgcaagcagc 4260
   agattacgcg cagaaaaaaa ggatctcaag aagatccttt gatcttttct acggggtctg 4320
   acgctcagtg gaacgaaaac tcacgttaag ggattttggt catgagatta tcaaaaagga 4380
   tcttcaccta gatcctttta aattaaaaat gaagttttaa atcaatctaa agtatatatg 4440
   agtaaacttg gtctgacagt taccaatgct taatcagtga ggcacctatc tcagcgatct 4500
   gtctatttcg ttcatccata gttgcctgac tccccgtcgt gtagataact acgatacggg 4560
   agggcttacc atctggcccc agtgctgcaa tgataccgcg agacccacgc tcaccggctc 4620
   cagatttatc agcaataaac cagccagccg gaagggccga gcgcagaagt ggtcctgcaa 4680
   ctttatccgc ctccatccag tctattaatt gttgccggga agctagagta agtagttcgc 4740
   cagttaatag tttgcgcaac gttgttgcca ttgctgcagg catcgtggtg tcacgctcgt 4800
   cgtttggtat ggcttcattc agctccggtt cccaacgatc aaggcgagtt acatgatccc 4860
   ccatgttgtg caaaaaagcg gttagctcct tcggtcctcc gatcgttgtc agaagtaagt 4920
   tggccgcagt gttatcactc atggttatgg cagcactgca taattctctt actgtcatgc 4980
   catccgtaag atgcttttct gtgactggtg agtactcaac caagtcattc tgagaatagt 5040
   gtatgcggcg accgagttgc tcttgcccgg cgtcaatacg ggataatacc gcgccacata 5100
   gcagaacttt aaaagtgctc atcattggaa aacgttcttc ggggcgaaaa ctctcaagga 5160
   tcttaccgct gttgagatcc agttcgatgt aacccactcg tgcacccaac tgatcttcag 5220
   catcttttac tttcaccagc gtttctgggt gagcaaaaac aggaaggcaa aatgccgcaa 5280
   aaaagggaat aagggcgaca cggaaatgtt gaatactcat actcttcctt tttcaatatt 5340
   attgaagcat ttatcagggt tattgtctca tgagcggata catatttgaa tgtatttaga 5400
   aaaataaaca aataggggtt ccgcgcacat ttccccgaaa agtgccacct gaaattgtaa 5460
   acgttaatat tttgttaaaa ttcgcgttaa atttttgtta aatcagctca ttttttaacc 5520
   aataggccga aatcggcaaa atcccttata aatcaaaaga atagaccgag atagggttga 5580
   gtgttgttcc agtttggaac aagagtccac tattaaagaa cgtggactcc aacgtcaaag 5640
   ggcgaaaaac cgtctatcag ggcgatggcc cactacgtga accatcaccc taatcaagtt 5700
   ttttggggtc gaggtgccgt aaagcactaa atcggaaccc taaagggagc ccccgattta 5760
   gagcttgacg gggaaagccg gcgaacgtgg cgagaaagga agggaagaaa gcgaaaggag 5820
   cgggcgctag ggcgctggca agtgtagcgg tcacgctgcg cgtaaccacc acacccgccg 5880
   cgcttaatgc gccgctacag ggcgcgtccc attcgcca                        5918
<210> 48
   <211> 513
   <212> ADN
   <213> Séquence artificielle
<220>
   <223> Description de la séquence artificielle: ADN codant pour la protéine de fusion TrX-DP-TME1_G358L dans le vecteur pET32a-DP-TME1_G358L
<400> 48
   atgagcgata aaattattca cctgactgac gacagttttg acacggatgt actcaaagcg 60
   gacggggcga tcctcgtcga tttctgggca gagtggtgcg gtccgtgcaa aatgatcgcc 120
   ccgattctgg atgaaatcgc tgacgaatat cagggcaaac tgaccgttgc aaaactgaac 180
   atcgatcaaa accctggcac tgcgccgaaa tatggcatcc gtggtatccc gactctgctg 240
   ctgttcaaaa acggtgaagt ggcggcaacc aaagtgggtg cactgtctaa aggtcagttg 300
   aaagagttcc tcgacgctaa cctggccggt tctggttctg gatctccaaa atcggatctg 360
   tctggtggtg gtggtggtct ggttccgcgt ggatccgacc cgatcgctgg tgctcactgg 420
   ggtgttctgg ctctgatcgc ttacttctct atggttggta actgggctaa agttctggtt 480
   gttctgctgc tgttcgctgg tgttgacgct tag                              513
<210> 49
   <211> 170
   <212> PRT
   <213> Séquence artificielle
<220>
   <223> Description de la séquence artificielle: protéine de fusion TrX-DP-TME1_G358L codée par le vecteur pET32a-DP-TME1_G358L
<400> 49
   Met Ser Asp Lys Ile Ile His Leu Thr Asp Asp Ser Phe Asp Thr Asp
    1               5                  10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly Ser Pro Lys Ser Asp Leu Ser Gly Gly Gly Gly Gly Leu Val
           115                 120                 125
   Pro Arg Gly Ser Asp Pro Ile Ala Gly Ala His Trp Gly Val Leu Ala
       130                 135                 140
   Leu Ile Ala Tyr Phe Ser Met Val Gly Asn Trp Ala Lys Val Leu Val
   145                 150                 155                 160
   Val Leu Leu Leu Phe Ala Gly Val Asp Ala
                   165                 170
<210> 50
   <211> 5918
   <212> ADN
   <213> Séquence artificielle
<220>
   <223> Description de la séquence artificielle: vecteur pET32a-DP-TME1_G354&G358L (brin complémentaire)
<400> 50
   atccggatat agttcctcct ttcagcaaaa aacccctcaa gacccgttta gaggccccaa 60
   ggggttatgc tagttattgc tcagcggtgg cagcagccaa ctcagcttcc tttcgggctt 120
   tgttagcagc cggatctcag tggtggtggt ggtggtgctc gagtgcggcc gcaagcttgt 180
   cgacggagct cgaattccta agcgtcaaca ccagcgaaca gcagcagaac aaccagaact 240
   ttagcccagt taccaaccat agagaagtaa gcgatcagag ccagaaccag ccagtgagca 300
   ccagcgatcg ggtcggatcc acgcggaacc agaccaccac caccaccaga cagatccgat 360
   tttggagatc cagaaccaga accggccagg ttagcgtcga ggaactcttt caactgacct 420
   ttagacagtg cacccacttt ggttgccgcc acttcaccgt ttttgaacag cagcagagtc 480
   gggataccac ggatgccata tttcggcgca gtgccagggt tttgatcgat gttcagtttt 540
   gcaacggtca gtttgccctg atattcgtca gcgatttcat ccagaatcgg ggcgatcatt 600
   ttgcacggac cgcaccactc tgcccagaaa tcgacgagga tcgccccgtc cgctttgagt 660
   acatccgtgt caaaactgtc gtcagtcagg tgaataattt tatcgctcat atgtatatct 720
   ccttcttaaa gttaaacaaa attatttcta gaggggaatt gttatccgct cacaattccc 780
   ctatagtgag tcgtattaat ttcgcgggat cgagatcgat ctcgatcctc tacgccggac 840
   gcatcgtggc cggcatcacc ggcgccacag gtgcggttgc tggcgcctat atcgccgaca 900
   tcaccgatgg ggaagatcgg gctcgccact tcgggctcat gagcgcttgt ttcggcgtgg 960
   gtatggtggc aggccccgtg gccgggggac tgttgggcgc catctccttg catgcaccat 1020
   tccttgcggc ggcggtgctc aacggcctca acctactact gggctgcttc ctaatgcagg 1080
   agtcgcataa gggagagcgt cgagatcccg gacaccatcg aatggcgcaa aacctttcgc 1140
   ggtatggcat gatagcgccc ggaagagagt caattcaggg tggtgaatgt gaaaccagta 1200
   acgttatacg atgtcgcaga gtatgccggt gtctcttatc agaccgtttc ccgcgtggtg 1260
   aaccaggcca gccacgtttc tgcgaaaacg cgggaaaaag tggaagcggc gatggcggag 1320
   ctgaattaca ttcccaaccg cgtggcacaa caactggcgg gcaaacagtc gttgctgatt 1380
   ggcgttgcca cctccagtct ggccctgcac gcgccgtcgc aaattgtcgc ggcgattaaa 1440
   tctcgcgccg atcaactggg tgccagcgtg gtggtgtcga tggtagaacg aagcggcgtc 1500
   gaagcctgta aagcggcggt gcacaatctt ctcgcgcaac gcgtcagtgg gctgatcatt 1560
   aactatccgc tggatgacca ggatgccatt gctgtggaag ctgcctgcac taatgttccg 1620
   gcgttatttc ttgatgtctc tgaccagaca cccatcaaca gtattatttt ctcccatgaa 1680
   gacggtacgc gactgggcgt ggagcatctg gtcgcattgg gtcaccagca aatcgcgctg 1740
   ttagcgggcc cattaagttc tgtctcggcg cgtctgcgtc tggctggctg gcataaatat 1800
   ctcactcgca atcaaattca gccgatagcg gaacgggaag gcgactggag tgccatgtcc 1860
   ggttttcaac aaaccatgca aatgctgaat gagggcatcg ttcccactgc gatgctggtt 1920
   gccaacgatc agatggcgct gggcgcaatg cgcgccatta ccgagtccgg gctgcgcgtt 1980
   ggtgcggaca tctcggtagt gggatacgac gataccgaag acagctcatg ttatatcccg 2040
   ccgttaacca ccatcaaaca ggattttcgc ctgctggggc aaaccagcgt ggaccgcttg 2100
   ctgcaactct ctcagggcca ggcggtgaag ggcaatcagc tgttgcccgt ctcactggtg 2160
   aaaagaaaaa ccaccctggc gcccaatacg caaaccgcct ctccccgcgc gttggccgat 2220
   tcattaatgc agctggcacg acaggtttcc cgactggaaa gcgggcagtg agcgcaacgc 2280
   aattaatgta agttagctca ctcattaggc accgggatct cgaccgatgc ccttgagagc 2340
   cttcaaccca gtcagctcct tccggtgggc gcggggcatg actatcgtcg ccgcacttat 2400
   gactgtcttc tttatcatgc aactcgtagg acaggtgccg gcagcgctct gggtcatttt 2460
   cggcgaggac cgctttcgct ggagcgcgac gatgatcggc ctgtcgcttg cggtattcgg 2520
   aatcttgcac gccctcgctc aagccttcgt cactggtccc gccaccaaac gtttcggcga 2580
   gaagcaggcc attatcgccg gcatggcggc cccacgggtg cgcatgatcg tgctcctgtc 2640
   gttgaggacc cggctaggct ggcggggttg ccttactggt tagcagaatg aatcaccgat 2700
   acgcgagcga acgtgaagcg actgctgctg caaaacgtct gcgacctgag caacaacatg 2760
   aatggtcttc ggtttccgtg tttcgtaaag tctggaaacg cggaagtcag cgccctgcac 2820
   cattatgttc cggatctgca tcgcaggatg ctgctggcta ccctgtggaa cacctacatc 2880
   tgtattaacg aagcgctggc attgaccctg agtgattttt ctctggtccc gccgcatcca 2940
   taccgccagt tgtttaccct cacaacgttc cagtaaccgg gcatgttcat catcagtaac 3000
   ccgtatcgtg agcatcctct ctcgtttcat cggtatcatt acccccatga acagaaatcc 3060
   cccttacacg gaggcatcag tgaccaaaca ggaaaaaacc gcccttaaca tggcccgctt 3120
   tatcagaagc cagacattaa cgcttctgga gaaactcaac gagctggacg cggatgaaca 3180
   ggcagacatc tgtgaatcgc ttcacgacca cgctgatgag ctttaccgca gctgcctcgc 3240
   gcgtttcggt gatgacggtg aaaacctctg acacatgcag ctcccggaga cggtcacagc 3300
   ttgtctgtaa gcggatgccg ggagcagaca agcccgtcag ggcgcgtcag cgggtgttgg 3360
   cgggtgtcgg ggcgcagcca tgacccagtc acgtagcgat agcggagtgt atactggctt 3420
   aactatgcgg catcagagca gattgtactg agagtgcacc atatatgcgg tgtgaaatac 3480
   cgcacagatg cgtaaggaga aaataccgca tcaggcgctc ttccgcttcc tcgctcactg 3540
   actcgctgcg ctcggtcgtt cggctgcggc gagcggtatc agctcactca aaggcggtaa 3600
   tacggttatc cacagaatca ggggataacg caggaaagaa catgtgagca aaaggccagc 3660
   aaaaggccag gaaccgtaaa aaggccgcgt tgctggcgtt tttccatagg ctccgccccc 3720
   ctgacgagca tcacaaaaat cgacgctcaa gtcagaggtg gcgaaacccg acaggactat 3780
   aaagatacca ggcgtttccc cctggaagct ccctcgtgcg ctctcctgtt ccgaccctgc 3840
   cgcttaccgg atacctgtcc gcctttctcc cttcgggaag cgtggcgctt tctcatagct 3900
   cacgctgtag gtatctcagt tcggtgtagg tcgttcgctc caagctgggc tgtgtgcacg 3960
   aaccccccgt tcagcccgac cgctgcgcct tatccggtaa ctatcgtctt gagtccaacc 4020
   cggtaagaca cgacttatcg ccactggcag cagccactgg taacaggatt agcagagcga 4080
   ggtatgtagg cggtgctaca gagttcttga agtggtggcc taactacggc tacactagaa 4140
   ggacagtatt tggtatctgc gctctgctga agccagttac cttcggaaaa agagttggta 4200
   gctcttgatc cggcaaacaa accaccgctg gtagcggtgg tttttttgtt tgcaagcagc 4260
   agattacgcg cagaaaaaaa ggatctcaag aagatccttt gatcttttct acggggtctg 4320
   acgctcagtg gaacgaaaac tcacgttaag ggattttggt catgagatta tcaaaaagga 4380
   tcttcaccta gatcctttta aattaaaaat gaagttttaa atcaatctaa agtatatatg 4440
   agtaaacttg gtctgacagt taccaatgct taatcagtga ggcacctatc tcagcgatct 4500
   gtctatttcg ttcatccata gttgcctgac tccccgtcgt gtagataact acgatacggg 4560
   agggcttacc atctggcccc agtgctgcaa tgataccgcg agacccacgc tcaccggctc 4620
   cagatttatc agcaataaac cagccagccg gaagggccga gcgcagaagt ggtcctgcaa 4680
   ctttatccgc ctccatccag tctattaatt gttgccggga agctagagta agtagttcgc 4740
   cagttaatag tttgcgcaac gttgttgcca ttgctgcagg catcgtggtg tcacgctcgt 4800
   cgtttggtat ggcttcattc agctccggtt cccaacgatc aaggcgagtt acatgatccc 4860
   ccatgttgtg caaaaaagcg gttagctcct tcggtcctcc gatcgttgtc agaagtaagt 4920
   tggccgcagt gttatcactc atggttatgg cagcactgca taattctctt actgtcatgc 4980
   catccgtaag atgcttttct gtgactggtg agtactcaac caagtcattc tgagaatagt 5040
   gtatgcggcg accgagttgc tcttgcccgg cgtcaatacg ggataatacc gcgccacata 5100
   gcagaacttt aaaagtgctc atcattggaa aacgttcttc ggggcgaaaa ctctcaagga 5160
   tcttaccgct gttgagatcc agttcgatgt aacccactcg tgcacccaac tgatcttcag 5220
   catcttttac tttcaccagc gtttctgggt gagcaaaaac aggaaggcaa aatgccgcaa 5280
   aaaagggaat aagggcgaca cggaaatgtt gaatactcat actcttcctt tttcaatatt 5340
   attgaagcat ttatcagggt tattgtctca tgagcggata catatttgaa tgtatttaga 5400
   aaaataaaca aataggggtt ccgcgcacat ttccccgaaa agtgccacct gaaattgtaa 5460
   acgttaatat tttgttaaaa ttcgcgttaa atttttgtta aatcagctca ttttttaacc 5520
   aataggccga aatcggcaaa atcccttata aatcaaaaga atagaccgag atagggttga 5580
   gtgttgttcc agtttggaac aagagtccac tattaaagaa cgtggactcc aacgtcaaag 5640
   ggcgaaaaac cgtctatcag ggcgatggcc cactacgtga accatcaccc taatcaagtt 5700
   ttttggggtc gaggtgccgt aaagcactaa atcggaaccc taaagggagc ccccgattta 5760
   gagcttgacg gggaaagccg gcgaacgtgg cgagaaagga agggaagaaa gcgaaaggag 5820
   cgggcgctag ggcgctggca agtgtagcgg tcacgctgcg cgtaaccacc acacccgccg 5880
   cgcttaatgc gccgctacag ggcgcgtccc attcgcca 5918
<210> 51
   <211> 513
   <212> ADN
   <213> Séquence artificielle
<220>
   <223> Description de la séquence artificielle: ADN codant pour la protéine de fusion TrX-DP-TME1_G354&G358L dans le vecteur pET32a-DP-TME1_G354&G358L
<400> 51
   atgagcgata aaattattca cctgactgac gacagttttg acacggatgt actcaaagcg 60
   gacggggcga tcctcgtcga tttctgggca gagtggtgcg gtccgtgcaa aatgatcgcc 120
   ccgattctgg atgaaatcgc tgacgaatat cagggcaaac tgaccgttgc aaaactgaac 180
   atcgatcaaa accctggcac tgcgccgaaa tatggcatcc gtggtatccc gactctgctg 240
   ctgttcaaaa acggtgaagt ggcggcaacc aaagtgggtg cactgtctaa aggtcagttg 300
   aaagagttcc tcgacgctaa cctggccggt tctggttctg gatctccaaa atcggatctg 360
   tctggtggtg gtggtggtct ggttccgcgt ggatccgacc cgatcgctgg tgctcactgg 420
   ctggttctgg ctctgatcgc ttacttctct atggttggta actgggctaa agttctggtt 480
   gttctgctgc tgttcgctgg tgttgacgct tag 513
<210> 52
   <211> 170
   <212> PRT
   <213> Séquence artificielle
<220>
   <223> Description de la séquence artificielle: protéine de fusion TrX-DP-TME1_G354&G358L codée par le vecteur pET32a-DP-TME_G354&G358L
<400> 52
   Met Ser Asp Lys Ile Ile His Leu Thr Asp Asp Ser Phe Asp Thr Asp
   1               5                   10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly Ser Pro Lys Ser Asp Leu Ser Gly Gly Gly Gly Gly Leu Val
           115                 120                 125
   Pro Arg Gly Ser Asp Pro Ile Ala Gly Ala His Trp Leu Val Leu Ala
       130                 135                 140
   Leu Ile Ala Tyr Phe Ser Met Val Gly Asn Trp Ala Lys Val Leu Val
   145                 150                 155                 160
   Val Leu Leu Leu Phe Ala Gly Val Asp Ala
                   165                 170
<210> 53
   <211> 5891
   <212> ADN
   <213> Séquence artificielle
<220>
   <223> Description de la séquence artificielle: vecteur pET32a-DP-TME2 (brin complémentaire)
<400> 53
   atccggatat agttcctcct ttcagcaaaa aacccctcaa gacccgttta gaggccccaa 60
   ggggttatgc tagttattgc tcagcggtgg cagcagccaa ctcagcttcc tttcgggctt 120
   tgttagcagc cggatctcag tggtggtggt ggtggtgctc gagtgcggcc gcaagcttgt 180
   cgacggagct cgaattccta agcttcagcc tgagagatca gcagcatcat ccacaggcaa 240
   gagcaaacac gagcgtcagc cagcagcagg aacagcagaa caacgtattc cgggtcggat 300
   ccacgcggaa ccagaccacc accaccacca gacagatcag atccagaacc agaaccggcc 360
   aggttagcgt cgaggaactc tttcaactga cctttagaca gtgcacccac tttggttgcc 420
   gccacttcac cgtttttgaa cagcagcaga gtcgggatac cacggatgcc atatttcggc 480
   gcagtgccag ggttttgatc gatgttcagt tttgcaacgg tcagtttgcc ctgatattcg 540
   tcagcgattt catccagaat cggggcgatc attttgcacg gaccgcacca ctctgcccag 600
   aaatcgacga ggatcgcccc gtccgctttg agtacatccg tgtcaaaact gtcgtcagtc 660
   aggtgaataa ttttatcgct catatgtata tctccttctt aaagttaaac aaaattattt 720
   ctagagggga attgttatcc gctcacaatt cccctatagt gagtcgtatt aatttcgcgg 780
   gatcgagatc gatctcgatc ctctacgccg gacgcatcgt ggccggcatc accggcgcca 840
   caggtgcggt tgctggcgcc tatatcgccg acatcaccga tggggaagat cgggctcgcc 900
   acttcgggct catgagcgct tgtttcggcg tgggtatggt ggcaggcccc gtggccgggg 960
   gactgttggg cgccatctcc ttgcatgcac cattccttgc ggcggcggtg ctcaacggcc 1020
   tcaacctact actgggctgc ttcctaatgc aggagtcgca taagggagag cgtcgagatc 1080
   ccggacacca tcgaatggcg caaaaccttt cgcggtatgg catgatagcg cccggaagag 1140
   agtcaattca gggtggtgaa tgtgaaacca gtaacgttat acgatgtcgc agagtatgcc 1200
   ggtgtctctt atcagaccgt ttcccgcgtg gtgaaccagg ccagccacgt ttctgcgaaa 1260
   acgcgggaaa aagtggaagc ggcgatggcg gagctgaatt acattcccaa ccgcgtggca 1320
   caacaactgg cgggcaaaca gtcgttgctg attggcgttg ccacctccag tctggccctg 1380
   cacgcgccgt cgcaaattgt cgcggcgatt aaatctcgcg ccgatcaact gggtgccagc 1440
   gtggtggtgt cgatggtaga acgaagcggc gtcgaagcct gtaaagcggc ggtgcacaat 1500
   cttctcgcgc aacgcgtcag tgggctgatc attaactatc cgctggatga ccaggatgcc 1560
   attgctgtgg aagctgcctg cactaatgtt ccggcgttat ttcttgatgt ctctgaccag 1620
   acacccatca acagtattat tttctcccat gaagacggta cgcgactggg cgtggagcat 1680
   ctggtcgcat tgggtcacca gcaaatcgcg ctgttagcgg gcccattaag ttctgtctcg 1740
   gcgcgtctgc gtctggctgg ctggcataaa tatctcactc gcaatcaaat tcagccgata 1800
   gcggaacggg aaggcgactg gagtgccatg tccggttttc aacaaaccat gcaaatgctg 1860
   aatgagggca tcgttcccac tgcgatgctg gttgccaacg atcagatggc gctgggcgca 1920
   atgcgcgcca ttaccgagtc cgggctgcgc gttggtgcgg acatctcggt agtgggatac 1980
   gacgataccg aagacagctc atgttatatc ccgccgttaa ccaccatcaa acaggatttt 2040
   cgcctgctgg ggcaaaccag cgtggaccgc ttgctgcaac tctctcaggg ccaggcggtg 2100
   aagggcaatc agctgttgcc cgtctcactg gtgaaaagaa aaaccaccct ggcgcccaat 2160
   acgcaaaccg cctctccccg cgcgttggcc gattcattaa tgcagctggc acgacaggtt 2220
   tcccgactgg aaagcgggca gtgagcgcaa cgcaattaat gtaagttagc tcactcatta 2280
   ggcaccggga tctcgaccga tgcccttgag agccttcaac ccagtcagct ccttccggtg 2340
   ggcgcggggc atgactatcg tcgccgcact tatgactgtc ttctttatca tgcaactcgt 2400
   aggacaggtg ccggcagcgc tctgggtcat tttcggcgag gaccgctttc gctggagcgc 2460
   gacgatgatc ggcctgtcgc ttgcggtatt cggaatcttg cacgccctcg ctcaagcctt 2520
   cgtcactggt cccgccacca aacgtttcgg cgagaagcag gccattatcg ccggcatggc 2580
   ggccccacgg gtgcgcatga tcgtgctcct gtcgttgagg acccggctag gctggcgggg 2640
   ttgccttact ggttagcaga atgaatcacc gatacgcgag cgaacgtgaa gcgactgctg 2700
   ctgcaaaacg tctgcgacct gagcaacaac atgaatggtc ttcggtttcc gtgtttcgta 2760
   aagtctggaa acgcggaagt cagcgccctg caccattatg ttccggatct gcatcgcagg 2820
   atgctgctgg ctaccctgtg gaacacctac atctgtatta acgaagcgct ggcattgacc 2880
   ctgagtgatt tttctctggt cccgccgcat ccataccgcc agttgtttac cctcacaacg 2940
   ttccagtaac cgggcatgtt catcatcagt aacccgtatc gtgagcatcc tctctcgttt 3000
   catcggtatc attaccccca tgaacagaaa tcccccttac acggaggcat cagtgaccaa 3060
   acaggaaaaa accgccctta acatggcccg ctttatcaga agccagacat taacgcttct 3120
   ggagaaactc aacgagctgg acgcggatga acaggcagac atctgtgaat cgcttcacga 3180
   ccacgctgat gagctttacc gcagctgcct cgcgcgtttc ggtgatgacg gtgaaaacct 3240
   ctgacacatg cagctcccgg agacggtcac agcttgtctg taagcggatg ccgggagcag 3300
   acaagcccgt cagggcgcgt cagcgggtgt tggcgggtgt cggggcgcag ccatgaccca 3360
   gtcacgtagc gatagcggag tgtatactgg cttaactatg cggcatcaga gcagattgta 3420
   ctgagagtgc accatatatg cggtgtgaaa taccgcacag atgcgtaagg agaaaatacc 3480
   gcatcaggcg ctcttccgct tcctcgctca ctgactcgct gcgctcggtc gttcggctgc 3540
   ggcgagcggt atcagctcac tcaaaggcgg taatacggtt atccacagaa tcaggggata 3600
   acgcaggaaa gaacatgtga gcaaaaggcc agcaaaaggc caggaaccgt aaaaaggccg 3660
   cgttgctggc gtttttccat aggctccgcc cccctgacga gcatcacaaa aatcgacgct 3720
   caagtcagag gtggcgaaac ccgacaggac tataaagata ccaggcgttt ccccctggaa 3780
   gctccctcgt gcgctctcct gttccgaccc tgccgcttac cggatacctg tccgcctttc 3840
   tcccttcggg aagcgtggcg ctttctcata gctcacgctg taggtatctc agttcggtgt 3900
   aggtcgttcg ctccaagctg ggctgtgtgc acgaaccccc cgttcagccc gaccgctgcg 3960
   ccttatccgg taactatcgt cttgagtcca acccggtaag acacgactta tcgccactgg 4020
   cagcagccac tggtaacagg attagcagag cgaggtatgt aggcggtgct acagagttct 4080
   tgaagtggtg gcctaactac ggctacacta gaaggacagt atttggtatc tgcgctctgc 4140
   tgaagccagt taccttcgga aaaagagttg gtagctcttg atccggcaaa caaaccaccg 4200
   ctggtagcgg tggttttttt gtttgcaagc agcagattac gcgcagaaaa aaaggatctc 4260
   aagaagatcc tttgatcttt tctacggggt ctgacgctca gtggaacgaa aactcacgtt 4320
   aagggatttt ggtcatgaga ttatcaaaaa ggatcttcac ctagatcctt ttaaattaaa 4380
   aatgaagttt taaatcaatc taaagtatat atgagtaaac ttggtctgac agttaccaat 4440
   gcttaatcag tgaggcacct atctcagcga tctgtctatt tcgttcatcc atagttgcct 4500
   gactccccgt cgtgtagata actacgatac gggagggctt accatctggc cccagtgctg 4560
   caatgatacc gcgagaccca cgctcaccgg ctccagattt atcagcaata aaccagccag 4620
   ccggaagggc cgagcgcaga agtggtcctg caactttatc cgcctccatc cagtctatta 4680
   attgttgccg ggaagctaga gtaagtagtt cgccagttaa tagtttgcgc aacgttgttg 4740
   ccattgctgc aggcatcgtg gtgtcacgct cgtcgtttgg tatggcttca ttcagctccg 4800
   gttcccaacg atcaaggcga gttacatgat cccccatgtt gtgcaaaaaa gcggttagct 4860
   ccttcggtcc tccgatcgtt gtcagaagta agttggccgc agtgttatca ctcatggtta 4920
   tggcagcact gcataattct cttactgtca tgccatccgt aagatgcttt tctgtgactg 4980
   gtgagtactc aaccaagtca ttctgagaat agtgtatgcg gcgaccgagt tgctcttgcc 5040
   cggcgtcaat acgggataat accgcgccac atagcagaac tttaaaagtg ctcatcattg 5100
   gaaaacgttc ttcggggcga aaactctcaa ggatcttacc gctgttgaga tccagttcga 5160
   tgtaacccac tcgtgcaccc aactgatctt cagcatcttt tactttcacc agcgtttctg 5220
   ggtgagcaaa aacaggaagg caaaatgccg caaaaaaggg aataagggcg acacggaaat 5280
   gttgaatact catactcttc ctttttcaat attattgaag catttatcag ggttattgtc 5340
   tcatgagcgg atacatattt gaatgtattt agaaaaataa acaaataggg gttccgcgca 5400
   catttccccg aaaagtgcca cctgaaattg taaacgttaa tattttgtta aaattcgcgt 5460
   taaatttttg ttaaatcagc tcatttttta accaataggc cgaaatcggc aaaatccctt 5520
   ataaatcaaa agaatagacc gagatagggt tgagtgttgt tccagtttgg aacaagagtc 5580
   cactattaaa gaacgtggac tccaacgtca aagggcgaaa aaccgtctat cagggcgatg 5640
   gcccactacg tgaaccatca ccctaatcaa gtttttgggg gtcgaggtgc cgtaaagcac 5700
   taaatcggaa ccctaaaggg agcccccgat ttagagcttg acggggaaag ccggcgaacg 5760
   tggcgagaaa ggaagggaag aaagcgaaag gagcgggcgc tagggcgctg gcaagtgtag 5820
   cggtcacgct gcgcgtaacc accacacccg ccgcgcttaa tgcgccgcta cagggcgcgt 5880
   cccattcgcc a                                                      5891
<210> 54
   <211> 486
   <212> ADN
   <213> Séquence artificielle
<220>
   <223> Description de la séquence artificielle: ADN codant pour la protéine de fusion TrX-DP-TME2 dans le vecteur pET32a-DP-TME2
<400> 54
   atgagcgata aaattattca cctgactgac gacagttttg acacggatgt actcaaagcg 60
   gacggggcga tcctcgtcga tttctgggca gagtggtgcg gtccgtgcaa aatgatcgcc 120
   ccgattctgg atgaaatcgc tgacgaatat cagggcaaac tgaccgttgc aaaactgaac 180
   atcgatcaaa accctggcac tgcgccgaaa tatggcatcc gtggtatccc gactctgctg 240
   ctgttcaaaa acggtgaagt ggcggcaacc aaagtgggtg cactgtctaa aggtcagttg 300
   aaagagttcc tcgacgctaa cctggccggt tctggttctg gatctgatct gtctggtggt 360
   ggtggtggtc tggttccgcg tggatccgac ccggaatacg ttgttctgct gttcctgctg 420
   ctggctgacg ctcgtgtttg ctcttgcctg tggatgatgc tgctgatctc tcaggctgaa 480
   gcttag                                                            486
<210> 55
   <211> 161
   <212> PRT
   <213> Séquence artificielle
<220>
   <223> Description de la séquence artificielle: protéine de fusion TrX-DP-TME2 codée par le vecteur pET32a-DP-TME2
<400> 55
   Met Ser Asp Lys Ile Ile His Leu Thr Asp Asp Ser Phe Asp Thr Asp
    1               5                  10                  15
   Val Leu Lys Ala Asp Gly Ala Ile Leu Val Asp Phe Trp Ala Glu Trp
               20                  25                  30
   Cys Gly Pro Cys Lys Met Ile Ala Pro Ile Leu Asp Glu Ile Ala Asp
           35                  40                  45
   Glu Tyr Gln Gly Lys Leu Thr Val Ala Lys Leu Asn Ile Asp Gln Asn
       50                  55                  60
   Pro Gly Thr Ala Pro Lys Tyr Gly Ile Arg Gly Ile Pro Thr Leu Leu
   65                  70                  75                  80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly Ser Asp Leu Ser Gly Gly Gly Gly Gly Leu Val Pro Arg Gly
           115                 120                 125
   Ser Asp Pro Glu Tyr Val Val Leu Leu Phe Leu Leu Leu Ala Asp Ala
       130                 135                 140
   Arg Val Cys Ser Cys Leu Trp Met Met Leu Leu Ile Ser Gln Ala Glu
   145                 150                 155                 160
   Ala
<210> 56
   <211> 5891
   <212> ADN
   <213> Séquence artificielle
<220>
   <223> Description de la séquence artificielle: vecteur pET32a-DP-TME2_C731&733A (brin complémentaire)
<400> 56
   atccggatat agttcctcct ttcagcaaaa aacccctcaa gacccgttta gaggccccaa 60
   ggggttatgc tagttattgc tcagcggtgg cagcagccaa ctcagcttcc tttcgggctt 120
   tgttagcagc cggatctcag tggtggtggt ggtggtgctc gagtgcggcc gcaagcttgt 180
   cgacggagct cgaattccta agcttcagcc tgagagatca gcagcatcat ccacagagca 240
   gaagcaacac gagcgtcagc cagcagcagg aacagcagaa caacgtattc cgggtcggat 300
   ccacgcggaa ccagaccacc accaccacca gacagatcag atccagaacc agaaccggcc 360
   aggttagcgt cgaggaactc tttcaactga cctttagaca gtgcacccac tttggttgcc 420
   gccacttcac cgtttttgaa cagcagcaga gtcgggatac cacggatgcc atatttcggc 480
   gcagtgccag ggttttgatc gatgttcagt tttgcaacgg tcagtttgcc ctgatattcg 540
   tcagcgattt catccagaat cggggcgatc attttgcacg gaccgcacca ctctgcccag 600
   aaatcgacga ggatcgcccc gtccgctttg agtacatccg tgtcaaaact gtcgtcagtc 660
   aggtgaataa ttttatcgct catatgtata tctccttctt aaagttaaac aaaattattt 720
   ctagagggga attgttatcc gctcacaatt cccctatagt gagtcgtatt aatttcgcgg 780
   gatcgagatc gatctcgatc ctctacgccg gacgcatcgt ggccggcatc accggcgcca 840
   caggtgcggt tgctggcgcc tatatcgccg acatcaccga tggggaagat cgggctcgcc 900
   acttcgggct catgagcgct tgtttcggcg tgggtatggt ggcaggcccc gtggccgggg 960
   gactgttggg cgccatctcc ttgcatgcac cattccttgc ggcggcggtg ctcaacggcc 1020
   tcaacctact actgggctgc ttcctaatgc aggagtcgca taagggagag cgtcgagatc 1080
   ccggacacca tcgaatggcg caaaaccttt cgcggtatgg catgatagcg cccggaagag 1140
   agtcaattca gggtggtgaa tgtgaaacca gtaacgttat acgatgtcgc agagtatgcc 1200
   ggtgtctctt atcagaccgt ttcccgcgtg gtgaaccagg ccagccacgt ttctgcgaaa 1260
   acgcgggaaa aagtggaagc ggcgatggcg gagctgaatt acattcccaa ccgcgtggca 1320
   caacaactgg cgggcaaaca gtcgttgctg attggcgttg ccacctccag tctggccctg 1380
   cacgcgccgt cgcaaattgt cgcggcgatt aaatctcgcg ccgatcaact gggtgccagc 1440
   gtggtggtgt cgatggtaga acgaagcggc gtcgaagcct gtaaagcggc ggtgcacaat 1500
   cttctcgcgc aacgcgtcag tgggctgatc attaactatc cgctggatga ccaggatgcc 1560
   attgctgtgg aagctgcctg cactaatgtt ccggcgttat ttcttgatgt ctctgaccag 1620
   acacccatca acagtattat tttctcccat gaagacggta cgcgactggg cgtggagcat 1680
   ctggtcgcat tgggtcacca gcaaatcgcg ctgttagcgg gcccattaag ttctgtctcg 1740
   gcgcgtctgc gtctggctgg ctggcataaa tatctcactc gcaatcaaat tcagccgata 1800
   gcggaacggg aaggcgactg gagtgccatg tccggttttc aacaaaccat gcaaatgctg 1860
   aatgagggca tcgttcccac tgcgatgctg gttgccaacg atcagatggc gctgggcgca 1920
   atgcgcgcca ttaccgagtc cgggctgcgc gttggtgcgg acatctcggt agtgggatac 1980
   gacgataccg aagacagctc atgttatatc ccgccgttaa ccaccatcaa acaggatttt 2040
   cgcctgctgg ggcaaaccag cgtggaccgc ttgctgcaac tctctcaggg ccaggcggtg 2100
   aagggcaatc agctgttgcc cgtctcactg gtgaaaagaa aaaccaccct ggcgcccaat 2160
   acgcaaaccg cctctccccg cgcgttggcc gattcattaa tgcagctggc acgacaggtt 2220
   tcccgactgg aaagcgggca gtgagcgcaa cgcaattaat gtaagttagc tcactcatta 2280
   ggcaccggga tctcgaccga tgcccttgag agccttcaac ccagtcagct ccttccggtg 2340
   ggcgcggggc atgactatcg tcgccgcact tatgactgtc ttctttatca tgcaactcgt 2400
   aggacaggtg ccggcagcgc tctgggtcat tttcggcgag gaccgctttc gctggagcgc 2460
   gacgatgatc ggcctgtcgc ttgcggtatt cggaatcttg cacgccctcg ctcaagcctt 2520
   cgtcactggt cccgccacca aacgtttcgg cgagaagcag gccattatcg ccggcatggc 2580
   ggccccacgg gtgcgcatga tcgtgctcct gtcgttgagg acccggctag gctggcgggg 2640
   ttgccttact ggttagcaga atgaatcacc gatacgcgag cgaacgtgaa gcgactgctg 2700
   ctgcaaaacg tctgcgacct gagcaacaac atgaatggtc ttcggtttcc gtgtttcgta 2760
   aagtctggaa acgcggaagt cagcgccctg caccattatg ttccggatct gcatcgcagg 2820
   atgctgctgg ctaccctgtg gaacacctac atctgtatta acgaagcgct ggcattgacc 2880
   ctgagtgatt tttctctggt cccgccgcat ccataccgcc agttgtttac cctcacaacg 2940
   ttccagtaac cgggcatgtt catcatcagt aacccgtatc gtgagcatcc tctctcgttt 3000
   catcggtatc attaccccca tgaacagaaa tcccccttac acggaggcat cagtgaccaa 3060
   acaggaaaaa accgccctta acatggcccg ctttatcaga agccagacat taacgcttct 3120
   ggagaaactc aacgagctgg acgcggatga acaggcagac atctgtgaat cgcttcacga 3180
   ccacgctgat gagctttacc gcagctgcct cgcgcgtttc ggtgatgacg gtgaaaacct 3240
   ctgacacatg cagctcccgg agacggtcac agcttgtctg taagcggatg ccgggagcag 3300
   acaagcccgt cagggcgcgt cagcgggtgt tggcgggtgt cggggcgcag ccatgaccca 3360
   gtcacgtagc gatagcggag tgtatactgg cttaactatg cggcatcaga gcagattgta 3420
   ctgagagtgc accatatatg cggtgtgaaa taccgcacag atgcgtaagg agaaaatacc 3480
   gcatcaggcg ctcttccgct tcctcgctca ctgactcgct gcgctcggtc gttcggctgc 3540
   ggcgagcggt atcagctcac tcaaaggcgg taatacggtt atccacagaa tcaggggata 3600
   acgcaggaaa gaacatgtga gcaaaaggcc agcaaaaggc caggaaccgt aaaaaggccg 3660
   cgttgctggc gtttttccat aggctccgcc cccctgacga gcatcacaaa aatcgacgct 3720
   caagtcagag gtggcgaaac ccgacaggac tataaagata ccaggcgttt ccccctggaa 3780
   gctccctcgt gcgctctcct gttccgaccc tgccgcttac cggatacctg tccgcctttc 3840
   tcccttcggg aagcgtggcg ctttctcata gctcacgctg taggtatctc agttcggtgt 3900
   aggtcgttcg ctccaagctg ggctgtgtgc acgaaccccc cgttcagccc gaccgctgcg 3960
   ccttatccgg taactatcgt cttgagtcca acccggtaag acacgactta tcgccactgg 4020
   cagcagccac tggtaacagg attagcagag cgaggtatgt aggcggtgct acagagttct 4080
   tgaagtggtg gcctaactac ggctacacta gaaggacagt atttggtatc tgcgctctgc 4140
   tgaagccagt taccttcgga aaaagagttg gtagctcttg atccggcaaa caaaccaccg 4200
   ctggtagcgg tggttttttt gtttgcaagc agcagattac gcgcagaaaa aaaggatctc 4260
   aagaagatcc tttgatcttt tctacggggt ctgacgctca gtggaacgaa aactcacgtt 4320
   aagggatttt ggtcatgaga ttatcaaaaa ggatcttcac ctagatcctt ttaaattaaa 4380
   aatgaagttt taaatcaatc taaagtatat atgagtaaac ttggtctgac agttaccaat 4440
   gcttaatcag tgaggcacct atctcagcga tctgtctatt tcgttcatcc atagttgcct 4500
   gactccccgt cgtgtagata actacgatac gggagggctt accatctggc cccagtgctg 4560
   caatgatacc gcgagaccca cgctcaccgg ctccagattt atcagcaata aaccagccag 4620
   ccggaagggc cgagcgcaga agtggtcctg caactttatc cgcctccatc cagtctatta 4680
   attgttgccg ggaagctaga gtaagtagtt cgccagttaa tagtttgcgc aacgttgttg 4740
   ccattgctgc aggcatcgtg gtgtcacgct cgtcgtttgg tatggcttca ttcagctccg 4800
   gttcccaacg atcaaggcga gttacatgat cccccatgtt gtgcaaaaaa gcggttagct 4860
   ccttcggtcc tccgatcgtt gtcagaagta agttggccgc agtgttatca ctcatggtta 4920
   tggcagcact gcataattct cttactgtca tgccatccgt aagatgcttt tctgtgactg 4980
   gtgagtactc aaccaagtca ttctgagaat agtgtatgcg gcgaccgagt tgctcttgcc 5040
   cggcgtcaat acgggataat accgcgccac atagcagaac tttaaaagtg ctcatcattg 5100
   gaaaacgttc ttcggggcga aaactctcaa ggatcttacc gctgttgaga tccagttcga 5160
   tgtaacccac tcgtgcaccc aactgatctt cagcatcttt tactttcacc agcgtttctg 5220
   ggtgagcaaa aacaggaagg caaaatgccg caaaaaaggg aataagggcg acacggaaat 5280
   gttgaatact catactcttc ctttttcaat attattgaag catttatcag ggttattgtc 5340
   tcatgagcgg atacatattt gaatgtattt agaaaaataa acaaataggg gttccgcgca 5400
   catttccccg aaaagtgcca cctgaaattg taaacgttaa tattttgtta aaattcgcgt 5460
   taaatttttg ttaaatcagc tcatttttta accaataggc cgaaatcggc aaaatccctt 5520
   ataaatcaaa agaatagacc gagatagggt tgagtgttgt tccagtttgg aacaagagtc 5580
   cactattaaa gaacgtggac tccaacgtca aagggcgaaa aaccgtctat cagggcgatg 5640
   gcccactacg tgaaccatca ccctaatcaa gttttttggg gtcgaggtgc cgtaaagcac 5700
   taaatcggaa ccctaaaggg agcccccgat ttagagcttg acggggaaag ccggcgaacg 5760
   tggcgagaaa ggaagggaag aaagcgaaag gagcgggcgc tagggcgctg gcaagtgtag 5820
   cggtcacgct gcgcgtaacc accacacccg ccgcgcttaa tgcgccgcta cagggcgcgt 5880
   cccattcgcc a 5891
<210> 57
   <211> 486
   <212> ADN
   <213> Séquence artificielle
<220>
   <223> Description de la séquence artificielle: ADN codant pour la protéine de fusion TrX-DP-TME2_C731&733A dans le vecteur pET32a-DP-TME2_C731&733A
<400> 57
   atgagcgata aaattattca cctgactgac gacagttttg acacggatgt actcaaagcg 60
   gacggggcga tcctcgtcga tttctgggca gagtggtgcg gtccgtgcaa aatgatcgcc 120
   ccgattctgg atgaaatcgc tgacgaatat cagggcaaac tgaccgttgc aaaactgaac 180
   atcgatcaaa accctggcac tgcgccgaaa tatggcatcc gtggtatccc gactctgctg 240
   ctgttcaaaa acggtgaagt ggcggcaacc aaagtgggtg cactgtctaa aggtcagttg 300
   aaagagttcc tcgacgctaa cctggccggt tctggttctg gatctgatct gtctggtggt 360
   ggtggtggtc tggttccgcg tggatccgac ccggaatacg ttgttctgct gttcctgctg 420
   ctggctgacg ctcgtgttgc ttctgctctg tggatgatgc tgctgatctc tcaggctgaa 480
   gcttag 486
<210> 58
   <211> 161
   <212> PRT
   <213> Séquence artificielle
<220>
   <223> Description de la séquence artificielle: protéine de fusion TrX-DP-TME2_C731&733A codée par le vecteur pET32a-DP-TME2_C731&733A
<210> 59
   <211> 30
   <212> ADN
   <213> Séquence artificielle
<220>
   <223> Description de la séquence artificielle: oligonucléotide PET998-AlwNI (+) d'amplification de la cassette TrX-DP-TME1
<400> 59
   ttcagtggct gtgcatgcaa ggagatggcg 30
<210> 60
   <211> 30
   <212> ADN
   <213> Séquence artificielle
<220>
   <223> Description de la séquence artificielle: oligonucléotide PET998-AlwNI (-) d'amplification de la cassette TrX-DP-TME1
<400> 60
   ttcagccact gctaagcgtc aacaccagcg 30
<210> 61
   <211> 5914
   <212> ADN
   <213> Séquence artificielle
<220>
   <223> Description de la séquence artificielle: plasmide pGEXKT-DP-TME2+TrX-DP-TME1
<400> 61
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cggaatacgt tgttctgctg ttcctgctgc tggctgacgc tcgtgtttgc tcttgcctgt 1020
   ggatgatgct gctgatctct caggctgaag cttaggaatt cgcccttttc agtggctgtg 1080
   catgcaagga gatggcgccc aacagtcccc cggccacggg gcctgccacc atacccacgc 1140
   cgaaacaagc gctcatgagc ccgaagtggc gagcccgatc ttccccatcg gtgatgtcgg 1200
   cgatataggc gccagcaacc gcacctgtgg cgccggtgat gccggccacg atgcgtccgg 1260
   cgtagaggat cgagatcgat ctcgatcccg cgaaattaat acgactcact ataggggaat 1320
   tgtgagcgga taacaattcc cctctagaaa taattttgtt taactttaag aaggagatat 1380
   acatatgagc gataaaatta ttcacctgac tgacgacagt tttgacacgg atgtactcaa 1440
   agcggacggg gcgatcctcg tcgatttctg ggcagagtgg tgcggtccgt gcaaaatgat 1500
   cgccccgatt ctggatgaaa tcgctgacga atatcagggc aaactgaccg ttgcaaaact 1560
   gaacatcgat caaaaccctg gcactgcgcc gaaatatggc atccgtggta tcccgactct 1620
   gctgctgttc aaaaacggtg aagtggcggc aaccaaagtg ggtgcactgt ctaaaggtca 1680
   gttgaaagag ttcctcgacg ctaacctggc cggttctggt tctggatctg atctgtctgg 1740
   tggtggtggt ggtctggttc cgcgtggatc cgacccgatc gctggtgctc actggggtgt 1800
   tctggctggt atcgcttact tctctatggt tggtaactgg gctaaagttc tggttgttct 1860
   gctgctgttc gctggtgttg acgcttagca gtggctgaaa agggcgaatt catcgtgact 1920
   gactgacgat ctgcctcgcg cgtttcggtg atgacggtga aaacctctga cacatgcagc 1980
   tcccggagac ggtcacagct tgtctgtaag cggatgccgg gagcagacaa gcccgtcagg 2040
   gcgcgtcagc gggtgttggc gggtgtcggg gcgcagccat gacccagtca cgtagcgata 2100
   gcggagtgta taattcttga agacgaaagg gcctcgtgat acgcctattt ttataggtta 2160
   atgtcatgat aataatggtt tcttagacgt caggtggcac ttttcgggga aatgtgcgcg 2220
   gaacccctat ttgtttattt ttctaaatac attcaaatat gtatccgctc atgagacaat 2280
   aaccctgata aatgcttcaa taatattgaa aaaggaagag tatgagtatt caacatttcc 2340
   gtgtcgccct tattcccttt tttgcggcat tttgccttcc tgtttttgct cacccagaaa 2400
   cgctggtgaa agtaaaagat gctgaagatc agttgggtgc acgagtgggt tacatcgaac 2460
   tggatctcaa cagcggtaag atccttgaga gttttcgccc cgaagaacgt tttccaatga 2520
   tgagcacttt taaagttctg ctatgtggcg cggtattatc ccgtgttgac gccgggcaag 2580
   agcaactcgg tcgccgcata cactattctc agaatgactt ggttgagtac tcaccagtca 2640
   cagaaaagca tcttacggat ggcatgacag taagagaatt atgcagtgct gccataacca 2700
   tgagtgataa cactgcggcc aacttacttc tgacaacgat cggaggaccg aaggagctaa 2760
   ccgctttttt gcacaacatg ggggatcatg taactcgcct tgatcgttgg gaaccggagc 2820
   tgaatgaagc cataccaaac gacgagcgtg acaccacgat gcctgcagca atggcaacaa 2880
   cgttgcgcaa actattaact ggcgaactac ttactctagc ttcccggcaa caattaatag 2940
   actggatgga ggcggataaa gttgcaggac cacttctgcg ctcggccctt ccggctggct 3000
   ggtttattgc tgataaatct ggagccggtg agcgtgggtc tcgcggtatc attgcagcac 3060
   tggggccaga tggtaagccc tcccgtatcg tagttatcta cacgacgggg agtcaggcaa 3120
   ctatggatga acgaaataga cagatcgctg agataggtgc ctcactgatt aagcattggt 3180
   aactgtcaga ccaagtttac tcatatatac tttagattga tttaaaactt catttttaat 3240
   ttaaaaggat ctaggtgaag atcctttttg ataatctcat gaccaaaatc ccttaacgtg 3300
   agttttcgtt ccactgagcg tcagaccccg tagaaaagat caaaggatct tcttgagatc 3360
   ctttttttct gcgcgtaatc tgctgcttgc aaacaaaaaa accaccgcta ccagcggtgg 3420
   tttgtttgcc ggatcaagag ctaccaactc tttttccgaa ggtaactggc ttcagcagag 3480
   cgcagatacc aaatactgtc cttctagtgt agccgtagtt aggccaccac ttcaagaact 3540
   ctgtagcacc gcctacatac ctcgctctgc taatcctgtt accagtggct gctgccagtg 3600
   gcgataagtc gtgtcttacc gggttggact caagacgata gttaccggat aaggcgcagc 3660
   ggtcgggctg aacggggggt tcgtgcacac agcccagctt ggagcgaacg acctacaccg 3720
   aactgagata cctacagcgt gagctatgag aaagcgccac gcttcccgaa gggagaaagg 3780
   cggacaggta tccggtaagc ggcagggtcg gaacaggaga gcgcacgagg gagcttccag 3840
   ggggaaacgc ctggtatctt tatagtcctg tcgggtttcg ccacctctga cttgagcgtc 3900
   gatttttgtg atgctcgtca ggggggcgga gcctatggaa aaacgccagc aacgcggcct 3960
   ttttacggtt cctggccttt tgctggcctt ttgctcacat gttctttcct gcgttatccc 4020
   ctgattctgt ggataaccgt attaccgcct ttgagtgagc tgataccgct cgccgcagcc 4080
   gaacgaccga gcgcagcgag tcagtgagcg aggaagcgga agagcgcctg atgcggtatt 4140
   ttctccttac gcatctgtgc ggtatttcac accgcataaa ttccgacacc atcgaatggt 4200
   gcaaaacctt tcgcggtatg gcatgatagc gcccggaaga gagtcaattc agggtggtga 4260
   atgtgaaacc agtaacgtta tacgatgtcg cagagtatgc cggtgtctct tatcagaccg 4320
   tttcccgcgt ggtgaaccag gccagccacg tttctgcgaa aacgcgggaa aaagtggaag 4380
   cggcgatggc ggagctgaat tacattccca accgcgtggc acaacaactg gcgggcaaac 4440
   agtcgttgct gattggcgtt gccacctcca gtctggccct gcacgcgccg tcgcaaattg 4500
   tcgcggcgat taaatctcgc gccgatcaac tgggtgccag cgtggtggtg tcgatggtag 4560
   aacgaagcgg cgtcgaagcc tgtaaagcgg cggtgcacaa tcttctcgcg caacgcgtca 4620
   gtgggctgat cattaactat ccgctggatg accaggatgc cattgctgtg gaagctgcct 4680
   gcactaatgt tccggcgtta tttcttgatg tctctgacca gacacccatc aacagtatta 4740
   ttttctccca tgaagacggt acgcgactgg gcgtggagca tctggtcgca ttgggtcacc 4800
   agcaaatcgc gctgttagcg ggcccattaa gttctgtctc ggcgcgtctg cgtctggctg 4860
   gctggcataa atatctcact cgcaatcaaa ttcagccgat agcggaacgg gaaggcgact 4920
   ggagtgccat gtccggtttt caacaaacca tgcaaatgct gaatgagggc atcgttccca 4980
   ctgcgatgct ggttgccaac gatcagatgg cgctgggcgc aatgcgcgcc attaccgagt 5040
   ccgggctgcg cgttggtgcg gatatctcgg tagtgggata cgacgatacc gaagacagct 5100
   catgttatat cccgccgtta accaccatca aacaggattt tcgcctgctg gggcaaacca 5160
   gcgtggaccg cttgctgcaa ctctctcagg gccaggcggt gaagggcaat cagctgttgc 5220
   ccgtctcact ggtgaaaaga aaaaccaccc tggcgcccaa tacgcaaacc gcctctcccc 5280
   gcgcgttggc cgattcatta atgcagctgg cacgacaggt ttcccgactg gaaagcgggc 5340
   agtgagcgca acgcaattaa tgtgagttag ctcactcatt aggcacccca ggctttacac 5400
   tttatgcttc cggctcgtat gttgtgtgga attgtgagcg gataacaatt tcacacagga 5460
   aacagctatg accatgatta cggattcact ggccgtcgtt ttacaacgtc gtgactggga 5520
   aaaccctggc gttacccaac ttaatcgcct tgcagcacat ccccctttcg ccagctggcg 5580
   taatagcgaa gaggcccgca ccgatcgccc ttcccaacag ttgcgcagcc tgaatggcga 5640
   atggcgcttt gcctggtttc cggcaccaga agcggtgccg gaaagctggc tggagtgcga 5700
   tcttcctgag gccgatactg tcgtcgtccc ctcaaactgg cagatgcacg gttacgatgc 5760
   gcccatctac accaacgtaa cctatcccat tacggtcaat ccgccgtttg ttcccacgga 5820
   gaatccgacg ggttgttact cgctcacatt taatgttgat gaaagctggc tacaggaagg 5880
   ccagacgcga attatttttg atggcgttgg aatt                             5914
<210> 62
   <211> 5914
   <212> ADN
   <213> Séquence artificielle
<220>
   <223> Description de la séquence artificielle: plasmide pGEXKT-DP-TME2_C731&733A+TrX-DP-TME1
<400> 62
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cggaatacgt tgttctgctg ttcctgctgc tggctgacgc tcgtgttgct tctgctctgt 1020
   ggatgatgct gctgatctct caggctgaag cttaggaatt cgcccttttc agccactgct 1080
   aagcgtcaac accagcgaac agcagcagaa caaccagaac tttagcccag ttaccaacca 1140
   tagagaagta agcgatacca gccagaacac cccagtgagc accagcgatc gggtcggatc 1200
   cacgcggaac cagaccacca ccaccaccag acagatcaga tccagaacca gaaccggcca 1260
   ggttagcgtc gaggaactct ttcaactgac ctttagacag tgcacccact ttggttgccg 1320
   ccacttcacc gtttttgaac agcagcagag tcgggatacc acggatgcca tatttcggcg 1380
   cagtgccagg gttttgatcg atgttcagtt ttgcaacggt cagtttgccc tgatattcgt 1440
   cagcgatttc atccagaatc ggggcgatca ttttgcacgg accgcaccac tctgcccaga 1500
   aatcgacgag gatcgccccg tccgctttga gtacatccgt gtcaaaactg tcgtcagtca 1560
   ggtgaataat tttatcgctc atatgtatat ctccttctta aagttaaaca aaattatttc 1620
   tagaggggaa ttgttatccg ctcacaattc ccctatagtg agtcgtatta atttcgcggg 1680
   atcgagatcg atctcgatcc tctacgccgg acgcatcgtg gccggcatca ccggcgccac 1740
   aggtgcggtt gctggcgcct atatcgccga catcaccgat ggggaagatc gggctcgcca 1800
   cttcgggctc atgagcgctt gtttcggcgt gggtatggtg gcaggccccg tggccggggg 1860
   actgttgggc gccatctcct tgcatgcaca gccactgaaa agggcgaatt catcgtgact 1920
   gactgacgat ctgcctcgcg cgtttcggtg atgacggtga aaacctctga cacatgcagc 1980
   tcccggagac ggtcacagct tgtctgtaag cggatgccgg gagcagacaa gcccgtcagg 2040
   gcgcgtcagc gggtgttggc gggtgtcggg gcgcagccat gacccagtca cgtagcgata 2100
   gcggagtgta taattcttga agacgaaagg gcctcgtgat acgcctattt ttataggtta 2160
   atgtcatgat aataatggtt tcttagacgt caggtggcac ttttcgggga aatgtgcgcg 2220
   gaacccctat ttgtttattt ttctaaatac attcaaatat gtatccgctc atgagacaat 2280
   aaccctgata aatgcttcaa taatattgaa aaaggaagag tatgagtatt caacatttcc 2340
   gtgtcgccct tattcccttt tttgcggcat tttgccttcc tgtttttgct cacccagaaa 2400
   cgctggtgaa agtaaaagat gctgaagatc agttgggtgc acgagtgggt tacatcgaac 2460
   tggatctcaa cagcggtaag atccttgaga gttttcgccc cgaagaacgt tttccaatga 2520
   tgagcacttt taaagttctg ctatgtggcg cggtattatc ccgtgttgac gccgggcaag 2580
   agcaactcgg tcgccgcata cactattctc agaatgactt ggttgagtac tcaccagtca 2640
   cagaaaagca tcttacggat ggcatgacag taagagaatt atgcagtgct gccataacca 2700
   tgagtgataa cactgcggcc aacttacttc tgacaacgat cggaggaccg aaggagctaa 2760
   ccgctttttt gcacaacatg ggggatcatg taactcgcct tgatcgttgg gaaccggagc 2820
   tgaatgaagc cataccaaac gacgagcgtg acaccacgat gcctgcagca atggcaacaa 2880
   cgttgcgcaa actattaact ggcgaactac ttactctagc ttcccggcaa caattaatag 2940
   actggatgga ggcggataaa gttgcaggac cacttctgcg ctcggccctt ccggctggct 3000
   ggtttattgc tgataaatct ggagccggtg agcgtgggtc tcgcggtatc attgcagcac 3060
   tggggccaga tggtaagccc tcccgtatcg tagttatcta cacgacgggg agtcaggcaa 3120
   ctatggatga acgaaataga cagatcgctg agataggtgc ctcactgatt aagcattggt 3180
   aactgtcaga ccaagtttac tcatatatac tttagattga tttaaaactt catttttaat 3240
   ttaaaaggat ctaggtgaag atcctttttg ataatctcat gaccaaaatc ccttaacgtg 3300
   agttttcgtt ccactgagcg tcagaccccg tagaaaagat caaaggatct tcttgagatc 3360
   ctttttttct gcgcgtaatc tgctgcttgc aaacaaaaaa accaccgcta ccagcggtgg 3420
   tttgtttgcc ggatcaagag ctaccaactc tttttccgaa ggtaactggc ttcagcagag 3480
   cgcagatacc aaatactgtc cttctagtgt agccgtagtt aggccaccac ttcaagaact 3540
   ctgtagcacc gcctacatac ctcgctctgc taatcctgtt accagtggct gctgccagtg 3600
   gcgataagtc gtgtcttacc gggttggact caagacgata gttaccggat aaggcgcagc 3660
   ggtcgggctg aacggggggt tcgtgcacac agcccagctt ggagcgaacg acctacaccg 3720
   aactgagata cctacagcgt gagctatgag aaagcgccac gcttcccgaa gggagaaagg 3780
   cggacaggta tccggtaagc ggcagggtcg gaacaggaga gcgcacgagg gagcttccag 3840
   ggggaaacgc ctggtatctt tatagtcctg tcgggtttcg ccacctctga cttgagcgtc 3900
   gatttttgtg atgctcgtca ggggggcgga gcctatggaa aaacgccagc aacgcggcct 3960
   ttttacggtt cctggccttt tgctggcctt ttgctcacat gttctttcct gcgttatccc 4020
   ctgattctgt ggataaccgt attaccgcct ttgagtgagc tgataccgct cgccgcagcc 4080
   gaacgaccga gcgcagcgag tcagtgagcg aggaagcgga agagcgcctg atgcggtatt 4140
   ttctccttac gcatctgtgc ggtatttcac accgcataaa ttccgacacc atcgaatggt 4200
   gcaaaacctt tcgcggtatg gcatgatagc gcccggaaga gagtcaattc agggtggtga 4260
   atgtgaaacc agtaacgtta tacgatgtcg cagagtatgc cggtgtctct tatcagaccg 4320
   tttcccgcgt ggtgaaccag gccagccacg tttctgcgaa aacgcgggaa aaagtggaag 4380
   cggcgatggc ggagctgaat tacattccca accgcgtggc acaacaactg gcgggcaaac 4440
   agtcgttgct gattggcgtt gccacctcca gtctggccct gcacgcgccg tcgcaaattg 4500
   tcgcggcgat taaatctcgc gccgatcaac tgggtgccag cgtggtggtg tcgatggtag 4560
   aacgaagcgg cgtcgaagcc tgtaaagcgg cggtgcacaa tcttctcgcg caacgcgtca 4620
   gtgggctgat cattaactat ccgctggatg accaggatgc cattgctgtg gaagctgcct 4680
   gcactaatgt tccggcgtta tttcttgatg tctctgacca gacacccatc aacagtatta 4740
   ttttctccca tgaagacggt acgcgactgg gcgtggagca tctggtcgca ttgggtcacc 4800
   agcaaatcgc gctgttagcg ggcccattaa gttctgtctc ggcgcgtctg cgtctggctg 4860
   gctggcataa atatctcact cgcaatcaaa ttcagccgat agcggaacgg gaaggcgact 4920
   ggagtgccat gtccggtttt caacaaacca tgcaaatgct gaatgagggc atcgttccca 4980
   ctgcgatgct ggttgccaac gatcagatgg cgctgggcgc aatgcgcgcc attaccgagt 5040
   ccgggctgcg cgttggtgcg gatatctcgg tagtgggata cgacgatacc gaagacagct 5100
   catgttatat cccgccgtta accaccatca aacaggattt tcgcctgctg gggcaaacca 5160
   gcgtggaccg cttgctgcaa ctctctcagg gccaggcggt gaagggcaat cagctgttgc 5220
   ccgtctcact ggtgaaaaga aaaaccaccc tggcgcccaa tacgcaaacc gcctctcccc 5280
   gcgcgttggc cgattcatta atgcagctgg cacgacaggt ttcccgactg gaaagcgggc 5340
   agtgagcgca acgcaattaa tgtgagttag ctcactcatt aggcacccca ggctttacac 5400
   tttatgcttc cggctcgtat gttgtgtgga attgtgagcg gataacaatt tcacacagga 5460
   aacagctatg accatgatta cggattcact ggccgtcgtt ttacaacgtc gtgactggga 5520
   aaaccctggc gttacccaac ttaatcgcct tgcagcacat ccccctttcg ccagctggcg 5580
   taatagcgaa gaggcccgca ccgatcgccc ttcccaacag ttgcgcagcc tgaatggcga 5640
   atggcgcttt gcctggtttc cggcaccaga agcggtgccg gaaagctggc tggagtgcga 5700
   tcttcctgag gccgatactg tcgtcgtccc ctcaaactgg cagatgcacg gttacgatgc 5760
   gcccatctac accaacgtaa cctatcccat tacggtcaat ccgccgtttg ttcccacgga 5820
   gaatccgacg ggttgttact cgctcacatt taatgttgat gaaagctggc tacaggaagg 5880
   ccagacgcga attatttttg atggcgttgg aatt 5914
<210> 63
   <211> 15
   <212> PRT
   <213> Séquence artificielle
<220>
   <223> Description de la séquence artificielle: séquence intégrée de pGEXKT
<400> 63
   Ser Asp Leu Ser Gly Gly Gly Gly Gly Leu Val Pro Arg Gly Ser
   1 5 10 15
<210> 64
   <211> 5082
   <212> ADN
   <213> Séquence artificielle
<220>
   <223> Description de la séquence artificielle: Vecteur pGEXKT-DP-TME1_G354L
<400> 64
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cgatcgctgg tgctcactgg ctggttctgg ctggtatcgc ttacttctct atggttggta 1020
   actgggctaa agttctggtt gttctgctgc tgttcgctgg tgttgacgct taggaattca 1080
   tcgtgactga ctgacgatct gcctcgcgcg tttcggtgat gacggtgaaa acctctgaca 1140
   catgcagctc ccggagacgg tcacagcttg tctgtaagcg gatgccggga gcagacaagc 1200
   ccgtcagggc gcgtcagcgg gtgttggcgg gtgtcggggc gcagccatga cccagtcacg 1260
   tagcgatagc ggagtgtata attcttgaag acgaaagggc ctcgtgatac gcctattttt 1320
   ataggttaat gtcatgataa taatggtttc ttagacgtca ggtggcactt ttcggggaaa 1380
   tgtgcgcgga acccctattt gtttattttt ctaaatacat tcaaatatgt atccgctcat 1440
   gagacaataa ccctgataaa tgcttcaata atattgaaaa aggaagagta tgagtattca 1500
   acatttccgt gtcgccctta ttcccttttt tgcggcattt tgccttcctg tttttgctca 1560
   cccagaaacg ctggtgaaag taaaagatgc tgaagatcag ttgggtgcac gagtgggtta 1620
   catcgaactg gatctcaaca gcggtaagat ccttgagagt tttcgccccg aagaacgttt 1680
   tccaatgatg agcactttta aagttctgct atgtggcgcg gtattatccc gtgttgacgc 1740
   cgggcaagag caactcggtc gccgcataca ctattctcag aatgacttgg ttgagtactc 1800
   accagtcaca gaaaagcatc ttacggatgg catgacagta agagaattat gcagtgctgc 1860
   cataaccatg agtgataaca ctgcggccaa cttacttctg acaacgatcg gaggaccgaa 1920
   ggagctaacc gcttttttgc acaacatggg ggatcatgta actcgccttg atcgttggga 1980
   accggagctg aatgaagcca taccaaacga cgagcgtgac accacgatgc ctgcagcaat 2040
   ggcaacaacg ttgcgcaaac tattaactgg cgaactactt actctagctt cccggcaaca 2100
   attaatagac tggatggagg cggataaagt tgcaggacca cttctgcgct cggcccttcc 2160
   ggctggctgg tttattgctg ataaatctgg agccggtgag cgtgggtctc gcggtatcat 2220
   tgcagcactg gggccagatg gtaagccctc ccgtatcgta gttatctaca cgacggggag 2280
   tcaggcaact atggatgaac gaaatagaca gatcgctgag ataggtgcct cactgattaa 2340
   gcattggtaa ctgtcagacc aagtttactc atatatactt tagattgatt taaaacttca 2400
   tttttaattt aaaaggatct aggtgaagat cctttttgat aatctcatga ccaaaatccc 2460
   ttaacgtgag ttttcgttcc actgagcgtc agaccccgta gaaaagatca aaggatcttc 2520
   ttgagatcct ttttttctgc gcgtaatctg ctgcttgcaa acaaaaaaac caccgctacc 2580
   agcggtggtt tgtttgccgg atcaagagct accaactctt tttccgaagg taactggctt 2640
   cagcagagcg cagataccaa atactgtcct tctagtgtag ccgtagttag gccaccactt 2700
   caagaactct gtagcaccgc ctacatacct cgctctgcta atcctgttac cagtggctgc 2760
   tgccagtggc gataagtcgt gtcttaccgg gttggactca agacgatagt taccggataa 2820
   ggcgcagcgg tcgggctgaa cggggggttc gtgcacacag cccagcttgg agcgaacgac 2880
   ctacaccgaa ctgagatacc tacagcgtga gctatgagaa agcgccacgc ttcccgaagg 2940
   gagaaaggcg gacaggtatc cggtaagcgg cagggtcgga acaggagagc gcacgaggga 3000
   gcttccaggg ggaaacgcct ggtatcttta tagtcctgtc gggtttcgcc acctctgact 3060
   tgagcgtcga tttttgtgat gctcgtcagg ggggcggagc ctatggaaaa acgccagcaa 3120
   cgcggccttt ttacggttcc tggccttttg ctggcctttt gctcacatgt tctttcctgc 3180
   gttatcccct gattctgtgg ataaccgtat taccgccttt gagtgagctg ataccgctcg 3240
   ccgcagccga acgaccgagc gcagcgagtc agtgagcgag gaagcggaag agcgcctgat 3300
   gcggtatttt ctccttacgc atctgtgcgg tatttcacac cgcataaatt ccgacaccat 3360
   cgaatggtgc aaaacctttc gcggtatggc atgatagcgc ccggaagaga gtcaattcag 3420
   ggtggtgaat gtgaaaccag taacgttata cgatgtcgca gagtatgccg gtgtctctta 3480
   tcagaccgtt tcccgcgtgg tgaaccaggc cagccacgtt tctgcgaaaa cgcgggaaaa 3540
   agtggaagcg gcgatggcgg agctgaatta cattcccaac cgcgtggcac aacaactggc 3600
   gggcaaacag tcgttgctga ttggcgttgc cacctccagt ctggccctgc acgcgccgtc 3660
   gcaaattgtc gcggcgatta aatctcgcgc cgatcaactg ggtgccagcg tggtggtgtc 3720
   gatggtagaa cgaagcggcg tcgaagcctg taaagcggcg gtgcacaatc ttctcgcgca 3780
   acgcgtcagt gggctgatca ttaactatcc gctggatgac caggatgcca ttgctgtgga 3840
   agctgcctgc actaatgttc cggcgttatt tcttgatgtc tctgaccaga cacccatcaa 3900
   cagtattatt ttctcccatg aagacggtac gcgactgggc gtggagcatc tggtcgcatt 3960
   gggtcaccag caaatcgcgc tgttagcggg cccattaagt tctgtctcgg cgcgtctgcg 4020
   tctggctggc tggcataaat atctcactcg caatcaaatt cagccgatag cggaacggga 4080
   aggcgactgg agtgccatgt ccggttttca acaaaccatg caaatgctga atgagggcat 4140
   cgttcccact gcgatgctgg ttgccaacga tcagatggcg ctgggcgcaa tgcgcgccat 4200
   taccgagtcc gggctgcgcg ttggtgcgga tatctcggta gtgggatacg acgataccga 4260
   agacagctca tgttatatcc cgccgttaac caccatcaaa caggattttc gcctgctggg 4320
   gcaaaccagc gtggaccgct tgctgcaact ctctcagggc caggcggtga agggcaatca 4380
   gctgttgccc gtctcactgg tgaaaagaaa aaccaccctg gcgcccaata cgcaaaccgc 4440
   ctctccccgc gcgttggccg attcattaat gcagctggca cgacaggttt cccgactgga 4500
   aagcgggcag tgagcgcaac gcaattaatg tgagttagct cactcattag gcaccccagg 4560
   ctttacactt tatgcttccg gctcgtatgt tgtgtggaat tgtgagcgga taacaatttc 4620
   acacaggaaa cagctatgac catgattacg gattcactgg ccgtcgtttt acaacgtcgt 4680
   gactgggaaa accctggcgt tacccaactt aatcgccttg cagcacatcc ccctttcgcc 4740
   agctggcgta atagcgaaga ggcccgcacc gatcgccctt cccaacagtt gcgcagcctg 4800
   aatggcgaat ggcgctttgc ctggtttccg gcaccagaag cggtgccgga aagctggctg 4860
   gagtgcgatc ttcctgaggc cgatactgtc gtcgtcccct caaactggca gatgcacggt 4920
   tacgatgcgc ccatctacac caacgtaacc tatcccatta cggtcaatcc gccgtttgtt 4980
   cccacggaga atccgacggg ttgttactcg ctcacattta atgttgatga aagctggcta 5040
   caggaaggcc agacgcgaat tatttttgat ggcgttggaa tt                    5082
<210> 65
   <211> 271
   <212> PRT
   <213> Séquence artificielle
<220>
   <223> Description de la séquence artificielle: Protéine chimère GSTkt-DP-TME1_G354L
<400> 65
   Met Ser Pro Ile Leu Gly Tyr Trp Lys Ile Lys Gly Leu Val Gln Pro
    1               5                  10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Leu Ser Gly Gly
       210                 215                 220
   Gly Gly Gly Leu Val Pro Arg Gly Ser Asp Pro Ile Ala Gly Ala His
   225                 230                 235                 240
   Trp Leu Val Leu Ala Gly Ile Ala Tyr Phe Ser Met Val Gly Asn Trp
                   245                 250                 255
   Ala Lys Val Leu Val Val Leu Leu Leu Phe Ala Gly Val Asp Ala
               260                 265                 270
<210> 66
   <211> 5082
   <212> ADN
   <213> Séquence artificielle
<220>
   <223> Description de la séquence artificielle: Vecteur pGEXKT-DP-TME1_G358L
<400> 66
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cgatcgctgg tgctcactgg ggtgttctgg ctctgatcgc ttacttctct atggttggta 1020
   actgggctaa agttctggtt gttctgctgc tgttcgctgg tgttgacgct taggaattca 1080
   tcgtgactga ctgacgatct gcctcgcgcg tttcggtgat gacggtgaaa acctctgaca 1140
   catgcagctc ccggagacgg tcacagcttg tctgtaagcg gatgccggga gcagacaagc 1200
   ccgtcagggc gcgtcagcgg gtgttggcgg gtgtcggggc gcagccatga cccagtcacg 1260
   tagcgatagc ggagtgtata attcttgaag acgaaagggc ctcgtgatac gcctattttt 1320
   ataggttaat gtcatgataa taatggtttc ttagacgtca ggtggcactt ttcggggaaa 1380
   tgtgcgcgga acccctattt gtttattttt ctaaatacat tcaaatatgt atccgctcat 1440
   gagacaataa ccctgataaa tgcttcaata atattgaaaa aggaagagta tgagtattca 1500
   acatttccgt gtcgccctta ttcccttttt tgcggcattt tgccttcctg tttttgctca 1560
   cccagaaacg ctggtgaaag taaaagatgc tgaagatcag ttgggtgcac gagtgggtta 1620
   catcgaactg gatctcaaca gcggtaagat ccttgagagt tttcgccccg aagaacgttt 1680
   tccaatgatg agcactttta aagttctgct atgtggcgcg gtattatccc gtgttgacgc 1740
   cgggcaagag caactcggtc gccgcataca ctattctcag aatgacttgg ttgagtactc 1800
   accagtcaca gaaaagcatc ttacggatgg catgacagta agagaattat gcagtgctgc 1860
   cataaccatg agtgataaca ctgcggccaa cttacttctg acaacgatcg gaggaccgaa 1920
   ggagctaacc gcttttttgc acaacatggg ggatcatgta actcgccttg atcgttggga 1980
   accggagctg aatgaagcca taccaaacga cgagcgtgac accacgatgc ctgcagcaat 2040
   ggcaacaacg ttgcgcaaac tattaactgg cgaactactt actctagctt cccggcaaca 2100
   attaatagac tggatggagg cggataaagt tgcaggacca cttctgcgct cggcccttcc 2160
   ggctggctgg tttattgctg ataaatctgg agccggtgag cgtgggtctc gcggtatcat 2220
   tgcagcactg gggccagatg gtaagccctc ccgtatcgta gttatctaca cgacggggag 2280
   tcaggcaact atggatgaac gaaatagaca gatcgctgag ataggtgcct cactgattaa 2340
   gcattggtaa ctgtcagacc aagtttactc atatatactt tagattgatt taaaacttca 2400
   tttttaattt aaaaggatct aggtgaagat cctttttgat aatctcatga ccaaaatccc 2460
   ttaacgtgag ttttcgttcc actgagcgtc agaccccgta gaaaagatca aaggatcttc 2520
   ttgagatcct ttttttctgc gcgtaatctg ctgcttgcaa acaaaaaaac caccgctacc 2580
   agcggtggtt tgtttgccgg atcaagagct accaactctt tttccgaagg taactggctt 2640
   cagcagagcg cagataccaa atactgtcct tctagtgtag ccgtagttag gccaccactt 2700
   caagaactct gtagcaccgc ctacatacct cgctctgcta atcctgttac cagtggctgc 2760
   tgccagtggc gataagtcgt gtcttaccgg gttggactca agacgatagt taccggataa 2820
   ggcgcagcgg tcgggctgaa cggggggttc gtgcacacag cccagcttgg agcgaacgac 2880
   ctacaccgaa ctgagatacc tacagcgtga gctatgagaa agcgccacgc ttcccgaagg 2940
   gagaaaggcg gacaggtatc cggtaagcgg cagggtcgga acaggagagc gcacgaggga 3000
   gcttccaggg ggaaacgcct ggtatcttta tagtcctgtc gggtttcgcc acctctgact 3060
   tgagcgtcga tttttgtgat gctcgtcagg ggggcggagc ctatggaaaa acgccagcaa 3120
   cgcggccttt ttacggttcc tggccttttg ctggcctttt gctcacatgt tctttcctgc 3180
   gttatcccct gattctgtgg ataaccgtat taccgccttt gagtgagctg ataccgctcg 3240
   ccgcagccga acgaccgagc gcagcgagtc agtgagcgag gaagcggaag agcgcctgat 3300
   gcggtatttt ctccttacgc atctgtgcgg tatttcacac cgcataaatt ccgacaccat 3360
   cgaatggtgc aaaacctttc gcggtatggc atgatagcgc ccggaagaga gtcaattcag 3420
   ggtggtgaat gtgaaaccag taacgttata cgatgtcgca gagtatgccg gtgtctctta 3480
   tcagaccgtt tcccgcgtgg tgaaccaggc cagccacgtt tctgcgaaaa cgcgggaaaa 3540
   agtggaagcg gcgatggcgg agctgaatta cattcccaac cgcgtggcac aacaactggc 3600
   gggcaaacag tcgttgctga ttggcgttgc cacctccagt ctggccctgc acgcgccgtc 3660
   gcaaattgtc gcggcgatta aatctcgcgc cgatcaactg ggtgccagcg tggtggtgtc 3720
   gatggtagaa cgaagcggcg tcgaagcctg taaagcggcg gtgcacaatc ttctcgcgca 3780
   acgcgtcagt gggctgatca ttaactatcc gctggatgac caggatgcca ttgctgtgga 3840
   agctgcctgc actaatgttc cggcgttatt tcttgatgtc tctgaccaga cacccatcaa 3900
   cagtattatt ttctcccatg aagacggtac gcgactgggc gtggagcatc tggtcgcatt 3960
   gggtcaccag caaatcgcgc tgttagcggg cccattaagt tctgtctcgg cgcgtctgcg 4020
   tctggctggc tggcataaat atctcactcg caatcaaatt cagccgatag cggaacggga 4080
   aggcgactgg agtgccatgt ccggttttca acaaaccatg caaatgctga atgagggcat 4140
   cgttcccact gcgatgctgg ttgccaacga tcagatggcg ctgggcgcaa tgcgcgccat 4200
   taccgagtcc gggctgcgcg ttggtgcgga tatctcggta gtgggatacg acgataccga 4260
   agacagctca tgttatatcc cgccgttaac caccatcaaa caggattttc gcctgctggg 4320
   gcaaaccagc gtggaccgct tgctgcaact ctctcagggc caggcggtga agggcaatca 4380
   gctgttgccc gtctcactgg tgaaaagaaa aaccaccctg gcgcccaata cgcaaaccgc 4440
   ctctccccgc gcgttggccg attcattaat gcagctggca cgacaggttt cccgactgga 4500
   aagcgggcag tgagcgcaac gcaattaatg tgagttagct cactcattag gcaccccagg 4560
   ctttacactt tatgcttccg gctcgtatgt tgtgtggaat tgtgagcgga taacaatttc 4620
   acacaggaaa cagctatgac catgattacg gattcactgg ccgtcgtttt acaacgtcgt 4680
   gactgggaaa accctggcgt tacccaactt aatcgccttg cagcacatcc ccctttcgcc 4740
   agctggcgta atagcgaaga ggcccgcacc gatcgccctt cccaacagtt gcgcagcctg 4800
   aatggcgaat ggcgctttgc ctggtttccg gcaccagaag cggtgccgga aagctggctg 4860
   gagtgcgatc ttcctgaggc cgatactgtc gtcgtcccct caaactggca gatgcacggt 4920
   tacgatgcgc ccatctacac caacgtaacc tatcccatta cggtcaatcc gccgtttgtt 4980
   cccacggaga atccgacggg ttgttactcg ctcacattta atgttgatga aagctggcta 5040
   caggaaggcc agacgcgaat tatttttgat ggcgttggaa tt                    5082
<210> 67
   <211> 271
   <212> PRT
   <213> Séquence artificielle
<220>
   <223> Description de la séquence artificielle: protéine chimère pGEXkt-DP-TME1_G358L
<400> 67
   Met Ser Pro Ile Leu Gly Tyr Trp Lys Ile Lys Gly Leu Val Gln Pro
    1               5                  10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Leu Ser Gly Gly
       210                 215                 220
   Gly Gly Gly Leu Val Pro Arg Gly Ser Asp Pro Ile Ala Gly Ala His
   225                 230                 235                 240
   Trp Gly Val Leu Ala Leu Ile Ala Tyr Phe Ser Met Val Gly Asn Trp
                   245                 250                 255
   Ala Lys Val Leu Val Val Leu Leu Leu Phe Ala Gly Val Asp Ala
               260                 265                 270
<210> 68
   <211> 5082
   <212> ADN
   <213> Séquence artificielle
<220>
   <223> Description de la séquence artificielle: Vecteur pGEXKT-DP-TME1_G354&358L
<400> 68
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cgatcgctgg tgctcactgg ctggttctgg ctctgatcgc ttacttctct atggttggta 1020
   actgggctaa agttctggtt gttctgctgc tgttcgctgg tgttgacgct taggaattca 1080
   tcgtgactga ctgacgatct gcctcgcgcg tttcggtgat gacggtgaaa acctctgaca 1140
   catgcagctc ccggagacgg tcacagcttg tctgtaagcg gatgccggga gcagacaagc 1200
   ccgtcagggc gcgtcagcgg gtgttggcgg gtgtcggggc gcagccatga cccagtcacg 1260
   tagcgatagc ggagtgtata attcttgaag acgaaagggc ctcgtgatac gcctattttt 1320
   ataggttaat gtcatgataa taatggtttc ttagacgtca ggtggcactt ttcggggaaa 1380
   tgtgcgcgga acccctattt gtttattttt ctaaatacat tcaaatatgt atccgctcat 1440
   gagacaataa ccctgataaa tgcttcaata atattgaaaa aggaagagta tgagtattca 1500
   acatttccgt gtcgccctta ttcccttttt tgcggcattt tgccttcctg tttttgctca 1560
   cccagaaacg ctggtgaaag taaaagatgc tgaagatcag ttgggtgcac gagtgggtta 1620
   catcgaactg gatctcaaca gcggtaagat ccttgagagt tttcgccccg aagaacgttt 1680
   tccaatgatg agcactttta aagttctgct atgtggcgcg gtattatccc gtgttgacgc 1740
   cgggcaagag caactcggtc gccgcataca ctattctcag aatgacttgg ttgagtactc 1800
   accagtcaca gaaaagcatc ttacggatgg catgacagta agagaattat gcagtgctgc 1860
   cataaccatg agtgataaca ctgcggccaa cttacttctg acaacgatcg gaggaccgaa 1920
   ggagctaacc gcttttttgc acaacatggg ggatcatgta actcgccttg atcgttggga 1980
   accggagctg aatgaagcca taccaaacga cgagcgtgac accacgatgc ctgcagcaat 2040
   ggcaacaacg ttgcgcaaac tattaactgg cgaactactt actctagctt cccggcaaca 2100
   attaatagac tggatggagg cggataaagt tgcaggacca cttctgcgct cggcccttcc 2160
   ggctggctgg tttattgctg ataaatctgg agccggtgag cgtgggtctc gcggtatcat 2220
   tgcagcactg gggccagatg gtaagccctc ccgtatcgta gttatctaca cgacggggag 2280
   tcaggcaact atggatgaac gaaatagaca gatcgctgag ataggtgcct cactgattaa 2340
   gcattggtaa ctgtcagacc aagtttactc atatatactt tagattgatt taaaacttca 2400
   tttttaattt aaaaggatct aggtgaagat cctttttgat aatctcatga ccaaaatccc 2460
   ttaacgtgag ttttcgttcc actgagcgtc agaccccgta gaaaagatca aaggatcttc 2520
   ttgagatcct ttttttctgc gcgtaatctg ctgcttgcaa acaaaaaaac caccgctacc 2580
   agcggtggtt tgtttgccgg atcaagagct accaactctt tttccgaagg taactggctt 2640
   cagcagagcg cagataccaa atactgtcct tctagtgtag ccgtagttag gccaccactt 2700
   caagaactct gtagcaccgc ctacatacct cgctctgcta atcctgttac cagtggctgc 2760
   tgccagtggc gataagtcgt gtcttaccgg gttggactca agacgatagt taccggataa 2820
   ggcgcagcgg tcgggctgaa cggggggttc gtgcacacag cccagcttgg agcgaacgac 2880
   ctacaccgaa ctgagatacc tacagcgtga gctatgagaa agcgccacgc ttcccgaagg 2940
   gagaaaggcg gacaggtatc cggtaagcgg cagggtcgga acaggagagc gcacgaggga 3000
   gcttccaggg ggaaacgcct ggtatcttta tagtcctgtc gggtttcgcc acctctgact 3060
   tgagcgtcga tttttgtgat gctcgtcagg ggggcggagc ctatggaaaa acgccagcaa 3120
   cgcggccttt ttacggttcc tggccttttg ctggcctttt gctcacatgt tctttcctgc 3180
   gttatcccct gattctgtgg ataaccgtat taccgccttt gagtgagctg ataccgctcg 3240
   ccgcagccga acgaccgagc gcagcgagtc agtgagcgag gaagcggaag agcgcctgat 3300
   gcggtatttt ctccttacgc atctgtgcgg tatttcacac cgcataaatt ccgacaccat 3360
   cgaatggtgc aaaacctttc gcggtatggc atgatagcgc ccggaagaga gtcaattcag 3420
   ggtggtgaat gtgaaaccag taacgttata cgatgtcgca gagtatgccg gtgtctctta 3480
   tcagaccgtt tcccgcgtgg tgaaccaggc cagccacgtt tctgcgaaaa cgcgggaaaa 3540
   agtggaagcg gcgatggcgg agctgaatta cattcccaac cgcgtggcac aacaactggc 3600
   gggcaaacag tcgttgctga ttggcgttgc cacctccagt ctggccctgc acgcgccgtc 3660
   gcaaattgtc gcggcgatta aatctcgcgc cgatcaactg ggtgccagcg tggtggtgtc 3720
   gatggtagaa cgaagcggcg tcgaagcctg taaagcggcg gtgcacaatc ttctcgcgca 3780
   acgcgtcagt gggctgatca ttaactatcc gctggatgac caggatgcca ttgctgtgga 3840
   agctgcctgc actaatgttc cggcgttatt tcttgatgtc tctgaccaga cacccatcaa 3900
   cagtattatt ttctcccatg aagacggtac gcgactgggc gtggagcatc tggtcgcatt 3960
   gggtcaccag caaatcgcgc tgttagcggg cccattaagt tctgtctcgg cgcgtctgcg 4020
   tctggctggc tggcataaat atctcactcg caatcaaatt cagccgatag cggaacggga 4080
   aggcgactgg agtgccatgt ccggttttca acaaaccatg caaatgctga atgagggcat 4140
   cgttcccact gcgatgctgg ttgccaacga tcagatggcg ctgggcgcaa tgcgcgccat 4200
   taccgagtcc gggctgcgcg ttggtgcgga tatctcggta gtgggatacg acgataccga 4260
   agacagctca tgttatatcc cgccgttaac caccatcaaa caggattttc gcctgctggg 4320
   gcaaaccagc gtggaccgct tgctgcaact ctctcagggc caggcggtga agggcaatca 4380
   gctgttgccc gtctcactgg tgaaaagaaa aaccaccctg gcgcccaata cgcaaaccgc 4440
   ctctccccgc gcgttggccg attcattaat gcagctggca cgacaggttt cccgactgga 4500
   aagcgggcag tgagcgcaac gcaattaatg tgagttagct cactcattag gcaccccagg 4560
   ctttacactt tatgcttccg gctcgtatgt tgtgtggaat tgtgagcgga taacaatttc 4620
   acacaggaaa cagctatgac catgattacg gattcactgg ccgtcgtttt acaacgtcgt 4680
   gactgggaaa accctggcgt tacccaactt aatcgccttg cagcacatcc ccctttcgcc 4740
   agctggcgta atagcgaaga ggcccgcacc gatcgccctt cccaacagtt gcgcagcctg 4800
   aatggcgaat ggcgctttgc ctggtttccg gcaccagaag cggtgccgga aagctggctg 4860
   gagtgcgatc ttcctgaggc cgatactgtc gtcgtcccct caaactggca gatgcacggt 4920
   tacgatgcgc ccatctacac caacgtaacc tatcccatta cggtcaatcc gccgtttgtt 4980
   cccacggaga atccgacggg ttgttactcg ctcacattta atgttgatga aagctggcta 5040
   caggaaggcc agacgcgaat tatttttgat ggcgttggaa tt                    5082
<210> 69
   <211> 271
   <212> PRT
   <213> Séquence artificielle
<220>
   <223> Description de la séquence artificielle: pGEXkt-DP-TME1_G354&358L
<400> 69
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Leu Ser Gly Gly
       210                 215                 220
   Gly Gly Gly Leu Val Pro Arg Gly Ser Asp Pro Ile Ala Gly Ala His
   225                 230                 235                 240
   Trp Leu Val Leu Ala Leu Ile Ala Tyr Phe Ser Met Val Gly Asn Trp
                   245                 250                 255
   Ala Lys Val Leu Val Val Leu Leu Leu Phe Ala Gly Val Asp Ala
               260                 265                 270
<210> 70
   <211> 5914
   <212> ADN
   <213> Séquence artificielle
<220>
   <223> Description de la séquence artificielle: Vecteur pGEXKT-DP-TME2 + TrX-DP-TME1_G354L
<400> 70
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cggaatacgt tgttctgctg ttcctgctgc tggctgacgc tcgtgtttgc tcttgcctgt 1020
   ggatgatgct gctgatctct caggctgaag cttaggaatt cgcccttttc agtggctgtg 1080
   catgcaagga gatggcgccc aacagtcccc cggccacggg gcctgccacc atacccacgc 1140
   cgaaacaagc gctcatgagc ccgaagtggc gagcccgatc ttccccatcg gtgatgtcgg 1200
   cgatataggc gccagcaacc gcacctgtgg cgccggtgat gccggccacg atgcgtccgg 1260
   cgtagaggat cgagatcgat ctcgatcccg cgaaattaat acgactcact ataggggaat 1320
   tgtgagcgga taacaattcc cctctagaaa taattttgtt taactttaag aaggagatat 1380
   acatatgagc gataaaatta ttcacctgac tgacgacagt tttgacacgg atgtactcaa 1440
   agcggacggg gcgatcctcg tcgatttctg ggcagagtgg tgcggtccgt gcaaaatgat 1500
   cgccccgatt ctggatgaaa tcgctgacga atatcagggc aaactgaccg ttgcaaaact 1560
   gaacatcgat caaaaccctg gcactgcgcc gaaatatggc atccgtggta tcccgactct 1620
   gctgctgttc aaaaacggtg aagtggcggc aaccaaagtg ggtgcactgt ctaaaggtca 1680
   gttgaaagag ttcctcgacg ctaacctggc cggttctggt tctggatctg atctgtctgg 1740
   tggtggtggt ggtctggttc cgcgtggatc cgacccgatc gctggtgctc actggctggt 1800
   tctggctggt atcgcttact tctctatggt tggtaactgg gctaaagttc tggttgttct 1860
   gctgctgttc gctggtgttg acgcttagca gtggctgaaa agggcgaatt catcgtgact 1920
   gactgacgat ctgcctcgcg cgtttcggtg atgacggtga aaacctctga cacatgcagc 1980
   tcccggagac ggtcacagct tgtctgtaag cggatgccgg gagcagacaa gcccgtcagg 2040
   gcgcgtcagc gggtgttggc gggtgtcggg gcgcagccat gacccagtca cgtagcgata 2100
   gcggagtgta taattcttga agacgaaagg gcctcgtgat acgcctattt ttataggtta 2160
   atgtcatgat aataatggtt tcttagacgt caggtggcac ttttcgggga aatgtgcgcg 2220
   gaacccctat ttgtttattt ttctaaatac attcaaatat gtatccgctc atgagacaat 2280
   aaccctgata aatgcttcaa taatattgaa aaaggaagag tatgagtatt caacatttcc 2340
   gtgtcgccct tattcccttt tttgcggcat tttgccttcc tgtttttgct cacccagaaa 2400
   cgctggtgaa agtaaaagat gctgaagatc agttgggtgc acgagtgggt tacatcgaac 2460
   tggatctcaa cagcggtaag atccttgaga gttttcgccc cgaagaacgt tttccaatga 2520
   tgagcacttt taaagttctg ctatgtggcg cggtattatc ccgtgttgac gccgggcaag 2580
   agcaactcgg tcgccgcata cactattctc agaatgactt ggttgagtac tcaccagtca 2640
   cagaaaagca tcttacggat ggcatgacag taagagaatt atgcagtgct gccataacca 2700
   tgagtgataa cactgcggcc aacttacttc tgacaacgat cggaggaccg aaggagctaa 2760
   ccgctttttt gcacaacatg ggggatcatg taactcgcct tgatcgttgg gaaccggagc 2820
   tgaatgaagc cataccaaac gacgagcgtg acaccacgat gcctgcagca atggcaacaa 2880
   cgttgcgcaa actattaact ggcgaactac ttactctagc ttcccggcaa caattaatag 2940
   actggatgga ggcggataaa gttgcaggac cacttctgcg ctcggccctt ccggctggct 3000
   ggtttattgc tgataaatct ggagccggtg agcgtgggtc tcgcggtatc attgcagcac 3060
   tggggccaga tggtaagccc tcccgtatcg tagttatcta cacgacgggg agtcaggcaa 3120
   ctatggatga acgaaataga cagatcgctg agataggtgc ctcactgatt aagcattggt 3180
   aactgtcaga ccaagtttac tcatatatac tttagattga tttaaaactt catttttaat 3240
   ttaaaaggat ctaggtgaag atcctttttg ataatctcat gaccaaaatc ccttaacgtg 3300
   agttttcgtt ccactgagcg tcagaccccg tagaaaagat caaaggatct tcttgagatc 3360
   ctttttttct gcgcgtaatc tgctgcttgc aaacaaaaaa accaccgcta ccagcggtgg 3420
   tttgtttgcc ggatcaagag ctaccaactc tttttccgaa ggtaactggc ttcagcagag 3480
   cgcagatacc aaatactgtc cttctagtgt agccgtagtt aggccaccac ttcaagaact 3540
   ctgtagcacc gcctacatac ctcgctctgc taatcctgtt accagtggct gctgccagtg 3600
   gcgataagtc gtgtcttacc gggttggact caagacgata gttaccggat aaggcgcagc 3660
   ggtcgggctg aacggggggt tcgtgcacac agcccagctt ggagcgaacg acctacaccg 3720
   aactgagata cctacagcgt gagctatgag aaagcgccac gcttcccgaa gggagaaagg 3780
   cggacaggta tccggtaagc ggcagggtcg gaacaggaga gcgcacgagg gagcttccag 3840
   ggggaaacgc ctggtatctt tatagtcctg tcgggtttcg ccacctctga cttgagcgtc 3900
   gatttttgtg atgctcgtca ggggggcgga gcctatggaa aaacgccagc aacgcggcct 3960
   ttttacggtt cctggccttt tgctggcctt ttgctcacat gttctttcct gcgttatccc 4020
   ctgattctgt ggataaccgt attaccgcct ttgagtgagc tgataccgct cgccgcagcc 4080
   gaacgaccga gcgcagcgag tcagtgagcg aggaagcgga agagcgcctg atgcggtatt 4140
   ttctccttac gcatctgtgc ggtatttcac accgcataaa ttccgacacc atcgaatggt 4200
   gcaaaacctt tcgcggtatg gcatgatagc gcccggaaga gagtcaattc agggtggtga 4260
   atgtgaaacc agtaacgtta tacgatgtcg cagagtatgc cggtgtctct tatcagaccg 4320
   tttcccgcgt ggtgaaccag gccagccacg tttctgcgaa aacgcgggaa aaagtggaag 4380
   cggcgatggc ggagctgaat tacattccca accgcgtggc acaacaactg gcgggcaaac 4440
   agtcgttgct gattggcgtt gccacctcca gtctggccct gcacgcgccg tcgcaaattg 4500
   tcgcggcgat taaatctcgc gccgatcaac tgggtgccag cgtggtggtg tcgatggtag 4560
   aacgaagcgg cgtcgaagcc tgtaaagcgg cggtgcacaa tcttctcgcg caacgcgtca 4620
   gtgggctgat cattaactat ccgctggatg accaggatgc cattgctgtg gaagctgcct 4680
   gcactaatgt tccggcgtta tttcttgatg tctctgacca gacacccatc aacagtatta 4740
   ttttctccca tgaagacggt acgcgactgg gcgtggagca tctggtcgca ttgggtcacc 4800
   agcaaatcgc gctgttagcg ggcccattaa gttctgtctc ggcgcgtctg cgtctggctg 4860
   gctggcataa atatctcact cgcaatcaaa ttcagccgat agcggaacgg gaaggcgact 4920
   ggagtgccat gtccggtttt caacaaacca tgcaaatgct gaatgagggc atcgttccca 4980
   ctgcgatgct ggttgccaac gatcagatgg cgctgggcgc aatgcgcgcc attaccgagt 5040
   ccgggctgcg cgttggtgcg gatatctcgg tagtgggata cgacgatacc gaagacagct 5100
   catgttatat cccgccgtta accaccatca aacaggattt tcgcctgctg gggcaaacca 5160
   gcgtggaccg cttgctgcaa ctctctcagg gccaggcggt gaagggcaat cagctgttgc 5220
   ccgtctcact ggtgaaaaga aaaaccaccc tggcgcccaa tacgcaaacc gcctctcccc 5280
   gcgcgttggc cgattcatta atgcagctgg cacgacaggt ttcccgactg gaaagcgggc 5340
   agtgagcgca acgcaattaa tgtgagttag ctcactcatt aggcacccca ggctttacac 5400
   tttatgcttc cggctcgtat gttgtgtgga attgtgagcg gataacaatt tcacacagga 5460
   aacagctatg accatgatta cggattcact ggccgtcgtt ttacaacgtc gtgactggga 5520
   aaaccctggc gttacccaac ttaatcgcct tgcagcacat ccccctttcg ccagctggcg 5580
   taatagcgaa gaggcccgca ccgatcgccc ttcccaacag ttgcgcagcc tgaatggcga 5640
   atggcgcttt gcctggtttc cggcaccaga agcggtgccg gaaagctggc tggagtgcga 5700
   tcttcctgag gccgatactg tcgtcgtccc ctcaaactgg cagatgcacg gttacgatgc 5760
   gcccatctac accaacgtaa cctatcccat tacggtcaat ccgccgtttg ttcccacgga 5820
   gaatccgacg ggttgttact cgctcacatt taatgttgat gaaagctggc tacaggaagg 5880
   ccagacgcga attatttttg atggcgttgg aatt                             5914
<210> 71
   <211> 5914
   <212> ADN
   <213> Séquence artificielle
<220>
   <223> Description de la séquence artificielle: Vecteur pGEXKT-DP-TME2 + TrX-DP-TME1_G358L
<400> 71
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cggaatacgt tgttctgctg ttcctgctgc tggctgacgc tcgtgtttgc tcttgcctgt 1020
   ggatgatgct gctgatctct caggctgaag cttaggaatt cgcccttttc agtggctgtg 1080
   catgcaagga gatggcgccc aacagtcccc cggccacggg gcctgccacc atacccacgc 1140
   cgaaacaagc gctcatgagc ccgaagtggc gagcccgatc ttccccatcg gtgatgtcgg 1200
   cgatataggc gccagcaacc gcacctgtgg cgccggtgat gccggccacg atgcgtccgg 1260
   cgtagaggat cgagatcgat ctcgatcccg cgaaattaat acgactcact ataggggaat 1320
   tgtgagcgga taacaattcc cctctagaaa taattttgtt taactttaag aaggagatat 1380
   acatatgagc gataaaatta ttcacctgac tgacgacagt tttgacacgg atgtactcaa 1440
   agcggacggg gcgatcctcg tcgatttctg ggcagagtgg tgcggtccgt gcaaaatgat 1500
   cgccccgatt ctggatgaaa tcgctgacga atatcagggc aaactgaccg ttgcaaaact 1560
   gaacatcgat caaaaccctg gcactgcgcc gaaatatggc atccgtggta tcccgactct 1620
   gctgctgttc aaaaacggtg aagtggcggc aaccaaagtg ggtgcactgt ctaaaggtca 1680
   gttgaaagag ttcctcgacg ctaacctggc cggttctggt tctggatctg atctgtctgg 1740
   tggtggtggt ggtctggttc cgcgtggatc cgacccgatc gctggtgctc actggggtgt 1800
   tctggctctg atcgcttact tctctatggt tggtaactgg gctaaagttc tggttgttct 1860
   gctgctgttc gctggtgttg acgcttagca gtggctgaaa agggcgaatt catcgtgact 1920
   gactgacgat ctgcctcgcg cgtttcggtg atgacggtga aaacctctga cacatgcagc 1980
   tcccggagac ggtcacagct tgtctgtaag cggatgccgg gagcagacaa gcccgtcagg 2040
   gcgcgtcagc gggtgttggc gggtgtcggg gcgcagccat gacccagtca cgtagcgata 2100
   gcggagtgta taattcttga agacgaaagg gcctcgtgat acgcctattt ttataggtta 2160
   atgtcatgat aataatggtt tcttagacgt caggtggcac ttttcgggga aatgtgcgcg 2220
   gaacccctat ttgtttattt ttctaaatac attcaaatat gtatccgctc atgagacaat 2280
   aaccctgata aatgcttcaa taatattgaa aaaggaagag tatgagtatt caacatttcc 2340
   gtgtcgccct tattcccttt tttgcggcat tttgccttcc tgtttttgct cacccagaaa 2400
   cgctggtgaa agtaaaagat gctgaagatc agttgggtgc acgagtgggt tacatcgaac 2460
   tggatctcaa cagcggtaag atccttgaga gttttcgccc cgaagaacgt tttccaatga 2520
   tgagcacttt taaagttctg ctatgtggcg cggtattatc ccgtgttgac gccgggcaag 2580
   agcaactcgg tcgccgcata cactattctc agaatgactt ggttgagtac tcaccagtca 2640
   cagaaaagca tcttacggat ggcatgacag taagagaatt atgcagtgct gccataacca 2700
   tgagtgataa cactgcggcc aacttacttc tgacaacgat cggaggaccg aaggagctaa 2760
   ccgctttttt gcacaacatg ggggatcatg taactcgcct tgatcgttgg gaaccggagc 2820
   tgaatgaagc cataccaaac gacgagcgtg acaccacgat gcctgcagca atggcaacaa 2880
   cgttgcgcaa actattaact ggcgaactac ttactctagc ttcccggcaa caattaatag 2940
   actggatgga ggcggataaa gttgcaggac cacttctgcg ctcggccctt ccggctggct 3000
   ggtttattgc tgataaatct ggagccggtg agcgtgggtc tcgcggtatc attgcagcac 3060
   tggggccaga tggtaagccc tcccgtatcg tagttatcta cacgacgggg agtcaggcaa 3120
   ctatggatga acgaaataga cagatcgctg agataggtgc ctcactgatt aagcattggt 3180
   aactgtcaga ccaagtttac tcatatatac tttagattga tttaaaactt catttttaat 3240
   ttaaaaggat ctaggtgaag atcctttttg ataatctcat gaccaaaatc ccttaacgtg 3300
   agttttcgtt ccactgagcg tcagaccccg tagaaaagat caaaggatct tcttgagatc 3360
   ctttttttct gcgcgtaatc tgctgcttgc aaacaaaaaa accaccgcta ccagcggtgg 3420
   tttgtttgcc ggatcaagag ctaccaactc tttttccgaa ggtaactggc ttcagcagag 3480
   cgcagatacc aaatactgtc cttctagtgt agccgtagtt aggccaccac ttcaagaact 3540
   ctgtagcacc gcctacatac ctcgctctgc taatcctgtt accagtggct gctgccagtg 3600
   gcgataagtc gtgtcttacc gggttggact caagacgata gttaccggat aaggcgcagc 3660
   ggtcgggctg aacggggggt tcgtgcacac agcccagctt ggagcgaacg acctacaccg 3720
   aactgagata cctacagcgt gagctatgag aaagcgccac gcttcccgaa gggagaaagg 3780
   cggacaggta tccggtaagc ggcagggtcg gaacaggaga gcgcacgagg gagcttccag 3840
   ggggaaacgc ctggtatctt tatagtcctg tcgggtttcg ccacctctga cttgagcgtc 3900
   gatttttgtg atgctcgtca ggggggcgga gcctatggaa aaacgccagc aacgcggcct 3960
   ttttacggtt cctggccttt tgctggcctt ttgctcacat gttctttcct gcgttatccc 4020
   ctgattctgt ggataaccgt attaccgcct ttgagtgagc tgataccgct cgccgcagcc 4080
   gaacgaccga gcgcagcgag tcagtgagcg aggaagcgga agagcgcctg atgcggtatt 4140
   ttctccttac gcatctgtgc ggtatttcac accgcataaa ttccgacacc atcgaatggt 4200
   gcaaaacctt tcgcggtatg gcatgatagc gcccggaaga gagtcaattc agggtggtga 4260
   atgtgaaacc agtaacgtta tacgatgtcg cagagtatgc cggtgtctct tatcagaccg 4320
   tttcccgcgt ggtgaaccag gccagccacg tttctgcgaa aacgcgggaa aaagtggaag 4380
   cggcgatggc ggagctgaat tacattccca accgcgtggc acaacaactg gcgggcaaac 4440
   agtcgttgct gattggcgtt gccacctcca gtctggccct gcacgcgccg tcgcaaattg 4500
   tcgcggcgat taaatctcgc gccgatcaac tgggtgccag cgtggtggtg tcgatggtag 4560
   aacgaagcgg cgtcgaagcc tgtaaagcgg cggtgcacaa tcttctcgcg caacgcgtca 4620
   gtgggctgat cattaactat ccgctggatg accaggatgc cattgctgtg gaagctgcct 4680
   gcactaatgt tccggcgtta tttcttgatg tctctgacca gacacccatc aacagtatta 4740
   ttttctccca tgaagacggt acgcgactgg gcgtggagca tctggtcgca ttgggtcacc 4800
   agcaaatcgc gctgttagcg ggcccattaa gttctgtctc ggcgcgtctg cgtctggctg 4860
   gctggcataa atatctcact cgcaatcaaa ttcagccgat agcggaacgg gaaggcgact 4920
   ggagtgccat gtccggtttt caacaaacca tgcaaatgct gaatgagggc atcgttccca 4980
   ctgcgatgct ggttgccaac gatcagatgg cgctgggcgc aatgcgcgcc attaccgagt 5040
   ccgggctgcg cgttggtgcg gatatctcgg tagtgggata cgacgatacc gaagacagct 5100
   catgttatat cccgccgtta accaccatca aacaggattt tcgcctgctg gggcaaacca 5160
   gcgtggaccg cttgctgcaa ctctctcagg gccaggcggt gaagggcaat cagctgttgc 5220
   ccgtctcact ggtgaaaaga aaaaccaccc tggcgcccaa tacgcaaacc gcctctcccc 5280
   gcgcgttggc cgattcatta atgcagctgg cacgacaggt ttcccgactg gaaagcgggc 5340
   agtgagcgca acgcaattaa tgtgagttag ctcactcatt aggcacccca ggctttacac 5400
   tttatgcttc cggctcgtat gttgtgtgga attgtgagcg gataacaatt tcacacagga 5460
   aacagctatg accatgatta cggattcact ggccgtcgtt ttacaacgtc gtgactggga 5520
   aaaccctggc gttacccaac ttaatcgcct tgcagcacat ccccctttcg ccagctggcg 5580
   taatagcgaa gaggcccgca ccgatcgccc ttcccaacag ttgcgcagcc tgaatggcga 5640
   atggcgcttt gcctggtttc cggcaccaga agcggtgccg gaaagctggc tggagtgcga 5700
   tcttcctgag gccgatactg tcgtcgtccc ctcaaactgg cagatgcacg gttacgatgc 5760
   gcccatctac accaacgtaa cctatcccat tacggtcaat ccgccgtttg ttcccacgga 5820
   gaatccgacg ggttgttact cgctcacatt taatgttgat gaaagctggc tacaggaagg 5880
   ccagacgcga attatttttg atggcgttgg aatt                             5914
<210> 72
   <211> 5914
   <212> ADN
   <213> Séquence artificielle
<220>
   <223> Description de la séquence artificielle: Vecteur pGEXKT-DP-TME2 + TrX-DP-TME1_G354&358L
<400> 72
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cggaatacgt tgttctgctg ttcctgctgc tggctgacgc tcgtgtttgc tcttgcctgt 1020
   ggatgatgct gctgatctct caggctgaag cttaggaatt cgcccttttc agtggctgtg 1080
   catgcaagga gatggcgccc aacagtcccc cggccacggg gcctgccacc atacccacgc 1140
   cgaaacaagc gctcatgagc ccgaagtggc gagcccgatc ttccccatcg gtgatgtcgg 1200
   cgatataggc gccagcaacc gcacctgtgg cgccggtgat gccggccacg atgcgtccgg 1260
   cgtagaggat cgagatcgat ctcgatcccg cgaaattaat acgactcact ataggggaat 1320
   tgtgagcgga taacaattcc cctctagaaa taattttgtt taactttaag aaggagatat 1380
   acatatgagc gataaaatta ttcacctgac tgacgacagt tttgacacgg atgtactcaa 1440
   agcggacggg gcgatcctcg tcgatttctg ggcagagtgg tgcggtccgt gcaaaatgat 1500
   cgccccgatt ctggatgaaa tcgctgacga atatcagggc aaactgaccg ttgcaaaact 1560
   gaacatcgat caaaaccctg gcactgcgcc gaaatatggc atccgtggta tcccgactct 1620
   gctgctgttc aaaaacggtg aagtggcggc aaccaaagtg ggtgcactgt ctaaaggtca 1680
   gttgaaagag ttcctcgacg ctaacctggc cggttctggt tctggatctg atctgtctgg 1740
   tggtggtggt ggtctggttc cgcgtggatc cgacccgatc gctggtgctc actggctggt 1800
   tctggctctg atcgcttact tctctatggt tggtaactgg gctaaagttc tggttgttct 1860
   gctgctgttc gctggtgttg acgcttagca gtggctgaaa agggcgaatt catcgtgact 1920
   gactgacgat ctgcctcgcg cgtttcggtg atgacggtga aaacctctga cacatgcagc 1980
   tcccggagac ggtcacagct tgtctgtaag cggatgccgg gagcagacaa gcccgtcagg 2040
   gcgcgtcagc gggtgttggc gggtgtcggg gcgcagccat gacccagtca cgtagcgata 2100
   gcggagtgta taattcttga agacgaaagg gcctcgtgat acgcctattt ttataggtta 2160
   atgtcatgat aataatggtt tcttagacgt caggtggcac ttttcgggga aatgtgcgcg 2220
   gaacccctat ttgtttattt ttctaaatac attcaaatat gtatccgctc atgagacaat 2280
   aaccctgata aatgcttcaa taatattgaa aaaggaagag tatgagtatt caacatttcc 2340
   gtgtcgccct tattcccttt tttgcggcat tttgccttcc tgtttttgct cacccagaaa 2400
   cgctggtgaa agtaaaagat gctgaagatc agttgggtgc acgagtgggt tacatcgaac 2460
   tggatctcaa cagcggtaag atccttgaga gttttcgccc cgaagaacgt tttccaatga 2520
   tgagcacttt taaagttctg ctatgtggcg cggtattatc ccgtgttgac gccgggcaag 2580
   agcaactcgg tcgccgcata cactattctc agaatgactt ggttgagtac tcaccagtca 2640
   cagaaaagca tcttacggat ggcatgacag taagagaatt atgcagtgct gccataacca 2700
   tgagtgataa cactgcggcc aacttacttc tgacaacgat cggaggaccg aaggagctaa 2760
   ccgctttttt gcacaacatg ggggatcatg taactcgcct tgatcgttgg gaaccggagc 2820
   tgaatgaagc cataccaaac gacgagcgtg acaccacgat gcctgcagca atggcaacaa 2880
   cgttgcgcaa actattaact ggcgaactac ttactctagc ttcccggcaa caattaatag 2940
   actggatgga ggcggataaa gttgcaggac cacttctgcg ctcggccctt ccggctggct 3000
   ggtttattgc tgataaatct ggagccggtg agcgtgggtc tcgcggtatc attgcagcac 3060
   tggggccaga tggtaagccc tcccgtatcg tagttatcta cacgacgggg agtcaggcaa 3120
   ctatggatga acgaaataga cagatcgctg agataggtgc ctcactgatt aagcattggt 3180
   aactgtcaga ccaagtttac tcatatatac tttagattga tttaaaactt catttttaat 3240
   ttaaaaggat ctaggtgaag atcctttttg ataatctcat gaccaaaatc ccttaacgtg 3300
   agttttcgtt ccactgagcg tcagaccccg tagaaaagat caaaggatct tcttgagatc 3360
   ctttttttct gcgcgtaatc tgctgcttgc aaacaaaaaa accaccgcta ccagcggtgg 3420
   tttgtttgcc ggatcaagag ctaccaactc tttttccgaa ggtaactggc ttcagcagag 3480
   cgcagatacc aaatactgtc cttctagtgt agccgtagtt aggccaccac ttcaagaact 3540
   ctgtagcacc gcctacatac ctcgctctgc taatcctgtt accagtggct gctgccagtg 3600
   gcgataagtc gtgtcttacc gggttggact caagacgata gttaccggat aaggcgcagc 3660
   ggtcgggctg aacggggggt tcgtgcacac agcccagctt ggagcgaacg acctacaccg 3720
   aactgagata cctacagcgt gagctatgag aaagcgccac gcttcccgaa gggagaaagg 3780
   cggacaggta tccggtaagc ggcagggtcg gaacaggaga gcgcacgagg gagcttccag 3840
   ggggaaacgc ctggtatctt tatagtcctg tcgggtttcg ccacctctga cttgagcgtc 3900
   gatttttgtg atgctcgtca ggggggcgga gcctatggaa aaacgccagc aacgcggcct 3960
   ttttacggtt cctggccttt tgctggcctt ttgctcacat gttctttcct gcgttatccc 4020
   ctgattctgt ggataaccgt attaccgcct ttgagtgagc tgataccgct cgccgcagcc 4080
   gaacgaccga gcgcagcgag tcagtgagcg aggaagcgga agagcgcctg atgcggtatt 4140
   ttctccttac gcatctgtgc ggtatttcac accgcataaa ttccgacacc atcgaatggt 4200
   gcaaaacctt tcgcggtatg gcatgatagc gcccggaaga gagtcaattc agggtggtga 4260
   atgtgaaacc agtaacgtta tacgatgtcg cagagtatgc cggtgtctct tatcagaccg 4320
   tttcccgcgt ggtgaaccag gccagccacg tttctgcgaa aacgcgggaa aaagtggaag 4380
   cggcgatggc ggagctgaat tacattccca accgcgtggc acaacaactg gcgggcaaac 4440
   agtcgttgct gattggcgtt gccacctcca gtctggccct gcacgcgccg tcgcaaattg 4500
   tcgcggcgat taaatctcgc gccgatcaac tgggtgccag cgtggtggtg tcgatggtag 4560
   aacgaagcgg cgtcgaagcc tgtaaagcgg cggtgcacaa tcttctcgcg caacgcgtca 4620
   gtgggctgat cattaactat ccgctggatg accaggatgc cattgctgtg gaagctgcct 4680
   gcactaatgt tccggcgtta tttcttgatg tctctgacca gacacccatc aacagtatta 4740
   ttttctccca tgaagacggt acgcgactgg gcgtggagca tctggtcgca ttgggtcacc 4800
   agcaaatcgc gctgttagcg ggcccattaa gttctgtctc ggcgcgtctg cgtctggctg 4860
   gctggcataa atatctcact cgcaatcaaa ttcagccgat agcggaacgg gaaggcgact 4920
   ggagtgccat gtccggtttt caacaaacca tgcaaatgct gaatgagggc atcgttccca 4980
   ctgcgatgct ggttgccaac gatcagatgg cgctgggcgc aatgcgcgcc attaccgagt 5040
   ccgggctgcg cgttggtgcg gatatctcgg tagtgggata cgacgatacc gaagacagct 5100
   catgttatat cccgccgtta accaccatca aacaggattt tcgcctgctg gggcaaacca 5160
   gcgtggaccg cttgctgcaa ctctctcagg gccaggcggt gaagggcaat cagctgttgc 5220
   ccgtctcact ggtgaaaaga aaaaccaccc tggcgcccaa tacgcaaacc gcctctcccc 5280
   gcgcgttggc cgattcatta atgcagctgg cacgacaggt ttcccgactg gaaagcgggc 5340
   agtgagcgca acgcaattaa tgtgagttag ctcactcatt aggcacccca ggctttacac 5400
   tttatgcttc cggctcgtat gttgtgtgga attgtgagcg gataacaatt tcacacagga 5460
   aacagctatg accatgatta cggattcact ggccgtcgtt ttacaacgtc gtgactggga 5520
   aaaccctggc gttacccaac ttaatcgcct tgcagcacat ccccctttcg ccagctggcg 5580
   taatagcgaa gaggcccgca ccgatcgccc ttcccaacag ttgcgcagcc tgaatggcga 5640
   atggcgcttt gcctggtttc cggcaccaga agcggtgccg gaaagctggc tggagtgcga 5700
   tcttcctgag gccgatactg tcgtcgtccc ctcaaactgg cagatgcacg gttacgatgc 5760
   gcccatctac accaacgtaa cctatcccat tacggtcaat ccgccgtttg ttcccacgga 5820
   gaatccgacg ggttgttact cgctcacatt taatgttgat gaaagctggc tacaggaagg 5880
   ccagacgcga attatttttg atggcgttgg aatt                            5914
<210> 73
   <211> 5914
   <212> ADN
   <213> Séquence artificielle
<220>
   <223> Description de la séquence artificielle: Vecteur pGEXKT-DP-TME2_C731&C733A + TrX-DP-TME1_G354L
<400> 73
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cggaatacgt tgttctgctg ttcctgctgc tggctgacgc tcgtgttgct tctgctctgt 1020
   ggatgatgct gctgatctct caggctgaag cttaggaatt cgcccttttc agtggctgtg 1080
   catgcaagga gatggcgccc aacagtcccc cggccacggg gcctgccacc atacccacgc 1140
   cgaaacaagc gctcatgagc ccgaagtggc gagcccgatc ttccccatcg gtgatgtcgg 1200
   cgatataggc gccagcaacc gcacctgtgg cgccggtgat gccggccacg atgcgtccgg 1260
   cgtagaggat cgagatcgat ctcgatcccg cgaaattaat acgactcact ataggggaat 1320
   tgtgagcgga taacaattcc cctctagaaa taattttgtt taactttaag aaggagatat 1380
   acatatgagc gataaaatta ttcacctgac tgacgacagt tttgacacgg atgtactcaa 1440
   agcggacggg gcgatcctcg tcgatttctg ggcagagtgg tgcggtccgt gcaaaatgat 1500
   cgccccgatt ctggatgaaa tcgctgacga atatcagggc aaactgaccg ttgcaaaact 1560
   gaacatcgat caaaaccctg gcactgcgcc gaaatatggc atccgtggta tcccgactct 1620
   gctgctgttc aaaaacggtg aagtggcggc aaccaaagtg ggtgcactgt ctaaaggtca 1680
   gttgaaagag ttcctcgacg ctaacctggc cggttctggt tctggatctg atctgtctgg 1740
   tggtggtggt ggtctggttc cgcgtggatc cgacccgatc gctggtgctc actggctggt 1800
   tctggctggt atcgcttact tctctatggt tggtaactgg gctaaagttc tggttgttct 1860
   gctgctgttc gctggtgttg acgcttagca gtggctgaaa agggcgaatt catcgtgact 1920
   gactgacgat ctgcctcgcg cgtttcggtg atgacggtga aaacctctga cacatgcagc 1980
   tcccggagac ggtcacagct tgtctgtaag cggatgccgg gagcagacaa gcccgtcagg 2040
   gcgcgtcagc gggtgttggc gggtgtcggg gcgcagccat gacccagtca cgtagcgata 2100
   gcggagtgta taattcttga agacgaaagg gcctcgtgat acgcctattt ttataggtta 2160
   atgtcatgat aataatggtt tcttagacgt caggtggcac ttttcgggga aatgtgcgcg 2220
   gaacccctat ttgtttattt ttctaaatac attcaaatat gtatccgctc atgagacaat 2280
   aaccctgata aatgcttcaa taatattgaa aaaggaagag tatgagtatt caacatttcc 2340
   gtgtcgccct tattcccttt tttgcggcat tttgccttcc tgtttttgct cacccagaaa 2400
   cgctggtgaa agtaaaagat gctgaagatc agttgggtgc acgagtgggt tacatcgaac 2460
   tggatctcaa cagcggtaag atccttgaga gttttcgccc cgaagaacgt tttccaatga 2520
   tgagcacttt taaagttctg ctatgtggcg cggtattatc ccgtgttgac gccgggcaag 2580
   agcaactcgg tcgccgcata cactattctc agaatgactt ggttgagtac tcaccagtca 2640
   cagaaaagca tcttacggat ggcatgacag taagagaatt atgcagtgct gccataacca 2700
   tgagtgataa cactgcggcc aacttacttc tgacaacgat cggaggaccg aaggagctaa 2760
   ccgctttttt gcacaacatg ggggatcatg taactcgcct tgatcgttgg gaaccggagc 2820
   tgaatgaagc cataccaaac gacgagcgtg acaccacgat gcctgcagca atggcaacaa 2880
   cgttgcgcaa actattaact ggcgaactac ttactctagc ttcccggcaa caattaatag 2940
   actggatgga ggcggataaa gttgcaggac cacttctgcg ctcggccctt ccggctggct 3000
   ggtttattgc tgataaatct ggagccggtg agcgtgggtc tcgcggtatc attgcagcac 3060
   tggggccaga tggtaagccc tcccgtatcg tagttatcta cacgacgggg agtcaggcaa 3120
   ctatggatga acgaaataga cagatcgctg agataggtgc ctcactgatt aagcattggt 3180
   aactgtcaga ccaagtttac tcatatatac tttagattga tttaaaactt catttttaat 3240
   ttaaaaggat ctaggtgaag atcctttttg ataatctcat gaccaaaatc ccttaacgtg 3300
   agttttcgtt ccactgagcg tcagaccccg tagaaaagat caaaggatct tcttgagatc 3360
   ctttttttct gcgcgtaatc tgctgcttgc aaacaaaaaa accaccgcta ccagcggtgg 3420
   tttgtttgcc ggatcaagag ctaccaactc tttttccgaa ggtaactggc ttcagcagag 3480
   cgcagatacc aaatactgtc cttctagtgt agccgtagtt aggccaccac ttcaagaact 3540
   ctgtagcacc gcctacatac ctcgctctgc taatcctgtt accagtggct gctgccagtg 3600
   gcgataagtc gtgtcttacc gggttggact caagacgata gttaccggat aaggcgcagc 3660
   ggtcgggctg aacggggggt tcgtgcacac agcccagctt ggagcgaacg acctacaccg 3720
   aactgagata cctacagcgt gagctatgag aaagcgccac gcttcccgaa gggagaaagg 3780
   cggacaggta tccggtaagc ggcagggtcg gaacaggaga gcgcacgagg gagcttccag 3840
   ggggaaacgc ctggtatctt tatagtcctg tcgggtttcg ccacctctga cttgagcgtc 3900
   gatttttgtg atgctcgtca ggggggcgga gcctatggaa aaacgccagc aacgcggcct 3960
   ttttacggtt cctggccttt tgctggcctt ttgctcacat gttctttcct gcgttatccc 4020
   ctgattctgt ggataaccgt attaccgcct ttgagtgagc tgataccgct cgccgcagcc 4080
   gaacgaccga gcgcagcgag tcagtgagcg aggaagcgga agagcgcctg atgcggtatt 4140
   ttctccttac gcatctgtgc ggtatttcac accgcataaa ttccgacacc atcgaatggt 4200
   gcaaaacctt tcgcggtatg gcatgatagc gcccggaaga gagtcaattc agggtggtga 4260
   atgtgaaacc agtaacgtta tacgatgtcg cagagtatgc cggtgtctct tatcagaccg 4320
   tttcccgcgt ggtgaaccag gccagccacg tttctgcgaa aacgcgggaa aaagtggaag 4380
   cggcgatggc ggagctgaat tacattccca accgcgtggc acaacaactg gcgggcaaac 4440
   agtcgttgct gattggcgtt gccacctcca gtctggccct gcacgcgccg tcgcaaattg 4500
   tcgcggcgat taaatctcgc gccgatcaac tgggtgccag cgtggtggtg tcgatggtag 4560
   aacgaagcgg cgtcgaagcc tgtaaagcgg cggtgcacaa tcttctcgcg caacgcgtca 4620
   gtgggctgat cattaactat ccgctggatg accaggatgc cattgctgtg gaagctgcct 4680
   gcactaatgt tccggcgtta tttcttgatg tctctgacca gacacccatc aacagtatta 4740
   ttttctccca tgaagacggt acgcgactgg gcgtggagca tctggtcgca ttgggtcacc 4800
   agcaaatcgc gctgttagcg ggcccattaa gttctgtctc ggcgcgtctg cgtctggctg 4860
   gctggcataa atatctcact cgcaatcaaa ttcagccgat agcggaacgg gaaggcgact 4920
   ggagtgccat gtccggtttt caacaaacca tgcaaatgct gaatgagggc atcgttccca 4980
   ctgcgatgct ggttgccaac gatcagatgg cgctgggcgc aatgcgcgcc attaccgagt 5040
   ccgggctgcg cgttggtgcg gatatctcgg tagtgggata cgacgatacc gaagacagct 5100
   catgttatat cccgccgtta accaccatca aacaggattt tcgcctgctg gggcaaacca 5160
   gcgtggaccg cttgctgcaa ctctctcagg gccaggcggt gaagggcaat cagctgttgc 5220
   ccgtctcact ggtgaaaaga aaaaccaccc tggcgcccaa tacgcaaacc gcctctcccc 5280
   gcgcgttggc cgattcatta atgcagctgg cacgacaggt ttcccgactg gaaagcgggc 5340
   agtgagcgca acgcaattaa tgtgagttag ctcactcatt aggcacccca ggctttacac 5400
   tttatgcttc cggctcgtat gttgtgtgga attgtgagcg gataacaatt tcacacagga 5460
   aacagctatg accatgatta cggattcact ggccgtcgtt ttacaacgtc gtgactggga 5520
   aaaccctggc gttacccaac ttaatcgcct tgcagcacat ccccctttcg ccagctggcg 5580
   taatagcgaa gaggcccgca ccgatcgccc ttcccaacag ttgcgcagcc tgaatggcga 5640
   atggcgcttt gcctggtttc cggcaccaga agcggtgccg gaaagctggc tggagtgcga 5700
   tcttcctgag gccgatactg tcgtcgtccc ctcaaactgg cagatgcacg gttacgatgc 5760
   gcccatctac accaacgtaa cctatcccat tacggtcaat ccgccgtttg ttcccacgga 5820
   gaatccgacg ggttgttact cgctcacatt taatgttgat gaaagctggc tacaggaagg 5880
   ccagacgcga attatttttg atggcgttgg aatt 5914
<210> 74
   <211> 5914
   <212> ADN
   <213> Séquence artificielle
<220>
   <223> Description de la séquence artificielle: Vecteur pGEXKT-DP-TME2_C731&C733A + TrX-DP-TME1_G358L
<400> 74
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cggaatacgt tgttctgctg ttcctgctgc tggctgacgc tcgtgttgct tctgctctgt 1020
   ggatgatgct gctgatctct caggctgaag cttaggaatt cgcccttttc agtggctgtg 1080
   catgcaagga gatggcgccc aacagtcccc cggccacggg gcctgccacc atacccacgc 1140
   cgaaacaagc gctcatgagc ccgaagtggc gagcccgatc ttccccatcg gtgatgtcgg 1200
   cgatataggc gccagcaacc gcacctgtgg cgccggtgat gccggccacg atgcgtccgg 1260
   cgtagaggat cgagatcgat ctcgatcccg cgaaattaat acgactcact ataggggaat 1320
   tgtgagcgga taacaattcc cctctagaaa taattttgtt taactttaag aaggagatat 1380
   acatatgagc gataaaatta ttcacctgac tgacgacagt tttgacacgg atgtactcaa 1440
   agcggacggg gcgatcctcg tcgatttctg ggcagagtgg tgcggtccgt gcaaaatgat 1500
   cgccccgatt ctggatgaaa tcgctgacga atatcagggc aaactgaccg ttgcaaaact 1560
   gaacatcgat caaaaccctg gcactgcgcc gaaatatggc atccgtggta tcccgactct 1620
   gctgctgttc aaaaacggtg aagtggcggc aaccaaagtg ggtgcactgt ctaaaggtca 1680
   gttgaaagag ttcctcgacg ctaacctggc cggttctggt tctggatctg atctgtctgg 1740
   tggtggtggt ggtctggttc cgcgtggatc cgacccgatc gctggtgctc actggggtgt 1800
   tctggctctg atcgcttact tctctatggt tggtaactgg gctaaagttc tggttgttct 1860
   gctgctgttc gctggtgttg acgcttagca gtggctgaaa agggcgaatt catcgtgact 1920
   gactgacgat ctgcctcgcg cgtttcggtg atgacggtga aaacctctga cacatgcagc 1980
   tcccggagac ggtcacagct tgtctgtaag cggatgccgg gagcagacaa gcccgtcagg 2040
   gcgcgtcagc gggtgttggc gggtgtcggg gcgcagccat gacccagtca cgtagcgata 2100
   gcggagtgta taattcttga agacgaaagg gcctcgtgat acgcctattt ttataggtta 2160
   atgtcatgat aataatggtt tcttagacgt caggtggcac ttttcgggga aatgtgcgcg 2220
   gaacccctat ttgtttattt ttctaaatac attcaaatat gtatccgctc atgagacaat 2280
   aaccctgata aatgcttcaa taatattgaa aaaggaagag tatgagtatt caacatttcc 2340
   gtgtcgccct tattcccttt tttgcggcat tttgccttcc tgtttttgct cacccagaaa 2400
   cgctggtgaa agtaaaagat gctgaagatc agttgggtgc acgagtgggt tacatcgaac 2460
   tggatctcaa cagcggtaag atccttgaga gttttcgccc cgaagaacgt tttccaatga 2520
   tgagcacttt taaagttctg ctatgtggcg cggtattatc ccgtgttgac gccgggcaag 2580
   agcaactcgg tcgccgcata cactattctc agaatgactt ggttgagtac tcaccagtca 2640
   cagaaaagca tcttacggat ggcatgacag taagagaatt atgcagtgct gccataacca 2700
   tgagtgataa cactgcggcc aacttacttc tgacaacgat cggaggaccg aaggagctaa 2760
   ccgctttttt gcacaacatg ggggatcatg taactcgcct tgatcgttgg gaaccggagc 2820
   tgaatgaagc cataccaaac gacgagcgtg acaccacgat gcctgcagca atggcaacaa 2880
   cgttgcgcaa actattaact ggcgaactac ttactctagc ttcccggcaa caattaatag 2940
   actggatgga ggcggataaa gttgcaggac cacttctgcg ctcggccctt ccggctggct 3000
   ggtttattgc tgataaatct ggagccggtg agcgtgggtc tcgcggtatc attgcagcac 3060
   tggggccaga tggtaagccc tcccgtatcg tagttatcta cacgacgggg agtcaggcaa 3120
   ctatggatga acgaaataga cagatcgctg agataggtgc ctcactgatt aagcattggt 3180
   aactgtcaga ccaagtttac tcatatatac tttagattga tttaaaactt catttttaat 3240
   ttaaaaggat ctaggtgaag atcctttttg ataatctcat gaccaaaatc ccttaacgtg 3300
   agttttcgtt ccactgagcg tcagaccccg tagaaaagat caaaggatct tcttgagatc 3360
   ctttttttct gcgcgtaatc tgctgcttgc aaacaaaaaa accaccgcta ccagcggtgg 3420
   tttgtttgcc ggatcaagag ctaccaactc tttttccgaa ggtaactggc ttcagcagag 3480
   cgcagatacc aaatactgtc cttctagtgt agccgtagtt aggccaccac ttcaagaact 3540
   ctgtagcacc gcctacatac ctcgctctgc taatcctgtt accagtggct gctgccagtg 3600
   gcgataagtc gtgtcttacc gggttggact caagacgata gttaccggat aaggcgcagc 3660
   ggtcgggctg aacggggggt tcgtgcacac agcccagctt ggagcgaacg acctacaccg 3720
   aactgagata cctacagcgt gagctatgag aaagcgccac gcttcccgaa gggagaaagg 3780
   cggacaggta tccggtaagc ggcagggtcg gaacaggaga gcgcacgagg gagcttccag 3840
   ggggaaacgc ctggtatctt tatagtcctg tcgggtttcg ccacctctga cttgagcgtc 3900
   gatttttgtg atgctcgtca ggggggcgga gcctatggaa aaacgccagc aacgcggcct 3960
   ttttacggtt cctggccttt tgctggcctt ttgctcacat gttctttcct gcgttatccc 4020
   ctgattctgt ggataaccgt attaccgcct ttgagtgagc tgataccgct cgccgcagcc 4080
   gaacgaccga gcgcagcgag tcagtgagcg aggaagcgga agagcgcctg atgcggtatt 4140
   ttctccttac gcatctgtgc ggtatttcac accgcataaa ttccgacacc atcgaatggt 4200
   gcaaaacctt tcgcggtatg gcatgatagc gcccggaaga gagtcaattc agggtggtga 4260
   atgtgaaacc agtaacgtta tacgatgtcg cagagtatgc cggtgtctct tatcagaccg 4320
   tttcccgcgt ggtgaaccag gccagccacg tttctgcgaa aacgcgggaa aaagtggaag 4380
   cggcgatggc ggagctgaat tacattccca accgcgtggc acaacaactg gcgggcaaac 4440
   agtcgttgct gattggcgtt gccacctcca gtctggccct gcacgcgccg tcgcaaattg 4500
   tcgcggcgat taaatctcgc gccgatcaac tgggtgccag cgtggtggtg tcgatggtag 4560
   aacgaagcgg cgtcgaagcc tgtaaagcgg cggtgcacaa tcttctcgcg caacgcgtca 4620
   gtgggctgat cattaactat ccgctggatg accaggatgc cattgctgtg gaagctgcct 4680
   gcactaatgt tccggcgtta tttcttgatg tctctgacca gacacccatc aacagtatta 4740
   ttttctccca tgaagacggt acgcgactgg gcgtggagca tctggtcgca ttgggtcacc 4800
   agcaaatcgc gctgttagcg ggcccattaa gttctgtctc ggcgcgtctg cgtctggctg 4860
   gctggcataa atatctcact cgcaatcaaa ttcagccgat agcggaacgg gaaggcgact 4920
   ggagtgccat gtccggtttt caacaaacca tgcaaatgct gaatgagggc atcgttccca 4980
   ctgcgatgct ggttgccaac gatcagatgg cgctgggcgc aatgcgcgcc attaccgagt 5040
   ccgggctgcg cgttggtgcg gatatctcgg tagtgggata cgacgatacc gaagacagct 5100
   catgttatat cccgccgtta accaccatca aacaggattt tcgcctgctg gggcaaacca 5160
   gcgtggaccg cttgctgcaa ctctctcagg gccaggcggt gaagggcaat cagctgttgc 5220
   ccgtctcact ggtgaaaaga aaaaccaccc tggcgcccaa tacgcaaacc gcctctcccc 5280
   gcgcgttggc cgattcatta atgcagctgg cacgacaggt ttcccgactg gaaagcgggc 5340
   agtgagcgca acgcaattaa tgtgagttag ctcactcatt aggcacccca ggctttacac 5400
   tttatgcttc cggctcgtat gttgtgtgga attgtgagcg gataacaatt tcacacagga 5460
   aacagctatg accatgatta cggattcact ggccgtcgtt ttacaacgtc gtgactggga 5520
   aaaccctggc gttacccaac ttaatcgcct tgcagcacat ccccctttcg ccagctggcg 5580
   taatagcgaa gaggcccgca ccgatcgccc ttcccaacag ttgcgcagcc tgaatggcga 5640
   atggcgcttt gcctggtttc cggcaccaga agcggtgccg gaaagctggc tggagtgcga 5700
   tcttcctgag gccgatactg tcgtcgtccc ctcaaactgg cagatgcacg gttacgatgc 5760
   gcccatctac accaacgtaa cctatcccat tacggtcaat ccgccgtttg ttcccacgga 5820
   gaatccgacg ggttgttact cgctcacatt taatgttgat gaaagctggc tacaggaagg 5880
   ccagacgcga attatttttg atggcgttgg aatt                            5914
<210> 75
   <211> 5914
   <212> ADN
   <213> Séquence artificielle
<220>
   <223> Description de la séquence artificielle: Vecteur pGEXKT-DP-TME2_C731&C733A + TrX-DP-TME1_G354&358L
<400> 75
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg tctggtggtg gtggtggtct ggttccgcgt ggatccgacc 960
   cggaatacgt tgttctgctg ttcctgctgc tggctgacgc tcgtgttgct tctgctctgt 1020
   ggatgatgct gctgatctct caggctgaag cttaggaatt cgcccttttc agtggctgtg 1080
   catgcaagga gatggcgccc aacagtcccc cggccacggg gcctgccacc atacccacgc 1140
   cgaaacaagc gctcatgagc ccgaagtggc gagcccgatc ttccccatcg gtgatgtcgg 1200
   cgatataggc gccagcaacc gcacctgtgg cgccggtgat gccggccacg atgcgtccgg 1260
   cgtagaggat cgagatcgat ctcgatcccg cgaaattaat acgactcact ataggggaat 1320
   tgtgagcgga taacaattcc cctctagaaa taattttgtt taactttaag aaggagatat 1380
   acatatgagc gataaaatta ttcacctgac tgacgacagt tttgacacgg atgtactcaa 1440
   agcggacggg gcgatcctcg tcgatttctg ggcagagtgg tgcggtccgt gcaaaatgat 1500
   cgccccgatt ctggatgaaa tcgctgacga atatcagggc aaactgaccg ttgcaaaact 1560
   gaacatcgat caaaaccctg gcactgcgcc gaaatatggc atccgtggta tcccgactct 1620
   gctgctgttc aaaaacggtg aagtggcggc aaccaaagtg ggtgcactgt ctaaaggtca 1680
   gttgaaagag ttcctcgacg ctaacctggc cggttctggt tctggatctg atctgtctgg 1740
   tggtggtggt ggtctggttc cgcgtggatc cgacccgatc gctggtgctc actggctggt 1800
   tctggctctg atcgcttact tctctatggt tggtaactgg gctaaagttc tggttgttct 1860
   gctgctgttc gctggtgttg acgcttagca gtggctgaaa agggcgaatt catcgtgact 1920
   gactgacgat ctgcctcgcg cgtttcggtg atgacggtga aaacctctga cacatgcagc 1980
   tcccggagac ggtcacagct tgtctgtaag cggatgccgg gagcagacaa gcccgtcagg 2040
   gcgcgtcagc gggtgttggc gggtgtcggg gcgcagccat gacccagtca cgtagcgata 2100
   gcggagtgta taattcttga agacgaaagg gcctcgtgat acgcctattt ttataggtta 2160
   atgtcatgat aataatggtt tcttagacgt caggtggcac ttttcgggga aatgtgcgcg 2220
   gaacccctat ttgtttattt ttctaaatac attcaaatat gtatccgctc atgagacaat 2280
   aaccctgata aatgcttcaa taatattgaa aaaggaagag tatgagtatt caacatttcc 2340
   gtgtcgccct tattcccttt tttgcggcat tttgccttcc tgtttttgct cacccagaaa 2400
   cgctggtgaa agtaaaagat gctgaagatc agttgggtgc acgagtgggt tacatcgaac 2460
   tggatctcaa cagcggtaag atccttgaga gttttcgccc cgaagaacgt tttccaatga 2520
   tgagcacttt taaagttctg ctatgtggcg cggtattatc ccgtgttgac gccgggcaag 2580
   agcaactcgg tcgccgcata cactattctc agaatgactt ggttgagtac tcaccagtca 2640
   cagaaaagca tcttacggat ggcatgacag taagagaatt atgcagtgct gccataacca 2700
   tgagtgataa cactgcggcc aacttacttc tgacaacgat cggaggaccg aaggagctaa 2760
   ccgctttttt gcacaacatg ggggatcatg taactcgcct tgatcgttgg gaaccggagc 2820
   tgaatgaagc cataccaaac gacgagcgtg acaccacgat gcctgcagca atggcaacaa 2880
   cgttgcgcaa actattaact ggcgaactac ttactctagc ttcccggcaa caattaatag 2940
   actggatgga ggcggataaa gttgcaggac cacttctgcg ctcggccctt ccggctggct 3000
   ggtttattgc tgataaatct ggagccggtg agcgtgggtc tcgcggtatc attgcagcac 3060
   tggggccaga tggtaagccc tcccgtatcg tagttatcta cacgacgggg agtcaggcaa 3120
   ctatggatga acgaaataga cagatcgctg agataggtgc ctcactgatt aagcattggt 3180
   aactgtcaga ccaagtttac tcatatatac tttagattga tttaaaactt catttttaat 3240
   ttaaaaggat ctaggtgaag atcctttttg ataatctcat gaccaaaatc ccttaacgtg 3300
   agttttcgtt ccactgagcg tcagaccccg tagaaaagat caaaggatct tcttgagatc 3360
   ctttttttct gcgcgtaatc tgctgcttgc aaacaaaaaa accaccgcta ccagcggtgg 3420
   tttgtttgcc ggatcaagag ctaccaactc tttttccgaa ggtaactggc ttcagcagag 3480
   cgcagatacc aaatactgtc cttctagtgt agccgtagtt aggccaccac ttcaagaact 3540
   ctgtagcacc gcctacatac ctcgctctgc taatcctgtt accagtggct gctgccagtg 3600
   gcgataagtc gtgtcttacc gggttggact caagacgata gttaccggat aaggcgcagc 3660
   ggtcgggctg aacggggggt tcgtgcacac agcccagctt ggagcgaacg acctacaccg 3720
   aactgagata cctacagcgt gagctatgag aaagcgccac gcttcccgaa gggagaaagg 3780
   cggacaggta tccggtaagc ggcagggtcg gaacaggaga gcgcacgagg gagcttccag 3840
   ggggaaacgc ctggtatctt tatagtcctg tcgggtttcg ccacctctga cttgagcgtc 3900
   gatttttgtg atgctcgtca ggggggcgga gcctatggaa aaacgccagc aacgcggcct 3960
   ttttacggtt cctggccttt tgctggcctt ttgctcacat gttctttcct gcgttatccc 4020
   ctgattctgt ggataaccgt attaccgcct ttgagtgagc tgataccgct cgccgcagcc 4080
   gaacgaccga gcgcagcgag tcagtgagcg aggaagcgga agagcgcctg atgcggtatt 4140
   ttctccttac gcatctgtgc ggtatttcac accgcataaa ttccgacacc atcgaatggt 4200
   gcaaaacctt tcgcggtatg gcatgatagc gcccggaaga gagtcaattc agggtggtga 4260
   atgtgaaacc agtaacgtta tacgatgtcg cagagtatgc cggtgtctct tatcagaccg 4320
   tttcccgcgt ggtgaaccag gccagccacg tttctgcgaa aacgcgggaa aaagtggaag 4380
   cggcgatggc ggagctgaat tacattccca accgcgtggc acaacaactg gcgggcaaac 4440
   agtcgttgct gattggcgtt gccacctcca gtctggccct gcacgcgccg tcgcaaattg 4500
   tcgcggcgat taaatctcgc gccgatcaac tgggtgccag cgtggtggtg tcgatggtag 4560
   aacgaagcgg cgtcgaagcc tgtaaagcgg cggtgcacaa tcttctcgcg caacgcgtca 4620
   gtgggctgat cattaactat ccgctggatg accaggatgc cattgctgtg gaagctgcct 4680
   gcactaatgt tccggcgtta tttcttgatg tctctgacca gacacccatc aacagtatta 4740
   ttttctccca tgaagacggt acgcgactgg gcgtggagca tctggtcgca ttgggtcacc 4800
   agcaaatcgc gctgttagcg ggcccattaa gttctgtctc ggcgcgtctg cgtctggctg 4860
   gctggcataa atatctcact cgcaatcaaa ttcagccgat agcggaacgg gaaggcgact 4920
   ggagtgccat gtccggtttt caacaaacca tgcaaatgct gaatgagggc atcgttccca 4980
   ctgcgatgct ggttgccaac gatcagatgg cgctgggcgc aatgcgcgcc attaccgagt 5040
   ccgggctgcg cgttggtgcg gatatctcgg tagtgggata cgacgatacc gaagacagct 5100
   catgttatat cccgccgtta accaccatca aacaggattt tcgcctgctg gggcaaacca 5160
   gcgtggaccg cttgctgcaa ctctctcagg gccaggcggt gaagggcaat cagctgttgc 5220
   ccgtctcact ggtgaaaaga aaaaccaccc tggcgcccaa tacgcaaacc gcctctcccc 5280
   gcgcgttggc cgattcatta atgcagctgg cacgacaggt ttcccgactg gaaagcgggc 5340
   agtgagcgca acgcaattaa tgtgagttag ctcactcatt aggcacccca ggctttacac 5400
   tttatgcttc cggctcgtat gttgtgtgga attgtgagcg gataacaatt tcacacagga 5460
   aacagctatg accatgatta cggattcact ggccgtcgtt ttacaacgtc gtgactggga 5520
   aaaccctggc gttacccaac ttaatcgcct tgcagcacat ccccctttcg ccagctggcg 5580
   taatagcgaa gaggcccgca ccgatcgccc ttcccaacag ttgcgcagcc tgaatggcga 5640
   atggcgcttt gcctggtttc cggcaccaga agcggtgccg gaaagctggc tggagtgcga 5700
   tcttcctgag gccgatactg tcgtcgtccc ctcaaactgg cagatgcacg gttacgatgc 5760
   gcccatctac accaacgtaa cctatcccat tacggtcaat ccgccgtttg ttcccacgga 5820
   gaatccgacg ggttgttact cgctcacatt taatgttgat gaaagctggc tacaggaagg 5880
   ccagacgcga attatttttg atggcgttgg aatt                             5914
<210> 76
   <211> 74
   <212> ADN
   <213> Séquence artificielle
<220>
   <223> Description de la séquence artificielle: oligonucléotide OL11(+) permettant de générer l'ADN codant pour TME1
<400> 76
   atgccatatg atcgctggtg ctcactgggg tgttctggct ggtatcgctt acttctctat 60
   ggttggtaac tggg                                                   74
<210> 77
   <211> 79
   <212> ADN
   <213> Séquence artificielle
<220>
   <223> Description de la séquence artificielle: oligonucléotide OL12(-) permettant de générer l'ADN codant pour TME1
<400> 77
   gcatatcgat ctaagcgtca acaccagcga acagcagcag aacaaccaga actttagccc 60
   agttaccaac catagagaa                                              79
<210> 78
   <211> 28
   <212> ADN
   <213> Séquence artificielle
<220>
   <223> Description de la séquence artificielle: oligonucléotide OL17 (+) permettant d'amplifier l'ADN codant pour TME1 pour insertion dans le plasmide pGEXKT
<400> 78
   ggatccgacc cgatggaata cgttgttc 28
<210> 79
   <211> 68
   <212> ADN
   <213> Séquence artificielle
<220>
   <223> Description de la séquence artificielle: oligonucléotide OL21 (+) permettant de générer l'ADN codant pour TME2
<400> 79
   catatggaat acgttgttct gctgttcctg ctgctggctg acgctcgtgt ttgctcttgc 60
   ctgtggat                                                          68
<210> 80
   <211> 57
   <212> ADN
   <213> Séquence artificielle
<220>
   <223> Description de la séquence artificielle: oligonucléotide OL22(-) permettant de générer l'ADN codant pour TME2
<400> 80
   aagcttaagc ttcagcctga gagatcagca gcatcatcca caggcaagag caaacac 57
<210> 81
   <211> 25
   <212> ADN
   <213> Séquence artificielle
<220>
   <223> Description de la séquence artificielle: oligonucléotide OL27(+) permettant d'amplifier l'ADN codant pour TME2 pour insertion dans le plasmide pGEXKT
<400> 81
   ggatccgacc cggaatacgt tgttc 25

## Description

### DOMAINE TECHNIQUE

La présente invention se rapporte à un vecteur de co-expression de domaines membranaires de protéines d'enveloppe d'un virus, ainsi qu'à un procédé de fabrication d'homo- et/ou hétéro-oligomères de ces domaines. Ces domaines membranaires sont les domaines des protéines d'enveloppe des virus qui permettent leur ancrage sur les cellules cibles qu'elles vont infecter.

Le vecteur de la présente invention permet par exemple la co-expression des domaines membranaires TME1 et TME2 des protéines de l'enveloppe du virus de l'hépatite C, et la fabrication d'homo- et/ou hétéro-oligomères de ces domaines TME1 et TME2.

La présente invention fournit un vecteur permettant de manière générale de produire les différents états d'association des domaines membranaires des protéines d'enveloppe des virus lors de leur constitution.

Ce vecteur permet par exemple de tester à grande échelle des composés chimiques ou biologiques, par exemple des peptides, susceptibles de perturber la formation des différents états d'association des domaines membranaires des protéines d'enveloppe des virus, et donc potentiellement de perturber la formation ou la fixation du virus sur ses cellules cibles hôtes.

La présente invention se rapporte donc également à un procédé de criblage permettant d'identifier des composés chimiques ou biologiques qui perturbent la formation des différents états d'association de domaines membranaires de protéines d'enveloppe des virus.

Elle trouve donc de nombreuses applications notamment dans la recherche concernant les mécanismes d'infections virales et dans recherche et la mise au point de nouveaux principes actifs pour lutter contre les infections virales.

Dans la description qui suit, les références entre crochets [ ] renvoient à la liste de références annexée.

### ETAT DE LA TECHNIQUE ANTERIEURE

La détermination de la structure tridimensionnelle (3D) est une étape décisive dans la compréhension structurale et fonctionnelle des protéines. Pour cela, il est nécessaire de pouvoir fabriquer des quantités suffisantes des protéines étudiées, et, si possible, que les protéines fabriquées retrouvent leur conformation fonctionnelle.

De très grands efforts et moyens ont été, et sont, mis en oeuvre pour parvenir à ce but, et se sont amplifiés avec l'accumulation des données apportées par les programmes de séquençage des génomes.

Dans ce contexte, la bactérie est un moyen de production largement utilisé par la communauté scientifique. La surexpression de protéines dans la bactérie ne s'effectue cependant pas sans problèmes. En effet, elle donne le plus souvent lieu aux deux cas de figure suivants :
- Le premier cas, le plus courant, est celui où la protéine est surproduite et agrégée sous la forme de corps d'inclusion. Cela concerne les protéines polytopiques et/ou de grande taille. Dans ce cas, la cinétique de repliement de la protéine est nettement plus lente que sa vitesse de biosynthèse. Cela favorise l'exposition au solvant aqueux des régions hydrophobes de la protéine qui sont normalement enfouies au coeur de celle-ci, et génère des interactions non spécifiques qui conduisent à la formation d'agrégats insolubles. Suivant le degré de désordre de ce repliement, les corps d'inclusions peuvent être solubilisés/dépliés dans des conditions non natives, avec de l'urée ou de la guanidine. La protéine solubilisée est ensuite soumise à différents traitements comme la dialyse ou la dilution pour accéder, dans certains cas seulement, à un repliement 3D natif.
- Le deuxième cas est celui où l'expression engendre une toxicité d'un degré variable. Celui-ci va de l'absence de produit d'expression si la cellule hôte parvient à s'adapter, à la mort de celle-ci si le produit est trop toxique. C'est un cas qui se présente assez fréquemment et le plus souvent avec des protéines ou des domaines de protéines membranaires ou membranaires, comme par exemple ceux des protéines d'enveloppe du virus de l'hépatite C [1] ou du virus d' immunodéficience humain [2].

Le problème de toxicité pour la cellule hôte concerne essentiellement l'expression de protéines membranaires, c'est à dire de protéines présentant un domaine hydrophobe. Or ces protéines présentent un intérêt grandissant. Elles sont d'une part relativement nombreuses puisque l'établissement des différents génomes confirme qu'elles représentent environ 30% des protéines potentiellement codées par ces génomes [3]. Elles constituent d'autre part 70% des cibles thérapeutiques et leur altération est à l'origine de nombreuses maladies génétiques [4].

Il est donc essentiel de mettre au point des méthodes facilitant ou permettant l'expression de telles protéines ou de leur partie membranaire.

Des efforts ont été faits dans ce sens avec par exemple la mise au point de souches bactériennes, soit tolérant mieux l'expression de protéines membranaires [5, 6], soit ayant une régulation plus stricte du mécanisme d'expression comme dans le cas de la souche *E. coli* BZ21(DE3)pLysS développée par Stratagene. Cependant, ces améliorations ne permettent pas de supprimer le phénomène de toxicité dans tous les cas, en particulier lors de l'expression de peptides hydrophobes correspondant à des ancres membranaires.

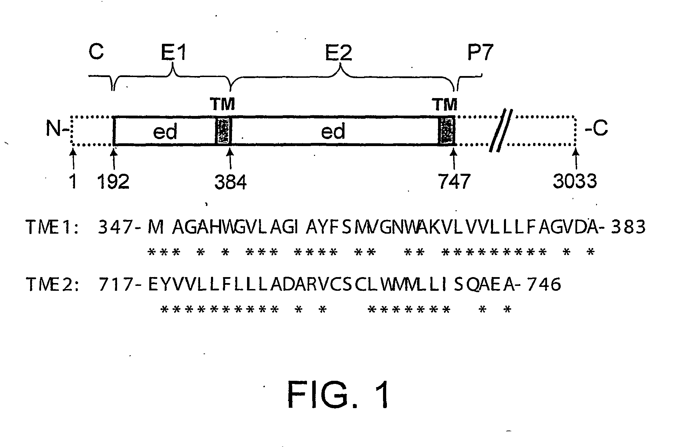

L'un des enjeux médicaux majeurs actuels concerne le traitement de l'hépatite C. Celle-ci est provoquée par le virus de l'hépatite C (VHC) de la famille des flaviviridae et qui infecte spécifiquement les cellules hépatiques [7]. Le VHC infecte 170 millions d'humains dans le monde. On estime que 75% des personnes séropositives développent une infection chronique [8]. Ce virus est constitué d'un ARN positif d'environ 9500 bases codant pour une polyprotéine de 3033 résidus [9], symbolisée sur la figure 1, qui, après expression, est clivée par des protéases endogènes et exogènes pour donner naissance à 10 protéines différentes. Deux d'entre elles, E1 et E2, sont glycosylées et forment l'enveloppe du virus.

Lors du processus de maturation du virus, les protéines E1 et E2 s'associent pour former des hétéro-oligomères dont on ignore encore la nature. E1 et E2 sont constituées chacune d'un ectodomaine et d'une région C-terminale, riche en acides aminés hydrophobes, qui forme un domaine membranaire assurant l'ancrage des protéines à la membrane du réticulum endoplasmique comme cela est exposé dans le document [10]. Chacun des ectodomaines ainsi que les domaines membranaires [11] sont impliqués dans le phénomène d'oligomérisation et conditionnent la structuration de l'enveloppe du virus. Etant impliqués dans le processus d'oligomérisation des protéines E1 et E2, les domaines membranaires TME1 et TME2 constituent des cibles thérapeutiques potentielles très intéressantes.

Les différents essais d'expression des protéines E1 ou E2 dans *E. coli* [12] [13] ou dans des cellules d'insecte *sf*9 infectées par baculovirus [14] ont été sans succès du fait de la toxicité induite par leur expression. Cette toxicité est essentiellement générée par les domaines membranaires. C'est un cas qui se présente assez fréquemment et le plus souvent avec des protéines ou des domaines de protéines membranaires, comme par exemple ceux des protéines d'enveloppe du VHC [13] ou du virus d'immunodéficience humain [15].

A ce jour les systèmes d'expression par recombinaison génétique mis au point ne permettent pas de fabriquer ces protéines membranaires. En outre, lorsque des segments membranaires, par exemple de TME1 ou de TME2 du virus de l'hépatite C, peuvent être obtenus, ils ne reproduisent jamais les états d'association naturels des protéines dans l'enveloppe du virus lorsque ces domaines sont mélangés.

Il existe donc un réel besoin d'un système permettant de produire des domaines membranaires coopérant dans leur conformation fonctionnelle dans l'enveloppe d'un virus, notamment pour la constitution de l'enveloppe du virus et/ou pour la reconnaissance et/ou la fixation du virus à sa cellule cible. Il est en plus souhaitable que ce système permette aux domaines produits de reproduire les différents états d'association de ces domaines lors de la constitution de l'enveloppe du virus et/ou dans la forme fonctionnelle de l'enveloppe dudit virus pour la reconnaissance et/ou la fixation du virus à sa cellule cible.

Ce système permettrait par exemple de tester à grande échelle des composés chimiques ou biologiques, par exemple des peptides, susceptibles de perturber la formation des différents états d'association des domaines membranaires, et donc potentiellement de perturber la formation du virus et/ou les mécanismes de reconnaissance de la cellule cible par le virus.

### EXPOSÉ DE L'INVENTION

L'objectif atteint par la présente invention est précisément celui de résoudre les problèmes précités de l'art antérieur et de répondre aux besoins précités en fournissant un système de co-expression de domaines membranaires coopérant ou interagissant dans leur conformation fonctionnelle dans l'enveloppe d'un virus, notamment pour la constitution de l'enveloppe du virus et/ou pour la reconnaissance et/ou la fixation du virus à sa cellule cible.

Le système de la présente invention est un vecteur de co-expression d'au moins deux domaines membranaires de protéines d'enveloppe d'un virus coopérant dans leur conformation fonctionnelle dans l'enveloppe du virus, ledit vecteur étant **caractérisé en ce qu**'il comprend :
- au moins une région de réplication et de maintien dudit vecteur dans la cellule hôte ;
- une première région constituée successivement, dans le sens de traduction du vecteur, d'un premier promoteur suivit d'une première séquence codant pour une première protéine chimère,
   la première séquence codant pour la première protéine chimère étant constituée dans ledit sens de traduction du vecteur, d'une première séquence codant pour une première protéine soluble, d'une séquence codant pour un dipeptide Asp-Pro et d'une séquence codant pour l'un desdits au moins deux domaines membranaires ; et
- une deuxième région constituée successivement, dans ledit sens de traduction du vecteur, d'un deuxième promoteur suivit d'une deuxième séquence codant pour une deuxième protéine chimère,
   la deuxième séquence codant pour la deuxième protéine chimère étant constituée dans ledit sens de traduction du vecteur, d'une deuxième séquence codant pour une deuxième protéine soluble, d'une séquence codant pour un dipeptide Asp-Pro et d'une séquence codant pour l'autre desdits au moins deux domaines membranaires.

Par « domaine de protéine membranaire » ou « domaine membranaire » on entend la partie d'une protéine d'enveloppe virale qui est hydrophobe en vue notamment de son ancrage sur la membrane des cellules cibles. Il peut s'agir bien entendu d'une protéine entière, qui est membranaire, ou d'une partie membranaire d'une protéine qui présente également un domaine hydrophile non membranaire.

Selon l'invention, avantageusement, la première et la deuxième régions sont contiguës mais la disposition de ces deux régions l'une par rapport à l'autre dans le vecteur ne présente à priori pas d'importance.

Selon l'invention, les première et deuxième protéines solubles peuvent être identiques ou différentes. Elles peuvent être choisies par exemple parmi la glutathion S-transférase (GST) et la thiorédoxine (TrX) ou toute autre protéine soluble équivalente. Les séquences peptidiques de la GST et de la TrX sont par exemple respectivement les séquences SEQ IDn°25 et SEQ IDn°37 de la liste de séquences annexée. Les séquences nucléotidiques codant pour la GST et de la TrX qui peuvent être utilisées dans le vecteur de la présente invention pour coder la GST et la TrX sont par exemple respectivement les séquences SEQ IDn°24 et SEQ IDn°36 de la liste de séquences annexée.

Selon l'invention, la séquence nucléotidique codant pour le dipeptide Asp-Pro peut être par exemple gacccg ou toute autre séquence nucléotidique codant pour ce dipeptide.

La séquence codant pour Asp-Pro (DP en code à une lettre) placée en amont de la séquence nucléotidique de chaque protéine membranaire, permet de manière tout à fait inattendue de supprimer l'effet toxique des protéines membranaires co-exprimées pour la cellule hôte. En outre, les inventeurs ont noté que, de manière tout à fait surprenante, la suppression de toxicité de la protéine chez l'hôte est encore plus efficace lorsque les peptides membranaires sont produits en fusion C-terminale avec une protéine soluble, par exemple la glutathion S-transférase ou la thiorédoxine, avec la séquence Asp-Pro insérée entre chaque protéine soluble et chaque peptide membranaire dans le vecteur de co-expression de la présente invention.

Le vecteur de la présente invention permet la surproduction en co-expression d'au moins deux domaines membranaires de protéines d'enveloppe d'un virus coopérant dans leur conformation fonctionnelle dans l'enveloppe du virus. Il s'agit de protéines membranaires également dans les cellules hôtes, en particulier de protéines hydrophobes, notamment de peptides qui correspondent à, ou qui comprennent, des domaines hydrophobes de protéines qui sont susceptibles de s'ancrer aux membranes des cellules hôtes. Il peut s'agir par exemple de protéines membranaires ou de domaines de protéines membranaires. Il peut s'agir par exemple de protéines d'enveloppe d'un virus, par exemple d'un virus de l'hépatite C, d'un virus du sida ou de tout autre virus pathogène pour l'homme et de manière générale pour les mammifères. Dans la présente invention, ces protéines d'enveloppe sont réduites à leur domaine membranaire, c'est à dire à leur domaine hydrophobe. C'est ce qui est entendu par l'expression « domaines membranaires ». Les virus concernés par la présente invention sont en fait tous les virus qui possèdent dans leur structure des protéines membranaires qui interagissent pour la constitution de l'enveloppe du virus et/ou pour la reconnaissance et/ou la fixation du virus à sa cellule cible.

Par exemple, dans le cas de protéines d'enveloppe du virus de l'hépatite C, le vecteur de la présente invention peut être un vecteur de co-expression des domaines membranaires TME1 et TME2. Ce vecteur permet la co-expression desdits domaines TME1 et TME2, correspondant en particulier aux segments 347-383 et 717-746 de la polyprotéine codée par l'ARN du virus et de séquences suivantes :
TME1 : 347-MIAGAHWGVLAGIAYFSMVGNWAKVLVVLLLFAGVDA-383 Séquence SEQ IDn°2
TME2 : 717-MEYVVLLFLLLADARVCSCLWMMLLISQAEA-746 Séquence SEQ IDn°16

Ainsi, selon l'invention, l'un des deux domaines membranaires peut avoir une séquence peptidique choisie parmi les séquences peptidiques SEQ IDn°2, SEQ IDn°10, SEQ IDn°12 et SEQ IDn°14 de la liste de séquences annexée, et l'autre des deux domaines a une séquence peptidique choisie parmi les séquences peptidiques SEQ IDn°16 et SEQ IDn°22 de la liste de séquences annexée.

Les séquences peptidiques SEQ IDn°10, SEQ IDn°12 et SEQ IDn°14 de la liste de séquences annexée correspondent à la séquence peptidique de TME1 (SEQ IDn°2) comportant des mutations ponctuelles. La séquence peptidique SEQ IDn°22 de la liste de séquences annexée correspond à la séquence peptidique de TME2 (SEQ IDn°16) comportant deux mutations ponctuelles. Le rôle de ces mutations ponctuelles conformes à la présente invention est expliqué ci-dessous.

Selon l'invention, dans le vecteur, la séquence nucléotidique codant pour l'un desdits au moins deux domaines membranaires peut par exemple être choisie parmi les séquences nucléotidiques SEQ IDn°1, SEQ IDn°9, SEQ IDn°11 et SEQ IDn°13 de la liste de séquences annexée, et la séquence nucléotidique codant pour l'autre desdits au moins deux domaines membranaires peut être choisie parmi les séquences SEQ IDn°15 et SEQ IDn°17 de la liste de séquences annexée. Ces séquence nucléotidiques codent respectivement pour les séquences peptidiques SEQ IDn°2, SEQ IDn°10, SEQ IDn°12, SEQ IDn°14, SEQ IDn°16 et SEQ IDn°22 précitées. L'homme du métier pourra aisément déterminer d'autres séquences nucléotidiques codant pour ces peptides ou peptides mutés.

Les peptides TME1 et TME2 sont respectivement produits en fusion C-terminale de protéines solubles, par exemple avec la glutathion S-transférase (GST) et/ou avec la thiorédoxine (TrX), pour former les chimères, par exemple GST-DP-TME1, GST-DP-TME2, TrX-DP-TME1 et TrX-DP-TME2.

Par exemple, dans le vecteur de la présente invention, la première protéine chimère peut être une protéine ayant une séquence choisie parmi les séquences SEQ IDn°28, SEQ IDn°43, SEQ IDn°46, SEQ IDn°49 et SEQ IDn°52, et la deuxième protéine chimère peut être une protéine ayant une séquence choisie parmi les séquences SEQ IDn°31, SEQ IDn°34, SEQ IDn°55 et SEQ IDn°58. Pour les mêmes raisons que celle évoquées ci-dessus, en particulier pour obtenir avantageusement des hétéro-oligomères des protéines co-exprimées, lorsque la première protéine chimère a la séquence SEQ IDn°28, l'autre protéine chimère est différente de SEQ IDn°31 et inversement.

Les protéines membranaires produites à partir du vecteur de la présente invention forment des monomères, dimères, trimères, et dans une moindre mesure des formes multimériques, qui sont parfois assez stables pour résister aux conditions dénaturantes de séparation sur gel de polyacrylamide en présence de SDS.

Les inventeurs ont découvert, de manière inattendue, pour les protéine membranaires TME1 et TME2 de l'enveloppe du virus de l'hépatite C, que quelque soient les forces d'interaction qui stabilisent ces formes oligomériques, elles peuvent être supprimées :
- par les mutations ponctuelles G354 et/ou G358L dans TME1 (la glycine en position 354 et/ou 358 de TME1 est remplacée par une leucine) ; et/ou
- par les mutations ponctuelles C731&C733A dans TME2 (les cystéines en position 731 et 733 de TME2 sont remplacées par une alanine).

Selon l'invention, il n'est pas nécessaire de produire toutes les mutations de TME1 et de TME2 dans le vecteur pour supprimer les forces d'interaction. Les mutations soit sur TME1 soit sur TME2, peuvent être suffisantes dans le vecteur de co-expression de la présente invention pour obtenir ce résultat. Ainsi, si des formes hétéro-oligomériques particulières sont désirées, de préférence, lorsque l'un des deux domaines est SEQ IDn°2, l'autre domaine est différent de SEQ IDn°16 et inversement. De la même manière, lorsque l'un des deux domaines est codé par la séquence SEQ IDn°1 (codant pour TME1), l'autre domaine codant est différent de SEQ IDn°15 (codant pour TME2) et inversement. Par le choix des mutations, selon l'invention, on peut donc obtenir préférentiellement certaines formes hétéro-oligomériques, ou aucune forme homo- ou hétéro-oligomérique.

Par exemple, dans un exemple particulier de réalisation de la présente invention, un vecteur a été construit en intégrant une région codant pour la chimère [protéine soluble-DP-TME1] et une région codant pour la chimère [protéine soluble-DP-TME2]. Cette co-expression a permis la formation d'une part des homo-oligomères observés avec des expressions indépendantes (essais sans co-expression), et d'autre part, de manière tout à fait surprenante, d'un hétéro-dimère {[protéine soluble-DP-TME1]₁-[protéine soluble-DP-TME2]₁} et un hétéro-trimère {[protéine soluble-DP-TME1]₂-[protéine soluble-DP-TME2]₁}. En outre, en utilisant un tel vecteur, mais avec la double mutation C731&C733A dans TME2, les inventeurs ont pu supprimer de façon remarquable non seulement les formes hétéro-oligomériques mais aussi le trimère [T_{DP}TME1]₃.

Des exemples de protéines chimères mutées de TME1 selon la présente invention sont GST-DP-TME1_G354L (SEQ IDn°65) ; GST-DP-TME1_G358L (SEQ IDn°67) ; et GST-DP-TME1_G354&358L (SEQ IDn°65) codées par exemple respectivement par les oligonucléotides de séquence SEQ IDn°64 ; SEQ IDn°66 et SEQ IDn°68 de la liste de séquences annexée. Dans ces exemples, GST peut aussi être TrX.

Des exemples de protéines chimères mutées de TME2 selon la présente invention sont GST-DP-TME2_C731&C733A de séquence peptidique SEQ IDn°34 ou TrX-DP-TME2_C731&C733A de séquence peptidique SEQ IDn°58, codées par exemple respectivement par les séquences oligonucléotidiques SEQ IDn° 33 et SEQ IDn°57 de la liste de séquences annexée.

Le vecteur de la présente invention peut être obtenu à partir de tout plasmide connu de l'homme du métier pour la recombinaison génétique, par exemple dans *E. coli*, et comprenant une région de réplication et de maintien dudit plasmide dans la cellule hôte, et des sites de restriction permettant d'insérer les régions codant pour les protéines chimères précitées. Le plasmide est choisi notamment en fonction de la cellule hôte dans laquelle il sera introduit pour la co-expression.

Le vecteur de l'invention peut être avantageusement obtenu à partir du plasmide pGEXKT de séquence SEQ IDn°23 de la liste de séquences annexée, ou du plasmide pET32a+ de séquence SEQ IDn°35 de la liste de séquences annexée. En effet, ils comprennent déjà une séquence codant pour une protéine soluble, respectivement pour la glutathion S-transférase (GST) et pour la thiorédoxine (TrX).

Dans le vecteur de l'invention, la région de réplication et de maintien dudit vecteur dans la cellule hôte est généralement déjà présente sur le plasmide choisi pour cloner les régions codant pour les protéines membranaires. Sinon, il peut être inséré. Ces régions sont connues de l'homme du métier.

Les promoteurs qui précèdent les séquences codantes pour les protéines chimères sont des séquences d'ADN que doit reconnaître l'ARN polymérase pour que soit initiée la transcription de ces régions, qui se déroule ensuite sous le contrôle de cette enzyme. Ces promoteurs sont connus de l'homme du métier.

Pour obtenir un vecteur selon l'invention, à partir d'un plasmide choisi pour le clonage des protéines membranaires à co-exprimer, il faut disposer des nucléotides codant pour lesdites protéines, auxquels sont adjointes, en amont dans le sens 5'->3' de chaque nucléotide et dans cet ordre, une séquence nucléotidique codant pour le dipeptide DP, et une séquence nucléotidique codant pour une protéine soluble. Les techniques habituelles de recombinaison génétique, connues de l'homme du métier sont utilisables. Brièvement, des enzymes de restriction permettant de couper le plasmide choisi en des endroits déterminés sont utilisés pour insérer dans le plasmide les régions codant pour les protéines membranaires à co-exprimer liées chacune à une séquence codante pour une protéine soluble par l'intermédiaire d'une séquence codante pour le dipeptide DP. Des techniques utilisables sont décrites par exemple dans [16]. On obtient alors un vecteur selon la présente invention.

A titre d'exemple, le vecteur de la présente invention peut être un vecteur de séquence oligonucléotidique SEQ IDn°61 ou SEQ IDn°62 de la liste de séquences annexée. Les protéines chimères co-exprimées avec ces vecteurs sont respectivement GST-DP-TME2+TrX-DP-TME1 (SEQ IDn°61) et GST-DP-TME2_C731&C733A+TrX-DP-TME1 (SEQ IDn°62).

A titre d'exemple également, le vecteur de la présente invention peut aussi être un des vecteurs suivants codant pour les protéines chimères suivantes :
- vecteur SEQ IDn°70 codant pour les protéines chimères GST-DP-TME2 + TrX-DP-TME1_G354L (SEQ IDn°31 + SEQ IDn°46) ;
- vecteur SEQ IDn°71 codant pour les protéines chimères GST-DP-TME2 + TrX-DP-TME1_G358L (SEQ IDn°31 + SEQ IDn°49) ;
- vecteur SEQ IDn°72 codant pour les protéines chimères GST-DP-TME2 + TrX-DP-TME1_G354&358L (SEQ IDn°31 + SEQ IDn°52);
- vecteur SEQ IDn°73 codant pour les protéines chimères TrX-DP-TME1_G354L + GST-DP-TME2_C731&733A (SEQ IDn°46 + SEQ IDn°34) ;
- vecteur SEQ IDn°74 codant pour les protéines chimères TrX-DP-TME1_G358L + GST-DP-TME2_C731&733A (SEQ IDn°49 + SEQ IDn°34) ; et
- vecteur SEQ IDn°75 codant pour les protéines chimères TrX-DP-TME1_G354&358L + GST-DP-TME2_C731&733A (SEQ IDn°52 + SEQ IDn°34).

Les autres combinaisons possibles avec les différentes protéines chimères présentées ci-dessus, par exemple avec TrX-DP-TME2 ou TrX-DP-TME2_C731&733A, ne sont pas décrites ici par soucis de concision uniquement, mais elles sont évidemment conformes à la présente invention.

Le vecteur de la présente invention permet de co-exprimer les protéines membranaires TME1 et TME2 de l'enveloppe du virus de l'hépatite C, et de reproduire des formes homo- et hétéro-oligomériques de ces protéines qui seraient présentes dans la formation de l'enveloppe du virus.

La présente invention se rapporte également à une cellule procaryote transformée par un vecteur d'expression selon l'invention. Cette cellule procaryote transformée par le vecteur d'expression de la présente invention doit de préférence permettre la surexpression des protéines membranaires co-exprimées pour lesquelles code le vecteur. Ainsi, toute cellule hôte capable d'exprimer le vecteur d'expression de la présente invention est utilisable. Il peut s'agir par exemple de *E.coli*, avantageusement, de la souche *E. coli* BL21(DE3)pLysS.

La présente invention se rapporte également à un procédé de fabrication par recombinaison génétique de formes hétéro-oligomériques ou d'un mélange d'au moins deux domaines membranaires de protéine d'enveloppe d'un virus qui interagissent dans leur conformation fonctionnelle dans l'enveloppe du virus, ledit procédé comprenant les étapes suivantes :
- transformer une cellule hôte avec un vecteur de co-expression selon l'invention,
- cultiver la cellule hôte transformée dans des conditions de culture telles qu'elle fabrique les formes hétéro-oligomériques ou le mélange des, au moins deux, domaines membranaires à partir dudit vecteur, et
- isoler lesdits hétéro-oligomères ou ledit mélange.

Les cellules hôtes utilisables sont décrites ci-dessus. Des vecteurs utilisables sont également décrits ci-dessus.

Ce procédé, grâce au plasmide de la présente invention, permet de fabriquer un ou plusieurs hétéro-oligomères ou un mélange d'au moins deux domaines membranaires de protéine d'enveloppe d'un virus, par exemple des domaines membranaires TME1 et TME2 des protéines d'enveloppe du virus de l'hépatite C. En effet, en appliquant les mutations ponctuelles adéquates pour altérer, voir supprimer, l'interaction entre les domaines membranaires fabriqués, et donc la formation des hétéro-oligomères, on peut obtenir, à partir du plasmide de la présente invention, un mélange de peptides mutés capables d'être utilisés dans les différentes applications décrites ci-dessous.

Ces formes hétéro-oligomériques ou ces mélanges peuvent se former à partir des protéines chimères, ou à partir des protéines membranaires fabriquées, séparées de leur protéine soluble et du dipeptide DP. En effet, un traitement de clivage des protéines chimères fabriquées peut être réalisé lors de l'étape consistant à isoler les protéines fabriquées , par exemple au moyen d'acide formique qui clive la protéine de fusion au niveau du dipeptide DP. Elle peut être réalisée par ailleurs par toute technique appropriée connue de l'homme du métier pour récupérer une protéine d'un échantillon à partir d'une protéine de fusion.

A ce titre, la présente invention se rapporte également à un hétéro-oligomère ou un mélange constitué d'au moins une première et une deuxième protéine,
- la première protéine ayant une séquence choisie parmi SEQ IDn°2, SEQ IDn°10, SEQ IDn°12, SEQ IDn°14, SEQ IDn°65, SEQ IDn°67 et SEQ IDn°69 de la liste de séquence annexée, et la deuxième protéine choisie parmi SEQ IDn°22, SEQ IDn°34 et SEQ IDn°58 ;
- ou la première protéine ayant une séquence choisie parmi SEQ IDn°14, SEQ IDn°65, SEQ IDn°67 et SEQ IDn°69 de la liste de séquence annexée, et la deuxième protéine ayant la séquence SEQ IDn°16

A ce titre également, ces protéines étant de tailles différentes et pouvant donc être séparées par exemple par électrophorèse, la présente invention se rapporte également à une protéine ayant une séquence peptidique choisie parmi les séquences SEQ IDn°14 de la liste de séquences annexée correspondant à la séquence peptidique mutée de TME1 ; et SEQ IDn°22, correspondant à la séquence peptidique mutée de TME2. En effet, ces protéines permettent d'empêcher, voire de supprimer la formation d'hétéro-oligomères.

Les protéines fabriquées peuvent être isolées des cellules hôtes par les techniques habituelles connues de l'homme du métier, pourvu que la technique utilisée n'altère pas l'oligomérisation des protéines fabriquées. Des techniques utilisables pour cette séparation sont par exemple les techniques électrophorétiques et d'immunodétection.

Dans cette démarche inventive, les inventeurs ont utilisé un vecteur d'expression en fusion avec la GST pour mettre en évidence des formes homo-oligomériques de ces chimères. Ils ont ensuite transposé ce système en remplaçant la GST par la thiorédoxine, ce qui a permis de produire les mêmes oligomères. Ils ont aussi montré que ceux-ci, malgré leur stabilité, ne sont pas maintenus lorsque l'on effectue certaines mutations. Le système a enfin été adapté pour permettre l'expression concomitante des chimères, ce qui a permis, de manière tout à fait inattendue, premièrement de révéler l'existence d'hétéro-oligomères et deuxièmement de montrer que les mutations peuvent limiter leur formation.

Ces associations ou ces mélanges de protéines d'enveloppe obtenus grâce au vecteur de la présente invention et qui apparaissent essentielles à la formation du virus, ainsi que la possibilité de les produire et de les altérer, fournissent un moyen thérapeutique nouveau.

Le premier élément d'application est le vecteur de co-expression des domaines membranaires qui est décrit ici. Mis au point pour une expression dans la bactérie, sa mise en oeuvre est très aisée et permet de tester rapidement et en grand nombre des composés permettant de moduler la formation des formes homo- et/ou hétéro-oligomériques qui sont identifiées ici. Ce système est d'une grande utilité pour les compagnies qui cherchent à mettre au point des agents chimiques contre le VHC.

Par exemple, ces associations de protéines, ou homo- et/ou hétéro-oligomères, mutées ou non, d'enveloppe de virus obtenues grâce au vecteur de la présente invention peuvent être utilisées pour la fabrication d'anticorps monoclonaux spécifiques desdites associations. Par exemple aussi, en introduisant des mutations dans l'une seulement ou dans plusieurs des protéines co-exprimées ces associations de protéines sont empêchées ou altérées.

La présente invention se rapporte également à l'utilisation
d'une protéine selon la revendication 23 ou d'un hétéro-oligomère ou mélange selon la revendication 24 pour la fabrication d'un médicament, et à l'utilisation
d'une protéine selon la revendication 23 ou d'un hétéro-oligomère ou mélange selon la revendication 24 pour la fabrication d'un médicament destiné au traitement ou à la prophylaxie de l'hépatite C.

Les formes mutées des domaines membranaires obtenues grâce au plasmide de la présente invention ont en effet cette faculté de réduire les forces d'interaction des domaines. Ces peptides constituent donc un nouveau type d'inhibiteur qui peut être utilisé pour entrer en compétition avec les formes non mutées des protéines d'enveloppe, contrarier leur association et ainsi inhiber la formation du virus. Ils peuvent aussi servir de base pour développer des inhibiteurs à partir de leur structure.

La présente invention se rapporte également à l'utilisation des protéines membranaires associées (hétéro-oligomères et/ou homo-oligomères), mutées ou non, par exemple TME1 et TME2 où une seule des deux protéines est mutée ou les deux, dans un procédé de criblage. En effet, il est possible de tester, grâce à la présente invention, des composés chimiques ou biologiques, par exemple des peptides, capable de perturber l'association de ces protéines. Les composés chimiques ou biologiques identifiés comme perturbant l'association des protéines sont des candidats potentiels de molécules qui peuvent servir à la mise au

point de nouveaux principes actifs pour lutter contre les infections virales.

Des vecteurs conformes à la présente invention et convenant aux applications précitées, en particulier lorsqu'elles concernent l'hépatite C, sont par exemple les vecteurs de séquence SEQ IDn°61 et 62 de la liste de séquences annexée.

D'après ce que l'on sait d'autres virus proches de HCV et dont le cycle de formation est mieux documenté, on pense que les protéines d'enveloppe adoptent intermédiairement des états d'association divers. La raison de ceci est que le virus utilise ces différentes formes pour permettre chaque étape de son cycle. Par exemple, la forme E1E2 présente lors de la phase finale de formation du virus n'est cependant pas celle qui permet la fusion/pénétration dans la cellule hôte lors de l'infection. Lors de cette étape, c'est une forme homo-trimérique de E1 qui sera générée et utilisée par le virus. On suppose qu'il existe aussi d'autres formes intermédiaires et c'est ce que les inventeurs ont découvert avec la présente invention.

L'autre fait connu et dont les inventeurs ont étendu le champ avec la présente invention est que les domaines membranaires des deux protéines d'enveloppe sont responsables pour une grande part de ce phénomène d'oligomérisation. Ce qui était ignoré jusqu'aux présents travaux des inventeurs, c'est qu'à la condition de pouvoir les produire, ils permettent de reproduire tous les états d'homo- et d'hétéro-oligomérisation des deux protéines d'enveloppe.

La présente description est basée sur le postulat que si l'on peut perturber la formation de ces formes complexes, on empêche du coup la formation de l'enveloppe et donc celle du virus. C'est une approche thérapeutique nouvelle. Il faut disposer pour cela d'un outil qui permette de tester les composés capables d'interférer dans la formation de ces oligomères. C'est ce que propose la présente invention.

L'idée sur laquelle s'appuie la présente description est que le virus, afin réaliser correctement les phases successives de son cycle de formation puis sa fusion à la membrane de la cellule hôte, doit associer les 2 protéines d'enveloppe sous différents états. Ces associations sont générées et/ou stabilisées en partie par les domaines membranaires C-terminaux des deux protéines. Si ces associations sont empêchées, la formation du virus sera bloquée à différents stades, ce qui limitera ou supprimera sa capacité d'infection. Les composés sélectionnés par l'utilisation de la présente invention devraient permettre d'atteindre ce but.

L'outil créé par les présents inventeurs (vecteur de co-expression) est un système qui permet précisément de produire avec une grande simplicité les différentes formes complexes que ces domaines sont capables de générer. Il utilise la bactérie et ne nécessite pas de système d'expression sophistiqué, à l'inverse de ce qui est nécessaire de mettre en oeuvre si l'on veut produire les protéines complètes ou leur ectodomaines.

La difficulté que pose la production dans la bactérie de protéines membranaires a été résolue en particulier grâce à l'association du dipeptide Asp-Pro et de la protéine soluble. Il n'est pas trivial à la fois de produire de tels domaines hydrophobes et de générer leurs différents états d'association. En effet, s'il est possible de produire de grandes quantités de peptides par voie chimique, le succès d'une telle approche dans les techniques de l'art antérieur était limité dès qu'il s'agissait de peptides hydrophobes et il n'était pas possible de générer leurs formes hétéro-oligomériques correspondantes in vitro.

En effet, les différents hétéro-oligomères, qu'ils soient issus des protéines d'enveloppe complètes ou de leur domaines membranaires C-terminaux ne peuvent pas être formés à partir d'une synthèse chimique ou biosynthèse indépendante de chacun des constituants suivie de leur mélange en solution.

La présente invention a surmonté ces obstacles et fournit une approche *in vivo* de fabrication de ces peptides associés en hétéro-oligomères grâce au plasmide de l'invention. La présente invention limite la formation des complexes à ceux générés par les domaines membranaires C-terminaux TME1 et TME2 dont le repliement dans l'espace est beaucoup plus simple que celui des protéines complètes E1 et E2 et peut donc être réalisé de façon satisfaisante dans un système biologique comme la bactérie.

Les exemples décrits ci-dessous illustrent l'application de la présente invention. Les inventeurs montrent en outre qu'en introduisant des mutations ponctuelles dans l'un des deux domaines, ils peuvent limiter leur interaction. Ceci montre d'une part que la co-production de ces protéines dans la bactérie est correcte et que les états d'association observés correspondent à une propriété intrinsèque d'interaction de ces peptides.

L'intérêt pour l'industrie pharmacologique est évident puisque les inventeurs apportent avec cette invention un moyen robuste et très peu coûteux de tester à haut débit des agents chimiques ou biologiques potentiellement capables d'empêcher la formation du virus. Les entreprises intéressées par la présente invention sont celles de l'industrie pharmaceutique qui cherchent à développer des inhibiteurs de ces virus ou protéines membranaires.

La stratégie mise au point dans la présente description (co-expression, puis mutations ponctuelles) concernant tant l'association de domaines membranaires que sa modulation par mutation est généralisable, non seulement à d'autres pathologies causées par des virus à enveloppe, mais aussi à toute protéine membranaire polytopique qui joue un rôle important dans une pathologie donnée. Un exemple est celui des transporteurs ABC qui jouent un rôle majeur dans le phénomène de chimiorésistance et pour lesquels il devient possible de rechercher des inactivateurs spécifiques par le même type d'approche.

D'autres caractéristiques et avantages de la présente invention apparaîtront encore à la lecture de la description qui suit donnée à titre illustratif en référence aux figures et à la liste de séquences annexées.

### BRÈVE DESCRIPTION DES DESSINS

- Figure 1 : représentation schématique d'une partie de la polyproptéine du VHC et séquences peptidiques des domaines membranaires C-terminaux des protéines d'enveloppe TME1 et TME2.

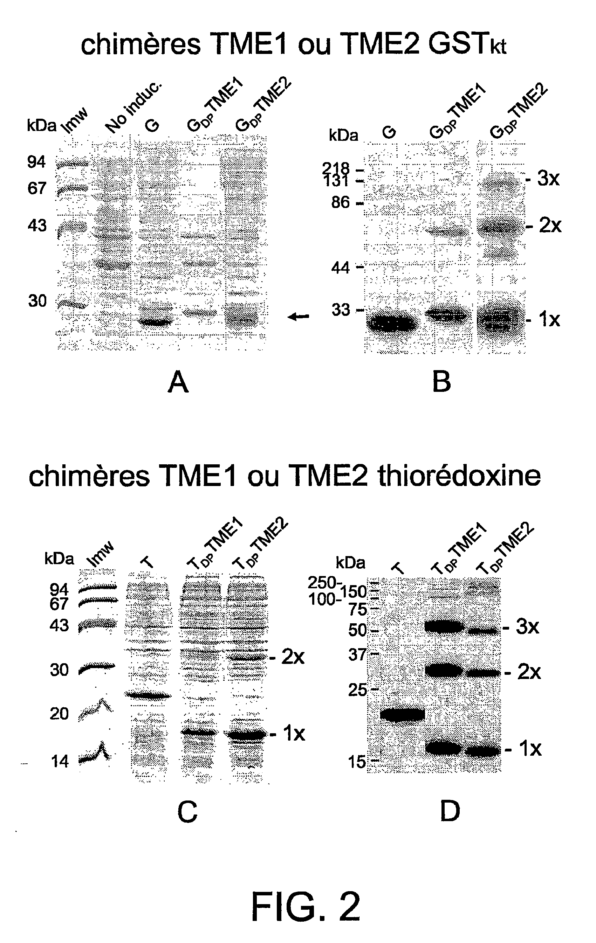

- Les figures 2A et 2C : photographies de deux gels de polyacrylamide 12% mettant en évidence la séparation par migration et coloration au bleu de Coomassie des chimères respectivement GST-DP-TME1 et GST-DP-TME2 (panneau A) et TrX-DP-TME1 et TrX-DP-TME2 (panneau C) obtenues.
- Les figures 2B et 2D : photographies des gels d'électrophorèse, respectivement des figures 2A et 2C, traités par Western blot (immunodétection) pour révéler d'une part (panneau B) spécifiquement les chimères de GST avec un anticorps dirigé contre la GST mettant en évidence des formes dimériques et trimériques des chimères GST-DP-TME1 et GST-DP-TME2, et d'autre part (panneau D) les chimères de TrX avec un anticorps dirigé contre TrX mettant en évidence des formes monomériques, dimériques et trimériques des chimères TrX-DP-TME1 et TrX-DP-TME2.

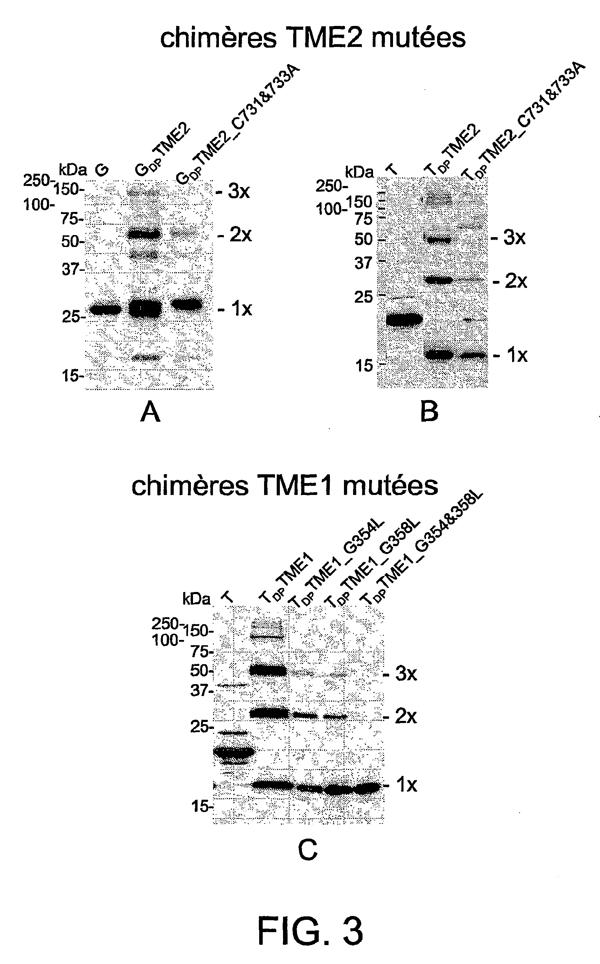

- La figure 3 : Sur le panneau A, photographie de gel d'électrophorèse pour la mise en évidence des chimères GST-DP-TME2 et GST-DP-TME2_C731&C733A et de leurs oligomères. Sur le panneau B, photographie de gel d'électrophorèse pour la mise en évidence des chimères TrX-DP-TME2 et TrX-DP-TME2_C731&C733A et de leurs oligomères. Sur le panneau C, photographie de gel d'électrophorèse pour la mise en évidence des chimères TrX-DP-TME1, TrX-DP-TME1_G354L, TrX-DP-TME1_G358L et TrX-DP-TME1_G354&358L et de leurs oligomères.

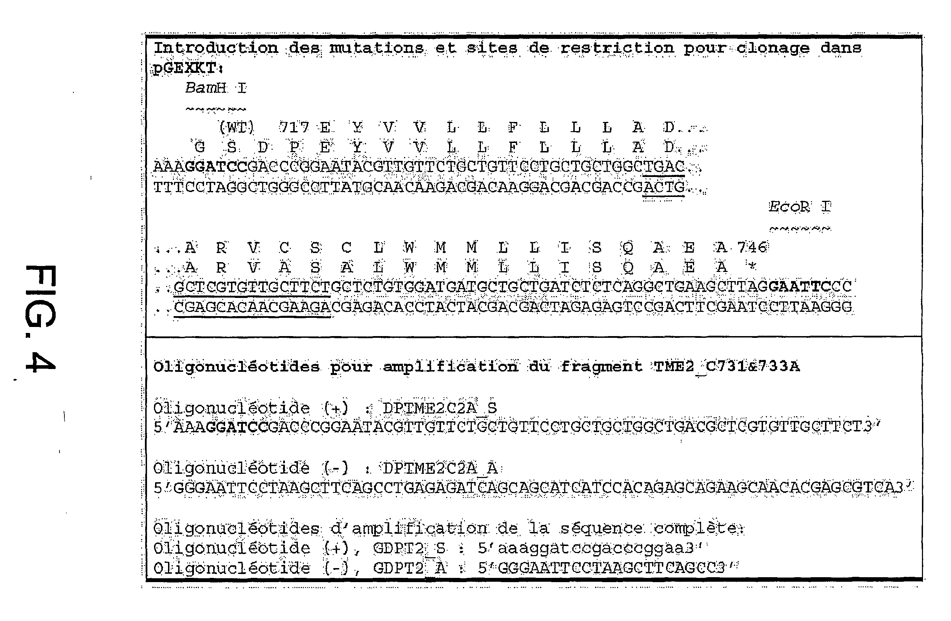

- La figure 4 : représente l'introduction des mutations et sites de restriction pour un clonage dans pGEXKT pou l'obtention d'un vecteur selon l'invention et les oligonucléotides réalisés pour l'amplification du fragment TME2-C731&733A.

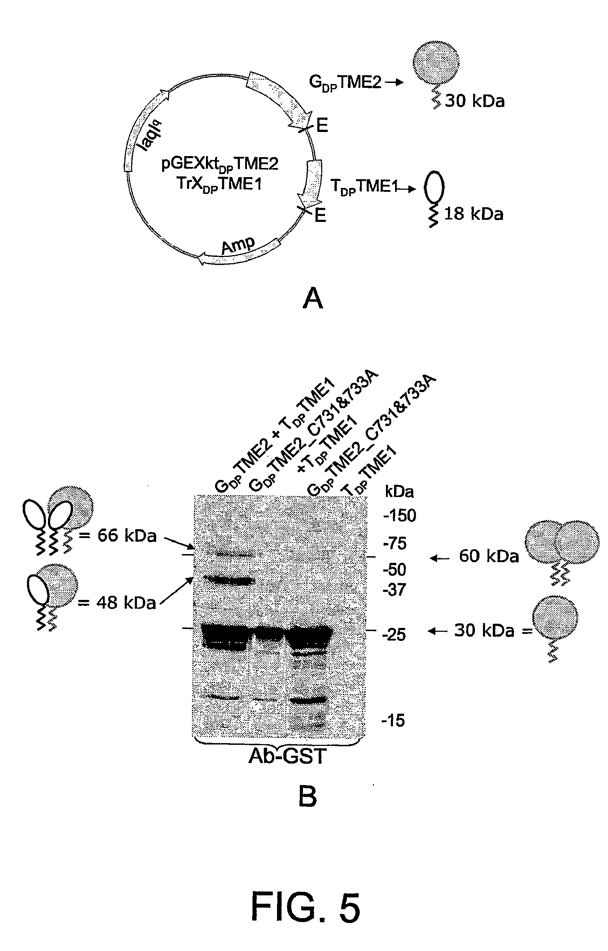

- Figure 5 : Co-expression des chimères GST et thiorédoxine fusionnées respectivement aux domaines membranaires TME2 et TME1 et effet de la mutation des cystéines dans TME2 sur leur homo- et hétéro-oligomérisation. Sur le panneau A : exemple de vecteur de co-expression de chimère GST-DP-TME2 et TrX-DP-TME1 selon la présente invention. Sur le panneau B : photographie de gel western blot mettant en évidence par immunodétection avec un anticorps dirigé contre la GST de l'expression de protéines membranaires dans des bactéries BL21 Gold(DE3)pLysS transformées avec les vecteurs pGEXKT-DP-TME2_C731&C733A, pGEXKT-DP-TME2+TrX-DP-TME1, pGEXKT-DP-TME2_C731&C733A+TrX-DP-TME1 et pET32a-TrX-DP-TME1 de la présente invention.

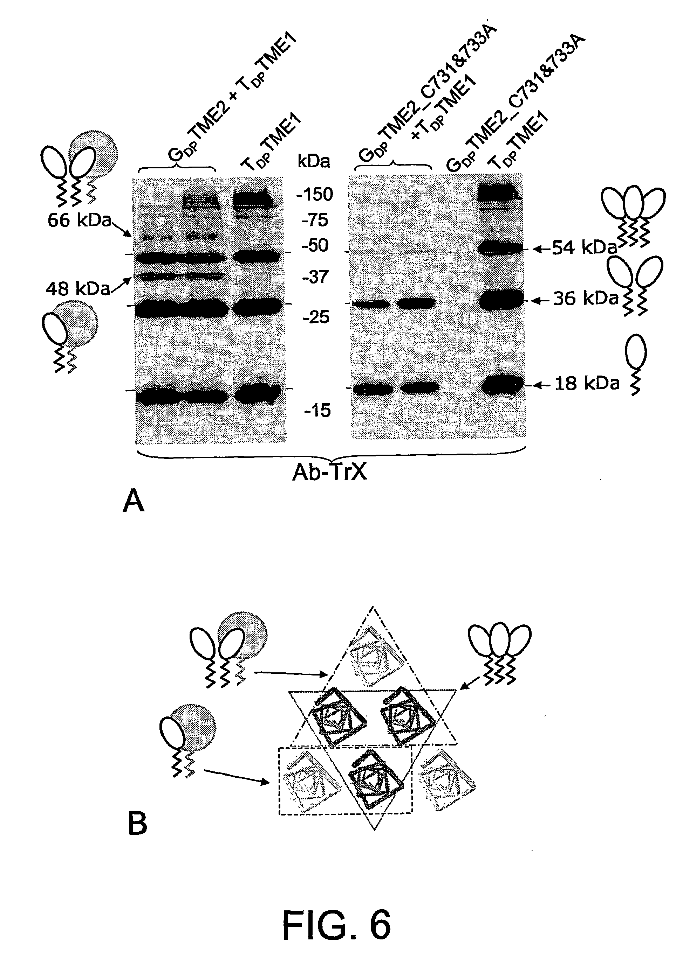

- Figure 6 : Co-expression des chimères GST et thiorédoxine fusionnées respectivement aux domaines membranaires TME2 et TME1 et effet de la mutation des cystéines dans TME2 sur leur homo- et hétéro-oligomérisation. Sur le panneau A : idem panneau B de la Figure 5 avec une immunodétection réalisée avec un anticorps dirigé contre la thiorédoxine. Sur le panneau B : représentation schématique de l'organisation d'oligomères à partir des protéines chimères obtenues à partir de vecteurs selon l'invention.

### Exemples

### MATERIELS

Les oligonucléotides utilisés ont été commandés aux Laboratoires EUROBIO, 07 Avenue de Scandinavie, 91953 Les Ulis Cedex B France, www.eurobio.fr. Les vecteurs ont été préparés avec le kit Qiaprep de QIAGEN, 3 avenue du Canada, LP 809, 91974 Courtaboeuf, Cedex, France, www.qiagen.com. Les séquences d'ADN ont été séquencées avec le kit ABI PRISM (marque déposée) BigDye (marque de commerce) Terminator Cycle d'Applied Biosystems, 25 Avenue de la Baltique, B.P. 96, 91943 Courtaboeuf, Cedex, France, home.appliedbiosystems.com. La souche d'*E*. *col*i BL21 Gold(DE3)pLysS et le système de mutagenèse QuickChange ont été obtenus chez Stratagene, 11011 North Torrey Pines Road, La Jolla, CA 92037, USA, www.stratagene.com. Les enzymes de restriction et modification d'ADN ont été obtenues chez New England Biolabs, 73 Knowl Piece Wilbury Way, Hitchin, Hertfordshire, SG4 OTY, England, UK, www.neb.com/neb. L'appareil d'électrophorèse et transfert de protéines est un miniprotean 3, le scanner GS700 couplé au logiciel Molecular Analyst, et les marqueurs de masse moléculaire « Precision Protein standards » et «Kaleidoscope prestained standards » ont été obtenus dans les Laboratoires Bio-Rad, Division BIO-RECHERCHE, 3 Boulevard Raymond Poincaré, 92430 Marnes la coquette, France, www.bio-rad.com. Le plasmide pET32a+ a été obtenu chez Novagen Inc, 601 Science Drive Madison, WI 53711 USA, www.novagen.com. Le plasmide pGEXKT décrit dans le document [18] a été demandé au Prof. Dixon, Department of Biological Chemistry, University of Michigan Medical School, Ann Arbor 48109-0606, USA. L'anticorps anti-GST GST(Z-5):sc-459 a été obtenu chez Santa Cruz Biotechnology, Inc. 2161 Delaware Avenue, Santa Cruz, California 95060 USA. L'anticorps antithiorédoxine Anti-Thio (#R920-25) et le vecteur pCRtopo2.1 (marque déposée) ont été obtenus chez Invitrogen, SARL BP 96, Cergy Pontoise 95613.0 France. Le kit de chimioluminescence ECL et les marqueurs de masse LMW ont été obtenus chez Amersham Biosciences AB SE-751 84 Uppsala, Sweden. L'anticorps de chèvre anti-souris conjugué à la peroxidase (#M32107) a été obtenu chez TEBU-bio SA, 39, Rue de Houdan, 78612 Le Perray en Yvelines Cedex France. Les autres produits ont été obtenus chez Sigma, L'Isle d'Abeau Chesnes- B.P. 701, 38297 Saint-Quentin Fallavier, France, www.sigma-aldrich.com.

### ABRÉVIATIONS

*E. coli : Escherichia coli.* DP : dipeptide aspartate-proline (Asp-Pro). GST : glutathion S-transférase. VHC: virus de l'hépatite C. TME1 et TME2 : domaines membranaires des glycoprotéines d'enveloppe E1 et E2 du VHC. PCR : « polymerase chain reaction ». LB (10 g tryptone, 5 g yeast extract, 5 g NaCl, q.s.p. 1 litre H₂O). Amp : Ampicilline. Kan : kanamycine. DO : densité optique. SL : solution de lyse (Tris-Cl 50 mM, pH 8,0, EDTA 2,5 mM, SDS 2%, Urée 4M, β-mercaptoéthanol 0,7 M). IPTG : isopropyle-1-thio-β-D-galactoside. aa : acides aminés.

Sur les figures : « ed » : ectodomaine ; « TM » : « domaine transmembranaire » ; « C » : capside ; « E1 » et « E2 » : protéines d'enveloppe E1 et E2 du virus de l'hépatite C ; « P7 » protéine P7 du virus de l'hépatite C, « Lmw » : low molecular weight markers ; « G » : GST, « T » : TrX ; « No induc. » : pas d'induction ; « Ab-TrX » : anticorps dirigé contre TrX ; et « Ab-GST » : anticorps dirigé contre GST.

Les exemples suivants ont été réalisés pour les domaines membranaires TME1 et TME2 des protéines E1 et E2 d'enveloppe du virus de l'hépatite C.

### Exemple 1 : Expression séparée des chimères GST-DP-TME1 et GST-DP-TME2

Comme indiqué sur la figure 1, les domaines membranaires des protéines d'enveloppe du virus de l'hépatite C, TME1 et TME2, correspondent respectivement aux segments 347-383 (SEQ IDn°2) et 717-746 (SEQ IDn°16) de la polyprotéine codée par l'ARN viral. Il existe plusieurs séquences d'ARN du VHC qui produisent un phénotype infectieux. Celles qui ont été utilisées pour exprimer TME1 et TME2 ont pour n° d'accès respectivement #D00831 et #M67463 dans la banque publique de séquences du Laboratoire Européen de Biologie Moléculaire (« European Molecular Biology Laboratory ») (EMBL).

Les ADN codant pour TME1 et TME2 utilisés dans cet exemple ont une séquence nucléotidique correspondante respectivement à SEQ IDn°1 et SEQ IDn°15.

Ces ADNs ont été synthétisés *de novo* à partir des oligonucléotides appropriés. Les codons ont été choisis suivant leur plus grande fréquence d'utilisation dans la bactérie, telle qu'elle a été quantifiée par Sharp et col. [26]. Chaque ADN synthétique a été généré en utilisant un jeu de deux oligonucléotides longs et chevauchants, OL11(SEQ IDn°76) et OL12 (SEQ IDn°77) pour TME1 et OL21(SEQ IDn°79) et OL22 (SEQ IDn°80) pour TME2.

Ces ADNs ont été ensuite amplifiés par la méthode de réaction de polymérisation en chaîne (« polymérase chain reaction ») dite de "PCR" [27] par hybridation avec deux oligonucléotides externes, OL17 (SEQ IDn°78) et OL16 (SEQ IDn°39) pour TME1 et OL27 (SEQ IDn°81) et OL26 (SEQ IDn°40) pour TME2, permettant ultérieurement un sous-clonage dans le plasmide pGEXKT.

Les ADN amplifiés ont été clonés dans un plasmide bactérien pCRtopo2.1 (marque de commerce) et séquencés. Ils ont été excisés puis sous-clonés dans le vecteur pGEXKT (SEQ IDn°23) suivant le protocole décrit dans les documents [17, 18], via les sites *Bam*H I et EcoR I insérés initialement en 5' et 3' des fragments de PCR.

Ces domaines membranaires sont produits en fusion C-terminale avec la GST en intégrant entre chaque domaine et chaque protéine soluble un site de clivage chimique, DP, qui permet de réduire la toxicité intrinsèque de la protéine membranaire hydrophobe.

La version de la GST déjà présente dans le plasmide pGEXKT intègre à la fin de sa séquence une série de 5 résidus glycine qui confère une certaine flexibilité entre la GST et la protéine attachée à cette extrémité.

Les vecteurs pGEXKT-DP-TME1 (SEQ IDn°26) et pGEXKT-DP-TME2 (SEQ IDn°29) ainsi générés ont été incorporés dans des bactéries BL21 Gold(DE3)pLysS (B F⁻ *dcm ompT hsdS*(r_{B}⁻ m_{B}⁻) *gal* λ (DE3) [pLysS Cam^{r}]) pour permettre l'expression des chimères GST-DP-TME1 et GST-DP-TME2 dont les caractéristiques sont rappelées dans le tableau 1 ci-dessous.

Dans ce tableau, les acides aminés sont indiqués avec le code à une lettre. La numérotation des séquences est effectuée par rapport aux protéines d'origine, GST et polyprotéine virale. Celle qui fait référence aux domaines membranaires est indiquée en italiques.

L'expression des chimères est induite par l'isopropyl-1-thio-β-D-galactoside (IPTG). Les bactéries hôtes ont été modifiées pour contenir dans le génome une copie du gène codant pour l'ARN polymérase du phage T7, placé sous contrôle d'un promoteur lacUV5 inductible par l'isopropyle-1-thio-β-D-galactoside (IPTG). Dans ce cas, les bactéries sont cultivées à leur température optimum de 37°C ou moins si nécessaire. L'expression est induite par addition d'IPTG dans la culture.

**Tableau 1 : chimères GST-DP-TME1 et GST-DP-TME2 obtenues**

| **Plasmide - vecteur** | **Chimère, abbréviation, SEQ** | **Construction** | **Taille (résidus aa)** | **Masse (Da)** |
|---|---|---|---|---|
| pGEXKT | GST SEQ IDn°25 | - | 239 | 27469 |
| pGEXKT-DP-*TME1* | GST-DP-*TME1*, GST-DP-TME1 SEQ IDn°28 | ₁M-S₂₃₃-DP-*₃₄₇M-A₃₈₃* | 271 | 30718 |
| pGEXKT-DP-*TME2* | GST-DP-*TME2*, GST-DP-TME2 SEQ IDn°30 | ₁M-S₂₃₃-DP-*₇₁₇E-A₇₄₆* | 265 | 30403 |

Les protéines produites sont ensuite séparées par migration sur gel de polyacrylamide 12% effectuée en conditions de type «Laemmli» de la manière décrite dans le document [19] et détectées par coloration au bleu de Coomassie. Dans ces conditions, les résultats de la figure 2A annexée permettent d'observer parmi les protéines bactériennes les chimères GST-DP-TME1 et GST-DP-TME2 qui sont surproduites et migrent à la taille attendue (-30 kDa).

De façon inattendue, lorsque les gels d'électrophorèse sont traités par Western blot pour révéler spécifiquement les chimères de GST avec un anticorps dirigé contre la GST comme cela est représenté sur la figure 2B, des formes dimériques et trimériques des chimères GST-DP-TME1 et GST-DP-TME2 apparaissent.

La GST non fusionnée aux domaines membranaires reste monomérique, ce qui laisse supposer que l'oligomérisation est dûe à la présence des régions hydrophobes. De même, les interactions qui contrôlent l'association des domaines membranaires sont suffisamment fortes pour résister au moins partiellement aux conditions très dénaturantes dans lesquelles les protéines sont soumises lors de la préparation des échantillons et leur migration en SDSPAGE (2% SDS, 4 M urée, 0,7 M de β-mercaptoéthanol, migration, (voir légende de la figure 2).

Ces premiers résultats révèlent les propriétés d'oligomérisation des domaines membranaires TME1 et TME2 mais sans permettre toutefois de s'en convaincre complètement. En effet la GST en solution est un dimère et cela peut favoriser le rapprochement des domaines. De même, TME2 contient les cystéines C731 et C733 pour lesquelles l'environnement hydrophobe favorise l'oxydation, qui peut favoriser à son tour l'agrégation des domaines. Pour évaluer ces possibilités, les inventeurs ont donc transposé les constructions dans un nouveau plasmide pour remplacer la GST par la thiorédoxine dans les chimères.

### Exemple 2 : Expression des chimères thiorédoxine-DP-TME1 et thiorédoxine-DP-TME2

Le remplacement de la GST par la thiorédoxine dans les chimères a été effectué en utilisant le plasmide d'expression pET32a+ (SEQ IDn°35). Dans ce dernier la séquence qui code pour la thiorédoxine intègre en phase codante une courte région 3' ajoutée pour la détection et la purification de la protéine. Ces éléments ne sont pas utilisés ici et l'insertion des domaines membranaires a été effectuée juste après la région codant pour la thiorédoxine, en amont de cette partie additionnelle.

Les fragments (régions codantes) à insérer ont été générés par PCR en utilisant comme matrice les vecteurs pGEXKT-DP-TME1 (SEQ IDn°26) et pGEXKT-DP-TME2 (SEQ IDn°29), et comme amorces les jeux d'oligonucléotides suivants :
TME1 et TME2, oligonucléotide amont OL18(+):
   5'gt**gatatc**tgatctgtctggtggtggt (SEQ IDn°38) TME1, oligonucléotide aval OL16(-):
   5'gaattcctaagcttcagcctgag (SEQ IDn°39) TME2, oligonucléotide aval OL26(-):
   5'gaattcttaagcttcagcctgagagatcag (SEQ IDn°40)

L'oligonucléotide amont OL18 intègre un site *Eco*R V et s'hybride avec le segment 915-932 de pGEXKT, correspondant à la région terminale du gène codant pour la GST. L'oligonucléotide aval OL16 ou OL26 est le même que celui utilisé pour le clonage dans pGEXKT. Utilisant les matrices pGEXKT-DP-TME1 et pGEXKT-DP-TME2, chaque fragment amplifié intègre la séquence SDLSGGGGGLVPRGS (SEQ IDn°63), présente en C-ter de la GST codée par pGEXKT, suivie du site DP, suivi du domaine membranaire.

L'insertion dans le plasmide pET32a se fait par les sites *Msc* I/*Eco*R V en 5' et EcoR I en 3'. Il permet d'insérer la séquence amplifiée à la fin de la séquence de la thiorédoxine et en phase avec le gène codant pour celle-ci.

Les vecteurs issus de ces constructions sont pET32a-TrX-DP-TME1 (SEQ IDn°41) et pET32a-TrX-DP-TME2 (SEQ IDn°53). Les protéines produites à partir de ces vecteurs sont T_{DP}TME1 (SEQ IDn°43) et T_{DP}TME2 (SEQ IDn°55). Leurs caractéristiques sont résumées dans le tableau 2 ci-dessous.

Dans ce tableau, les acides aminés sont indiqués avec le code à une lettre. La numérotation des séquences est effectuée par rapport aux protéines d'origine, GST et polyprotéine virale. Celle qui fait référence aux domaines membranaires est indiquée en italiques.

**Tableau 2 : caractéristiques des protéines chimères à partir des vecteurs construits**

| **Plasmide - vecteur** | **Chimère, abbréviation** | **construction** | **Taille, résidus aa** | **Masse, Da** |
|---|---|---|---|---|
| pET32a | thiorédoxine, TrX (SEQ IDn°37) | ₁M-C₁₈₉ | 189 | 20397 |
| pET32a-DP-*TME1* | TrX-DP-*TME1*, T_{DP}*TME1* (SEQ IDn°43) | ₁M-S₁₁₅-DP-*T1* | 171 | 17796 |
| pET32a-DP-*TME2* | TrX-DP-*TME2*, T_{DP}*TME2* (SEQ IDn°55) | ₁M-S₁₁₅-DP-*T2* | 165 | 17481 |

Les chimères thiorédoxine-SDLSGGGGGLVPRGS-DP-(TME1 ou TME2) (SEQ IDn°43 et SEQ IDn°55) sont plus courtes que la protéine codée dans le vecteur d'origine car l'insertion est effectuée immédiatement après la thiorédoxine, ce qui élimine la partie ajoutée en aval de la thiorédoxine, sans intérêt ici.

L'expression des chimères de thiorédoxine et la détection des protéines produites ont été réalisées comme décrit dans l'exemple 1.

Les protéines produites ont été séparées par SDS-PAGE 14% puis détectées par coloration au bleu de Coomassie ou par immunodétection.

La figure 2C permet d'observer parmi les protéines bactériennes les chimères TrX-DP-TME1 et TrX-DP-TME2 qui sont cette fois nettement surproduites et qui migrent à la taille attendue (-18 kDa).

Ce résultat confirme que le vecteur d'expression fonctionne avec une autre protéine que la GST.

Le niveau de surexpression des 2 protéines est tel que leur forme dimérique (au niveau du repère 2x dans la Figure 2C) est visible sur le gel coloré au bleu de Coomassie.

L'immunodétection réalisée après Western blot représenté sur la figure 2D permet de visualiser les monomères et dimères mais aussi très nettement les formes trimériques.

La thiorédoxine ne formant pas de dimère, ces résultats montrent clairement que l'oligomérisation est due à la présence des domaines membranaires.

Sur le plan fondamental, ces résultats sont la première démonstration expérimentale de l'existence de formes oligomériques de TME1 et TME2.

### Exemple 3 : Expression des formes mutées dans les domaines membranaires des chimères GST et thiorédoxine

### MUTATION C731A ET C733A DANS TME2

Comme cela a été mentionné plus haut, la mutation des résidus cystéines de TME2 a été réalisée pour tester leur influence sur l'oligomérisation des chimères GST-DP-TME2.

La mutagenèse a été réalisée en créant un nouveau brin d'ADN à partir d'oligonucléotides longs comme décrit sur la figure 4 annexée. Les fragments générés ont été tout d'abords clonés dans le plasmide pGEXKT pour créer le vecteur pGEXKT-DP-TME2_C731&C733A (SEQ IDn°32) permettant l'expression de la chimère GST-DP-TME2-C731&C733A (SEQ IDn°34), puis transférés dans le plasmide pET32a avec la stratégie décrite dans l'exemple précédent pour créer le vecteur pET32a-DP-TME2_C731&C733A (SEQ IDn°56) et générer la chimère T_{DP}TME2-C731&C733A (SEQ IDn°58).

La séquence d'ADN codant pour le domaine TME2 doublement muté C731A et C733A (SEQ IDn°22) est synthétisée *de novo* par PCR en utilisant le jeu d'oligonucléotides longs DPTME2C2A_S (SEQ IDn°18) et DPTME2C2A_A (SEQ IDn°19), qui s'hybrident par leurs extrémités 3' (soulignées) tandis que les oligonucléotides externes GDPT2_S (SEQ IDn°20) et GDPT2_A (SEQ IDn°20) sont utilisés pour faciliter l'amplification après hybridation. L'ADN généré est coupé par *Bam*H I et EcoR I puis inséré dans le plasmide pGEXKT (SEQ IDn°23). La séquence du vecteur pGEXKT-DP-TME2_C731&C733A résultant (SEQ IDn°32) est vérifiée par séquençage.

Les vecteurs résultant des constructions furent introduits dans les bactéries BL21 Gold(DE3)pLysS et l'expression fut réalisée comme précédemment.

Les protéines exprimées furent révélées par coloration au bleu de Coomassie (non représenté) et Western blot représenté sur la figure 3 annexée.

Sur le panneau A de la figure 3, il apparaît que la chimère GST-DP-TME2-C731&C733A est aussi bien produite que sa forme non mutée. La mutation diminue cependant de façon très nette le niveau de dimère et réduit à l'état de traces celui du trimère. Le même résultat est obtenu lorsque la thiorédoxine remplace la GST, comme c'est illustré sur le panneau B de la figure 3.

Ces résultats montrent tout d'abord que la formation des oligomères impliquant TME2 n'est pas irréversible puisqu'une double mutation dans le domaine en réduit la quantité formée. Etant donné que des traces d'oligomères sont encore visibles sur gel il est probable que les domaines mutés forment encore ces oligomères, cependant la double mutation réduit suffisamment leur force d'interaction pour les empêcher de se maintenir dans les conditions dénaturantes imposées par le SDS-PAGE.

### MUTATIONS G354L, G358L ET G354&G358L DANS TME1

De la même façon, les inventeurs ont testé l'effet de mutations sur l'oligomérisation de TME1.

Le choix des résidus à muter a été effectué en se basant sur les travaux d'Op De Beeck et col. exposés dans le document [11] montrant que l'addition de résidus alanine dans la région 354-358 diminue la formation de l'hétéro-dimère E1-E2. Cette région contient un motif « glycine » GXXXG. Comme cela a été décrit par Engelman dans le document [20], un tel motif est critique pour l'association de domaines membranaires. En effet, un domaine membranaire est généralement organisé en hélice-α dans laquelle les 2 résidus glycine du motif, distants de 4 résidus, se retrouvent localisés dans l'espace l'un en dessous de l'autre. La chaîne latérale des résidus glycine se limitant à un hydrogène, l'espace vacant qui résulte de l'empilement des deux glycines peut être comblé par des résidus hydrophobes volumineux, comme la leucine par exemple. Il en résulte un encastrement qui renforce l'interaction entre les domaines. Suivant ce principe, les inventeurs ont donc remplacé les résidus glycine 354 et 358, indépendamment et ensemble, par une leucine pour estimer leur importance dans ce phénomène.

Les mutations G354L, G358L et G354&G358L ont été générées par le système QuickChange (marque de commerce) de Stratagene en utilisant comme matrice le vecteur pET32A-TrX-DP-TME1. Les mutations n'ont pas été introduites dans les chimères GST.

Les jeux d'oligonucléotides utilisés pour effectuer les mutagenèses G354L et G358L sont décrits ci-après :
T1G354L (SEQ IDn°3) :
   5'GTAAGCGATACCAGCCAGAACCAGCCAGTGAGCACCAGCGAT3'
T1G354Lc (SEQ IDn°4) :
   5'ATCGCTGGTGCTCACTGGCTGGTTCTGGCTGGTATCGCTTAC3'
T1G358L (SEQ IDn°5) :
   5'CAACCATAGAGAAGTAAGCGATCAGAGCCAGAACACCCCAGTG3'
T1G358Lc (SEQ IDn°6) :
   5'CACTGGGGTGTTCTGGCTCTGATCGCTTACTTCTCTATGGTTG3'

Les vecteurs générés sont pET32A-TrX-DP-TME1_G354L (SEQ IDn°44) et pET32A-TrX-DP-TME1_G358L (SEQ IDn°47). Ils permettent l'expression des chimères TDPTME1-G354L (SEQ IDn°46) et TDPTME1-G358L (SEQ IDn°49).

La double mutagenèse a été générée en utilisant comme matrice le vecteur pET32A-TrX-DP-TME1_G354L et les oligonucléotides suivants (les bases soulignées correspondent au codon déjà muté):
T1G2L (SEQ IDn°7) :
   5'CAACCATAGAGAAGTAAGCGATCAGAGCCAGAACCAGCCAGTG3'
T1G2Lc (SEQ IDn°8) :
   5'CACTGGCTGGTTCTGGCTCTGATCGCTTACTTCTCTATGGTTG3'

Le vecteur crée est pET32A-TrX-DP-TME1_G354&G358L (SEQ IDn°50), générant la chimère TDPTME1-G354&G358L (SEQ IDn°52).

Comme précédemment, les vecteurs résultant de ces constructions furent introduits dans les bactéries BL21 Gold(DE3)pLysS et l'expression des chimères réalisée.

Les protéines exprimées furent révélées par Western blot. Les résultats sont représentés sur la figure 3C. Ces résultats montrent que, par comparaison au domaine non modifié, le remplacement des résidus glycine 354 ou 358 par des leucines réduit nettement la quantité de trimère et un peu moins celle de dimère. Le remplacement simultané des deux glycines conduit par contre à une disparition totale des oligomères.

Les résidus glycine sont donc importants pour favoriser l'oligomérisation de TME1, et cette interaction est principalement le fait du motif qu'ils constituent puisqu'il est nécessaire de les supprimer ensemble pour obtenir un effet complet.

Ces résultats complètent les observations d'Op De Beeck et col. décrites dans le document [11] montrant que l'addition de résidus alanine dans la région 354-358 qui inclut les deux résidus glycine (et non pas le remplacement comme c'est le cas ici) diminue la formation de l'hétéro-dimère E1-E2. L'expression de E1-E2 décrite par ces auteurs avait été effectuée dans un système vaccine, assez proche des conditions natives d'expression de ces protéines complètes (il n'existe pas de système d'expression du virus complet, ni de système permettant sa multiplication. Le système vaccine est l'un des rares décrits dans la littérature permettant l'expression fonctionnelle des protéines d'enveloppe E1 et E2 complètes).

Il est donc particulièrement intéressant de relever le fait que, au moins pour les domaines membranaires de ces protéines, que le vecteur d'expression bactérien de la présente invention permet de reproduire des effets similaires.

Sur ce plan, le vecteur de la présente invention apparaît donc aussi fiable que le système vaccine, et beaucoup plus simple à mettre en oeuvre.

### Exemple 4 : Co-Expression des chimères GST et thiorédoxine

Comme cela a déjà été mentionné, il n'existe pas de système permettant la genèse du virus et il est donc impossible de suivre les étapes qui conduisent à sa formation. Les quelques systèmes permettant de co-exprimer E1 et E2 restent difficiles à mettre en oeuvre [28] et ne permettent pas de produire des quantités importantes de protéines.

Les présents inventeurs ont développé un système de co-expression de ces domaines et il ont montré que ce système, conforme à la présente invention telle qu'elle est revendiquée, permet d'identifier des formes hétéro-oligomériques des chimères telles qu'elles existeraient lors de la formation du virus.

Pour réaliser ce système, la région du vecteur pET32a-TrX-DP-TME1 contenant le gène codant pour la chimère thiorédoxine-DP-TME1 et son promoteur T7 a été tout d'abord amplifiée par PCR (comme décrit dans l'exemple 1) en utilisant le jeu d'oligonucléotides suivant :
PET998-*Alw*NI (SEQ IDn°59) :
   5'TTCAGTGGCTGTGCATGCAAGGAGATGGCG-3'
AST1-*Alw*NI (SEQ IDn°60) :
   5'TTCAGCCACTGCTAAGCGTCAACACCAGCG3'

La cassette d'ADN provenant du vecteur d'expression pET32a-TrX-DP-TME1 (SEQ IDn°41), dont la construction est décrite dans l'exemple 2, contient un promoteur T7 suivit d'un séquence ouverte de lecture contenant en phase le gène codant pour la thiorédoxine suivit d'un fragment d'ADN codant pour le dipeptide Asp-Pro, suivit de la région Met347-Ala383 correspondant au domaine membranaire C-terminal de E1. La protéine chimère correspondante est T_{DP}TME1 (SEQ IDn°43, voir exemple 2).

L'oligonucléotide PET998-*Alw*NI s'hybride avec le segment 980-998 de pET32a, en amont du promoteur T7 de la thiorédoxine. L'oligonucléotide AST1-*Alw*NI s'hybride avec la région 3' du gène codant pour TME1.

Le fragment de PCR a été généré en utilisant ces oligonucléotides et le vecteur pET32a-TrX-DP-TME1 (SEQ IDn°41) comme matrice. Il a été ensuite sous-cloné dans le plasmide pCRtopo2.1 (marque déposée) et séquencé. Il a ensuite été extrait du plasmide pCRtopo2.1 par restriction avec l'enzyme EcoR I dont les 2 sites présents sur le plasmide sont situés quelques bases avant et après le fragment sous-cloné. Le fragment ainsi extrait a été introduit dans le site unique EcoR I présent dans pGEXKT-DP-TME2 et pGEXKT-DP-TME2_C731&C733A, localisé en aval du gène codant pour TME2 (cf. panneau A de la Figure 5 pour la position du site EcoR I).

Les vecteurs ainsi crées sont pGEXKT-DP-TME2+TrX-DP-TME1 (SEQ IDn°61) et pGEXKT-DP-TME2_C731&C733A+TrX-DP-TME1 (SEQ IDn°62). Un exemple de vecteur est illustré dans le panneau A de la figure 5 annexée. Sur cette figure, le site EcoR I ayant servi à insérer la cassette codant pour la chimère thiorédoxine-DP-TME1 est indiqué par la lettre E. Les protéines chimères obtenues sont représentées schématiquement à droite du vecteur, en fonction de leur taille.

Les constructions réciproques produisant les vecteurs pGEXKT-DP-TME1+TrX-DP-TME2 ne sont pas décrites ici. Elles conduisent au même type de résultats que ceux décrits ci-après.

Les clones positifs ont été mis en culture et induits comme décrit dans l'exemple 1. Si les chimères sont exprimées et s'associent, on verra apparaître différents hétéro-oligomères dont le tableau 4 suivant résume les différents cas possibles et les masses moléculaires résultantes.

**Tableau 4 : Possibilités d'association des chimères GST-DP-TME2 ou GST-DP-TME2-C731&733A avec T_{DP}TME1 avec les masses moléculaires correspondantes**

| Masse Moléculaire KDa | | GST-DP-TME2 ou GST-DP-TME2-C731&733^{a} | | | |
|---|---|---|---|---|---|
| | | non exprimé | monomère | dimère | trimère |
| TrX-DP-TME1 | **non exprimé** | **-** | **30** | **60** | **90** |
| | **monomère** | **18** | **48** | **78** | **108** |
| | **dimère** | **36** | **66** | **96** | **126** |
| | **trimère** | **54** | **84** | **114** | **144** |

Comme précédemment, les vecteurs résultant de ces constructions furent introduits dans les bactéries BL21 Gold(DE3)pLysS et l'expression des chimères réalisée.

Après expression, les bactéries ont été traitées comme décrit dans l'exemple 1. Les différentes chimères ainsi que leurs formes oligomériques ont été révélées par Western blot et immunodétection comme décrit dans les exemples ci-dessus en utilisant les anticorps anti-GST (figure 5, panneau B) et anti-thiorédoxine (figure 6, panneau A).

Les essais de co-expression ont été doublés pour montrer les nouvelles espèces formées. Pour aider la lecture des Figure 5 et 6, les protéines chimères GST-DP-TME2 et TrX-DP-TME1 (mutées ou non mutées) ont été symbolisées par des icônes, utilisées pour montrer à quoi peuvent correspondre les formes homo- et hétéro-oligomériques observées. Les marqueurs de masse moléculaire utilisés sont les « Precision Protein standards ».

Comme illustré sur le panneau B de la figure 5, la révélation des chimères de GST produites permet tout d'abord de détecter la forme monomérique GST-DP-TME2 migrant à 30 kDa. Elle est visible dans tous les puits sauf dans celui contenant seulement la chimère TrX-DP-TME1. Dans le premier puits de ce panneau, une bande plus lourde que la forme monomérique est ensuite visible. Sa migration la rend compatible avec une masse de 48 kDa correspondant à l'hétéro-dimère {GST-DP-TME2₁+ TrX-DP-TME1₁}.

Comme on peut le voir dans le deuxième puits, cette espèce hétéro-dimérique est très défavorisée lorsque la mutation C731&C733A est présente dans TME2. Enfin, au dessus de cette forme apparaît une bande dont la migration à ce niveau suggère qu'elle pourrait correspondre à l'hétéro-trimère {GST-DP-TME2 + TrX-DP-TME1₂}. Malgré le peu de résolution dans cette région, la position sur le gel de cet hétéro-trimère de 66 kDa reste distincte de celle de l'homo-dimère GST-DP-TME2₂ de 60 kDa dont des traces sont visibles dans le troisième puits où seul le mutant GST-DP-TME2-C731&C733A est exprimé. Cette forme hétéro-trimérique est absente lorsque TrX-DP-TME1 est co-exprimé avec le mutant GST-DP-TME2-C731&C733A tel que l'on peut le voir dans le deuxième puits.

Lorsque l'immunodétection est effectuée avec un anticorps anti-thiorédoxine, les résultats présentés dans le panneau A de la figure 6 montrent que la co-expression de T-DP-TME1 avec GST-DP-TME2 conduit tout d'abord à la formation des formes monomérique, dimérique et trimérique de TrX-DP-TME1, telles qu'on peut les observer dans les puits 1, 2 et 3. Apparaissent ensuite deux nouvelles formes qui migrent de part et d'autre de l'homo-trimère T-DP-TME1₃. Les masses moléculaires de ces protéines sont compatibles à celles de l'hétéro-dimère {GST-DP-TME2₁+T-DP-TME1₁} et de l'hétéro-trimère {GST-DP-TME2₁+T-DP-TME1₂}, qui sont respectivement de 48 et 66 kDa.

Ce résultat confirme donc celui obtenu avec l'anticorps anti-GST présenté dans le premier puits du panneau B de la figure 5. Lorsque les essais de co-expression sont réalisés avec la chimère T-DP-TME1 et le mutant GST-DP-TME2-C731&C733A, comme c'est présenté dans les quatrièmes et cinquièmes puits du panneau A de la figure 6, de façon remarquable on peut constater que les formes hétéro-oligomériques ne sont plus formées et, plus inattendu, que la forme trimérique T-DP-TME1₃ est spécifiquement peu abondante.

Ces résultats montrent donc très clairement que la présence de la double mutation C731&C733A dans TME2 fragilise - au point de les faire disparaître - les formes hétéro-oligomériques et de façon plus remarquable encore, contribue aussi à diminuer la quantité du trimère TrX-DP-TME1₃.

La première conclusion des expériences exposées dans les exemples ci-dessus est que la co-expression de protéines solubles comme la GST ou la thiorédoxine, fusionnées aux domaines membranaires TME1 et TME2 des protéines d'enveloppe du virus de l'hépatite C conduit à la formation d'espèces hétéro-oligomériques telles que {GST-DP-TME2₁+T_{DP}TME1₁} et {GST-DP-TME2₁+T_{DP}TME1₂}, qui sont suffisamment stables pour résister aux conditions dénaturantes dans lesquelles elles sont soumises lors de la migration électrophorétique. C'est la première démonstration expérimentale de la capacité de ces domaines membranaires à s'associer entre eux lorsqu'ils sont exprimés, indépendamment ou ensemble. Comme ces expériences sont effectuées en absence de l'ectodomaine (« ed » dans la figure 1), la partie extra membranaire des protéines E1 et E2, cela montre la contribution essentielle des domaines membranaires dans ce phénomène d'association.

Ces résultats montrent aussi assez clairement que la force des interactions qui conduisent à la formation des homo-dimères n'est pas équivalente pour TME1 et TME2. C'est particulièrement net avec les expériences de co-expression qui montrent que les espèces dimérique et trimérique de TrX-DP-TME1 sont toujours bien formées malgré la présence des hétéro-oligomères, tandis que dans le cas de GST-DP-TME2, les mêmes espèces ont disparues au profit des hétéro-oligomères.

Cela met en relief des capacités intrinsèquement différentes de TME1 et TME2 à oligomériser, et renseigne sur leur rôle respectif lors de la formation du virus. En effet, les complexes les plus abondants/stables formés lors de la co-expression et encore visibles sur SDS-PAGE sont les homo-oligomères (TrX-DP-TME1)₂ et (TrX-DP-TME1)₃ et les hétéro-oligomères {(GST-DP-TME2)₁+(TrX-DP-TME1)₁} et {(GST-DP-TME2)₁+(TrX-DP-TME1)₂}.

Ces espèces ont résisté aux conditions dénaturantes imposées par le SDS-PAGE et il est donc probable qu'elles forment un complexe d'ordre supérieur dans des conditions plus physiologiques.

L'organisation la plus simple d'un tel complexe regroupant toutes ces espèces observées pourrait correspondre au modèle de condensation représenté schématiquement sur la figure 6, panneau B. La forme ainsi générée est constituée en son centre d'un trimère TrX-DP-TME1₃ qui est entouré aux sommets d'un monomère de GST-DP-TME2. Cette forme pourrait être la plus aboutie en terme d'organisation structurale du virus, celle qui existe juste avant l'étape de fusion. On retrouve une organisation similaire dans le cas du virus de l'encéphalite porté par une tique comme cela est exposé dans les documents [22], [23] et [24].

### Exemple 5 : Validité du système de co-expression des domaines membranaires et utilisation pour découvrir et tester des composés capables d'altérer la stabilité de leurs formes homo-oligomériques et hétéro-oligomériques.

Les résultats décrits ci-dessus montrent que les inventeurs ont inventé un système de co-expression dans la bactérie des domaines membranaires des protéines d'enveloppe du virus de l'hépatite C.

Les formes hétéro-dimériques que les inventeurs ont obtenu correspondent à celles qui ont été précédemment décrites avec les protéines complètes E1 et E2 co-exprimées dans le système vaccine [11], ce qui montre que le vecteur de la présente invention permet de générer cette forme. Il permet aussi de générer la forme hétéro-trimérique {GST-DP-TME2₁+TrX-DP-TME1₂} qui n'a pas été observée auparavant.

Le vecteur de la présente invention apparaît donc comme une excellente alternative pour étudier les interactions créées par les régions membranaires des protéines d'enveloppe.

Partant du fait que l'interaction des protéines d'enveloppe engage les régions membranaires et que les complexes qui en résultent sont essentiels à la formation du virus, il apparaît que ce système permet de tester des composés capables de moduler les interactions mises en oeuvre dans ces complexes serait un atout majeur dans la lutte contre ce virus.

Une fois la mise au point du vecteur de la présente invention réalisée, ce vecteur est extrêmement aisé à mettre en oeuvre, à peu de frais, et permet de tester des composés à une échelle compatible à celle de la chimie combinatoire par exemple.

Indépendamment de cette première approche réalisée par les inventeurs, ils présentent aussi une deuxième approche qui montre qu'il est possible de limiter ou supprimer l'interaction des domaines membranaires dans les complexes qu'ils sont capables de générer en les mutants.

Dans un premier temps ils montrent que la double mutation des résidus glycine 354 et 358 en résidus leucine supprime la formation du trimère de TME1, qui serait d'après le modèle de la figure 5D un des éléments de la forme la plus ultime du complexe. D'autre part, ils montrent que la double mutation des résidus cystéine 731 et 733 empêche la formation des hétéro-oligomères de TME1 et TME2 mais aussi celle du trimère de TME1.

Avec ces mutants les inventeurs fournissent donc deux exemples de molécules potentiellement compétitrices de leur forme sauvage. A ce titre, ces molécules (peptides) constituent d'excellents candidats pour lutter contre le virus en gênant sa formation et peuvent être testés tels quel ou sous forme de produits dérivés dans un but thérapeutique.

### Liste des références

[1] Ciccaglione A.R., Marcantonio C., Costantino A., Equestre M., Geraci A. and Rapicetta M. (2000) Virus Genes 21, 223-226 ;
[2] Sisk W.P., Bradley J.D., Kingsley D., and Patterson T.A. (1992) Gene 112, 157-162 ;
[3] Paulsen I.T., Sliwinski M.K., Nelissen B., Goffeau A., and Saier M.H. Jr. (1998) FEBS Lett 430, 116-125 ;
[4] Decottignies A. and Goffeau A. (1997) Nat Genet 15, 137-145 ;
[5] Arechaga I., Miroux B., Karrasch S., Huijbregts R., de Kruijff B., Runswick M.J. and Walker J.E. (2000) FEBS Lett 482, 215-219 ;
[6] Miroux B. and Walker J.E. (1996) J. Mol. Biol. 260, 289-298 ;
[7] Mayo M.A., and Pringle C.R. (1998) J. Gen Virol. 79 (Pt4), 649-657 ;
[8] Rosenberg, S. (2001) J Mol Biol 313, 451-464.
[9] Choo, Q. L., Kuo, G., Weiner, A. J., Overby, L. R., Bradley, D. W., and Houghton, M. (1989) Science 244, 359-362.
[10] Op De Beeck, A., Cocquerel, L., and Dubuisson, J. (2001) J Gen Virol 82, 2589-2595.
[11] Op De Beeck, A., Montserret, R., Duvet, S., Cocquerel, L., Cacan, R., Barberot, B., Le Maire, M., Penin, F., and Dubuisson, J. (2000) J Biol Chem 275, 31428-31437.
[12] Ciccaglione, A. R., Marcantonio, C., Costantino, A., Equestre, M., Geraci, A., and Rapicetta, M. (1998) Virology 250, 1-8.
[13] Ciccaglione, A. R., Marcantonio, C., Costantino, A., Equestre, M., Geraci, A., and Rapicetta, M. (2000) Virus Genes 21, 223-226.
[14] Ciccaglione, A. R., Marcantonio, C., Equestre, M., Jones, I. M., and Rapicetta, M. (1998) Virus Res 55, 157-165.
[15] Sisk, W. P., Bradley, J. D., Kingsley, D., and Patterson, T. A. (1992) Gene 112, 157-162.
[16] J. SAMBROOK, E.F. FRITSCH and T. MANIATIS, Molecular cloning, A laboratory manual, second edition, Cold spring Harbor Laboratory Press, 1989.
[17] Guan, K. L., and Dixon, J. E. (1991) Anal Biochem 192, 262-267.
[18] Hakes, D. J., and Dixon, J. E. (1992) Anal Biochem 202, 293-298.
[19] Laemmli, U. K. (1970) Nature 227, 680-685.
[20] MacKenzie, K. R., and Engelman, D. M. (1998) Proc Natl Acad Sci U S A 95, 3583-3590.
[21] Dubuisson, J., Penin, F., and Moradpour, D. (2002) Trends Cell Biol 12, 517-523.
[22] Ferlenghi, I., Clarke, M., Ruttan, T., Allison, S. L., Schalich, J., Heinz, F. X., Harrison, S. C., Rey, F. A., and Fuller, S. D. (2001) Mol Cell 7, 593-602.
[23] Ferlenghi, I., Clarke, M., Ruttan, T., Allison, S. L., Schalich, J., Heinz, F. X., Harrison, S. C., Rey, F. A., and Fuller, S. D. (2001) Mol Cell 7, 593-602.
[24] Lorenz, I. C., Allison, S. L., Heinz, F. X., and Helenius, A. (2002) J Virol 76, 5480-5491.
[25] Stiasny, K., Allison, S. L., Schalich, J., and Heinz, F. X. (2002) J Virol 76, 3784-3790.
[26] Sharp, P. M., Cowe, E., Higgins, D. G., Shields, D. C., Wolfe, K. H. and Wright, F. (1988) Nucleic Acids Res. 16, 8207-8211.
[27] Mullis K.B., and Faloona F.A. (1987) Methods Enzymol 155, 335-350.
[28] Bartosch, B., Dubuisson, J., Cosset, François-Loïc. (2003) J. Exp. Med. 197, 633-642.

## Revendications

1. Vecteur de co-expression d'au moins deux domaines membranaires de protéines d'enveloppe d'un virus qui interagissent dans leur conformation fonctionnelle dans l'enveloppe du virus, ledit **vecteur** étant **caractérisé en ce qu'**il comprend :
- au moins une région de réplication et de maintien dudit vecteur dans la cellule hôte ;
- une première région constituée successivement, dans le sens de traduction du vecteur, d'un premier promoteur suivit d'une première séquence codant pour une première protéine chimère,
la première séquence codant pour la première protéine chimère étant constituée dans ledit sens de traduction du vecteur, d'une première séquence codant pour une première protéine soluble, d'une séquence codant pour un dipeptide Asp-Pro et d'une séquence codant pour l'un desdits au moins deux domaines membranaires ; et
- une deuxième région constituée successivement, dans ledit sens de traduction du vecteur, d'un deuxième promoteur suivit d'une deuxième séquence codant pour une deuxième protéine chimère,
la deuxième séquence codant pour la deuxième protéine chimère étant constituée dans ledit sens de traduction du vecteur, d'une deuxième séquence codant pour une deuxième protéine soluble, d'une séquence codant pour un dipeptide Asp-Pro et d'une séquence codant pour l'autre desdits au moins deux domaines membranaires.

2. Vecteur selon la revendication 1, dans lequel le virus est choisi parmi le virus de l'hépatite C, le virus du sida, un virus pathogène pour l'homme, un virus pathogène pour un mammifère.

3. Vecteur selon la revendication 1, dans lequel la première et la deuxième régions se succèdent.

4. Vecteur selon la revendication 1, dans lequel les première et deuxième protéines solubles sont identiques ou différentes et choisie parmi la glutathion S-transférase et la thiorédoxine.

5. Vecteur selon la revendication 1, dans lequel la séquence nucléotidique codant pour le dipeptide Asp-Pro est gacccg.

6. Vecteur selon la revendication 1, dans lequel l'un des deux domaines membranaires a une séquence peptidique choisie parmi les séquences peptidiques SEQ IDn°2 ; SEQ IDn°10 ; SEQ IDn°12 ; SEQ IDn°14 de la liste de séquences annexée, et l'autre des deux domaines a une séquence peptidique choisie parmi les séquences peptidiques SEQ IDn°16 ; SEQ IDn°22 de la liste de séquences annexée.

7. Vecteur selon la revendication 6, dans lequel lorsque l'un des deux domaines est SEQ IDn°2, l'autre domaine est différent de SEQ IDn°16 et inversement.

8. Vecteur selon l'une quelconque des revendications précédentes, ledit vecteur étant obtenu à partir du plasmide pEGEXKT de séquence SEQ IDn°23 ou du plasmide pET32a+ de séquence SEQ IDn°35

9. Vecteur selon la revendication 1, dans lequel la séquence codant pour l'un desdits au moins deux domaines membranaires a une séquence nucléotidique choisie parmi les séquences SEQ IDn°1, SEQ IDn°9, SEQ IDn°11 et SEQ IDn°13 de la liste de séquences annexée, et l'autre séquence codant pour l'autre desdits au moins deux domaines membranaires a une séquence nucléotidique choisie parmi les séquences SEQ IDn°15 et SEQ IDn°17 de la liste de séquences annexée.

10. Vecteur selon la revendication 9, dans lequel lorsque l'un des deux domaines est SEQ IDn°1, l'autre domaine est différent de SEQ IDn°15 et inversement.

11. Vecteur d'expression selon la revendication 1, dans lequel la première protéine chimère est une protéine ayant une séquence choisie parmi les séquences SEQ IDn°28, SEQ IDn°43, SEQ IDn°46, SEQ IDn°49 et SEQ IDn°52, et dans lequel la deuxième protéine chimère est une protéine ayant une séquence choisie parmi les séquences SEQ IDn°31, SEQ IDn°34, SEQ IDn°55 et SEQ IDn°58.

12. Vecteur selon la revendication 11, dans lequel lorsque la première protéine chimère a la séquence SEQ IDn°28, l'autre protéine chimère est différente de SEQ IDn°31 et inversement.

13. Vecteur d'expression selon la revendication 1, dans lequel le vecteur a une séquence nucléotidique choisie dans le groupe constitué par les séquences SEQ IDn°61 ; SEQ IDn°62 ; SEQ IDn°70 ; SEQ IDn°71 ; SEQ IDn°72 ; SEQ IDn°73 ; SEQ IDn°74 et SEQ IDn°75 de la liste de séquences annexée.

14. Cellule procaryote transformée par un vecteur selon l'une quelconque des revendications 1 à 13.

15. Cellule procaryote selon la revendication 14, dans laquelle la cellule procaryote est *E. coli.*

16. Procédé de fabrication par recombinaison génétique de formes hétéro-oligomériques ou d'un mélange d'au moins deux domaines membranaires de protéine d'enveloppe d'un virus qui interagissent dans leur conformation fonctionnelle dans l'enveloppe du virus comprenant les étapes suivantes :
- transformer une cellule hôte avec un vecteur selon la revendication 1,
- cultiver la cellule hôte transformée dans des conditions de culture telles qu'elle fabrique les formes hétéro-oligomériques ou le mélange des, au moins deux, domaines membranaires à partir dudit vecteur, et
- isoler lesdits hétéro-oligomères ou ledit mélange de domaines membranaires.

17. Procédé selon la revendication 16, dans lequel la cellule hôte est *E*. *coli.*

18. Procédé selon la revendication 16, dans lequel le vecteur est un vecteur selon la revendication 6 ou 7.

19. Procédé selon la revendication 16, dans lequel le vecteur est un vecteur selon la revendication 8.

20. Procédé selon la revendication 16, dans lequel le vecteur est un vecteur selon la revendication 9 ou 10.

21. Procédé selon la revendication 16, dans lequel le vecteur est un vecteur selon la revendication 11 ou 12.

22. Procédé selon la revendication 16, dans lequel le vecteur est un vecteur selon la revendication 13.

23. Protéine de séquence peptidique choisie parmi SEQ IDn°14 et **SEQ** IDn°22 de la liste de séquences annexée.

24. Hétéro-oligomère ou mélange constitué d'au moins une première et une deuxième protéine,
- la première protéine ayant une séquence choisie parmi SEQ IDn°2, SEQ IDn°10, SEQ IDn°12, SEQ IDn°14, SEQ IDn°65, SEQ IDn°67 et SEQ IDn°69 de la liste de séquence annexée, et la deuxième protéine choisie parmi SEQ IDn°22, SEQ IDn°34 et SEQ IDn°58 ;
- ou la première protéine ayant une séquence choisie parmi SEQ IDn°14, SEQ IDn°65, SEQ IDn°67 et SEQ IDn°69 de la liste de séquence annexée, et la deuxième protéine ayant la séquence SEQ IDn°16

25. Utilisation d'une protéine selon la revendication 23 ou d'un hétéro-oligomère ou mélange selon la revendication 24 pour la fabrication d'un médicament.

26. Utilisation d'une protéine selon la revendication 23 ou d'un hétéro-oligomère ou mélange selon la revendication 24 pour la fabrication d'un médicament destiné au traitement ou à la prophylaxie de l'hépatite C.

## Claims

1. Vector for the coexpression of at least two membrane domains of the envelope proteins of a virus that interact in their functional conformation in the virus envelope, said vector being **characterized in that** it comprises:
- at least one region for replication and for maintenance of said vector in the host cell;
- a first region consisting successively, in the direction of translation of the vector, of a first promoter followed by a first sequence encoding a first chimeric protein,
the first sequence encoding the first chimeric protein consisting, in said direction of translation of the vector, of a first sequence encoding a first soluble protein, of a sequence encoding an Asp-Pro dipeptide and of a sequence encoding one of said at least two membrane domains; and
- a second region consisting successively, in said direction of translation of the vector, of a second promoter followed by a second sequence encoding a second chimeric protein,
the second sequence encoding the second chimeric protein consisting, in said direction of translation of the vector, of a second sequence encoding a second soluble protein, of a sequence encoding an Asp-Pro dipeptide and of a sequence encoding the other of said at least two membrane domains.

2. Vector according to claim 1, in which the virus is chosen from the hepatitis C virus, the AIDS virus, a virus that is pathogenic for humans and a virus that is pathogenic for a mammal.

3. Vector according to claim 1, in which the first and the second regions follow one another.

4. Vector according to claim 1, in which the first and second soluble proteins are identical or different and are chosen from glutathione S-transferase and thioredoxin.

5. Vector according to claim 1, in which the nucleotide sequence encoding the Asp-Pro dipeptide is gacccg.

6. Vector according to claim 1, in which one of the two membrane domains has a peptide sequence chosen from the peptide sequences SEQ ID NO. 2; SEQ ID NO. 10; SEQ ID NO. 12 and SEQ ID NO. 14 of the attached sequence listing, and the other of the two domains has a peptide sequence chosen from the peptide sequences SEQ ID NO. 16; SEQ ID NO. 22 of the attached sequence listing.

7. Vector according to claim 6, in which, when one of the two domains is SEQ ID NO. 2, the other domain is different from SEQ ID NO. 16, and vice versa.

8. Vector according to any one of preceding claims, said vector being obtained from the plasmid pEGEXKT of sequence SEQ ID NO. 23 or the plasmid pET32a+ of sequence SEQ ID NO. 35.

9. Vector according to claim 1, in which the sequence encoding one of said at least two membrane domains has a nucleotide sequence chosen from the sequences SEQ ID NO: 1, SEQ ID NO: 9, SEQ ID NO: 11 and SEQ ID NO: 13 of the attached sequence listing, and the other sequence encoding the other of said at least two membrane domains having a nucleotide sequence chosen from the sequences SEQ ID NO: 15 and SEQ ID NO: 17 of the attached sequence listing.

10. Vector according to claim 9, in which, when one of the two domains is SEQ ID NO: 1, the other domain is different from SEQ ID NO: 15, and vice versa.

11. Expression vector according to claim 1, in which the first chimeric protein is a protein having a sequence chosen from the sequences SEQ ID NO: 28, SEQ ID NO: 43, SEQ ID NO: 46, SEQ ID NO: 49 and SEQ ID NO: 52, and in which the second chimeric protein is a protein having a sequence chosen from the sequences SEQ ID NO: 31, SEQ ID NO: 34, SEQ ID NO: 55 and SEQ ID NO: 58.

12. Vector according to claim 11, in which, when the first chimeric protein has the sequence SEQ ID NO: 28, the other chimeric protein is different from SEQ ID NO: 31, and vice versa.

13. Expression vector according to claim 1, in which the vector has a nucleotide sequence chosen from the group consisting of the sequences SEQ ID NO: 61; SEQ ID NO: 62; SEQ ID NO: 70; SEQ ID NO: 71; SEQ ID NO: 72; SEQ ID NO: 73; SEQ ID NO: 74 and SEQ ID NO: 75 of the attached sequence listing.

14. Prokaryotic cell transformed with a vector according to any one of claim 1 to 13.

15. Prokaryotic cell according to claim 14, in which the prokaryotic cell is E. *coli.*

16. Method for producing, by genetic recombination, hetero-oligomeric forms or a mixture of at least two membrane domains of the envelope proteins of a virus that interact in their functional conformation in the virus envelope, comprising the following steps:
- transforming a host cell with a vector according to claim 1,
- culturing the transformed host cell under culture conditions such that it produces the hetero-oligomeric forms or the mixture of the at least two membrane domains from said vector, and
- isolating said hetero-oligomers or said mixture of membrane domains.

17. Method according to claim 16, in which the host cell is E. *coli.*

18. Method according to claim 16, in which the vector is a vector according to claim 6 or 7.

19. Method according to claim 16, in which the vector is a vector according to claim 8.

20. Method according to claim 16, in which the vector is a vector according to claim 9 or 10.

21. Method according to claim 16, in which the vector is a vector according to claim 11 or 12.

22. Method according to claim 16, in which the vector is a vector according to claim 13.

23. Protein having a peptide sequence chosen from SEQ ID NO: 14 and SEQ ID NO: 22 of the attached sequence listing.

24. Hetero-oligomer or mixture consisting of at least a first and a second protein,
- the first protein having a sequence chosen from SEQ ID NO: 2, SEQ ID NO: 10, SEQ ID NO: 12, SEQ ID NO: 14, SEQ ID NO: 65, SEQ ID NO: 67 and SEQ ID NO: 69 of the attached sequence listing, and the second protein chosen from SEQ ID NO: 22, SEQ ID NO: 34 and SEQ ID NO: 58 of the attached sequence listing;
- or the first protein having a sequence chosen from SEQ ID NO: 14, SEQ ID NO: 65, SEQ ID NO: 67 and SEQ ID NO: 69 of the attached sequence listing, and the second protein having the sequence SEQ ID NO: 16.

25. Use of a protein according to claim 23 or of a hetero-oligomer or mixture according to claim 24, for the production of a medicinal product.

26. Use of a protein according to Claim 23 or of a hetero-oligomer or mixture according to Claim 24, for the production of a medicinal product for use in the treatment or in the prophylaxis of hepatitis C.

## Patentansprüche

1. Vektor zur Co-Expression wenigstens zweier Membrandomänen von Hüllproteinen eines Virus, die in ihrer funktionellen Konformation in der Virushülle interagieren, wobei der Vektor **dadurch gekennzeichnet ist, dass** er Folgendes umfasst:
- wenigstens eine Region der Replikation und des Erhalts des Vektors in der WirtsZelle,
- eine erste Region, die sich nacheinander in Richtung der Translation des Vektors aus einem ersten Promotor, gefolgt von einer für ein erstes chimäres Protein kodierenden ersten Sequenz zusammensetzt,
wobei sich die für das erste chimäre Protein kodierende erste Sequenz in Richtung der Translation des Vektors aus einer für ein erstes lösliches Protein kodierenden ersten Sequenz, einer für ein Dipeptid Asp-Pro kodierenden Sequenz und einer für eine der wenigstens zwei Membrandomänen kodierenden Sequenz zusammensetzt, und
- eine zweite Region, die sich nacheinander in Richtung der Translation des Vektors aus einem zweiten Promotor, gefolgt von einer für ein zweites chimäres Protein kodierenden zweiten Sequenz zusammensetzt,
wobei sich die für das zweite chimäre Protein kodierende zweite Sequenz in Richtung der Translation des Vektors aus einer für ein zweites lösliches Protein kodierenden zweiten Sequenz, einer für ein Dipeptid Asp-Pro kodierenden Sequenz und einer für die andere der wenigstens zwei Membrandomänen kodierenden Sequenz zusammensetzt.

2. Vektor gemäß Anspruch 1, wobei das Virus aus dem Hepatitis-C-Virus, dem AIDS-Virus, einem für den Menschen pathogenen Virus und einem für ein Säugetier pathogenen Virus ausgewählt ist.

3. Vektor gemäß Anspruch 1, wobei die erste und die zweite Region aufeinanderfolgen.

4. Vektor gemäß Anspruch 1, wobei die ersten und zweiten löslichen Proteine gleich oder verschieden sind und aus Glutathion-S-Transferase und Thioredoxin ausgewählt sind.

5. Vektor gemäß Anspruch 1, wobei die für das Dipeptid Asp-Pro kodierende Nucleotidsequenz gacccg ist.

6. Vektor gemäß Anspruch 1, wobei die eine der beiden Membrandomänen eine Peptidsequenz aufweist, die aus den Peptidsequenzen SEQ ID NO 2, SEQ ID NO 10, SEQ ID NO 12 und SEQ ID NO 14 der angefügten Sequenzliste ausgewählt ist, und die andere der beiden Domänen eine Peptidsequenz aufweist, die aus den Peptidsequenzen SEQ ID NO 16 und SEQ ID NO 22 der angefügten Sequenzliste ausgewählt ist.

7. Vektor gemäß Anspruch 6, wobei, wenn die eine der beiden Domänen SEQ ID NO 2 ist, die andere Domäne von SEQ ID NO 16 verschieden ist, und umgekehrt.

8. Vektor gemäß einem der vorangehenden Ansprüche, wobei der Vektor aus dem Plasmid pEGEXKT mit der Sequenz SEQ ID NO 23 oder dem Plasmid pET32a+ mit der Sequenz SEQ ID NO 35 erhalten wurde.

9. Vektor gemäß Anspruch 1, wobei die für eine der wenigstens zwei Membrandomänen kodierende Sequenz eine Nucleotidsequenz aufweist, die aus den Sequenzen SEQ ID NO 1, SEQ ID NO 9, SEQ ID NO 11 und SEQ ID NO 13 der angefügten Sequenzliste ausgewählt ist, und die für die andere der wenigstens zwei Membrandomänen kodierende Sequenz eine Nucleotidsequenz aufweist, die aus den Sequenzen SEQ ID NO 15 und SEQ ID NO 17 der angefügten Sequenzliste ausgewählt ist.

10. Vektor gemäß Anspruch 9, wobei, wenn die eine der beiden Domänen SEQ ID NO 1 ist, die andere Domäne von SEQ ID NO 15 verschieden ist, und umgekehrt.

11. Expressionsvektor gemäß Anspruch 1, wobei das erste chimäre Protein ein Protein ist, das eine aus den Sequenzen SEQ ID NO 28, SEQ ID NO 43, SEQ ID NO 46, SEQ ID NO 49 und SEQ ID NO 52 ausgewählte Sequenz aufweist, und wobei das zweite chimäre Protein ein Protein ist, das eine aus den Sequenzen SEQ ID NO 31, SEQ ID NO 34, SEQ ID NO 55 und SEQ ID NO 58 ausgewählte Sequenz aufweist.

12. Vektor gemäß Anspruch 11, wobei, wenn das erste chimäre Protein die SEQ ID NO 28 aufweist, das andere chimäre Protein von SEQ ID NO 31 verschieden ist, und umgekehrt.

13. Expressionsvektor gemäß Anspruch 1, wobei der Vektor eine Nucleotidsequenz aufweist, die aus der Gruppe ausgewählt ist, die durch die Sequenzen SEQ ID NO 61, SEQ ID NO 62, SEQ ID NO 70, SEQ ID NO 71, SEQ ID NO 72, SEQ ID NO 73, SEQ ID NO 74 und SEQ ID NO 75 der angefügten Sequenzliste dargestellt wird.

14. Prokaryontenzelle, die durch einen Vektor gemäß einem der Ansprüche 1 bis 13 transformiert wurde.

15. Prokaryontenzelle gemäß Anspruch 14, wobei die Prokaryontenzelle *E. coli* ist.

16. Verfahren zur Herstellung durch genetische Rekombination heterooligomerer Formen oder eines Gemischs wenigstens zweier Membrandomänen eines Hüllproteins eines Virus, die in ihrer funktionellen Konformation in der Virushülle interagieren, das die folgenden Schritte umfasst:
- Transformieren einer Wirtszelle mit einem Vektor gemäß Anspruch 1,
- Kultivieren der transformierten Wirtszelle unter Kulturbedingungen, bei denen sie die heterooligomeren Formen oder das Gemisch der wenigstens zwei Membrandomänen aus dem Vektor erzeugt, und
- Isolieren der Heterooligomeren oder des Gemischs der Membrandomänen.

17. Verfahren gemäß Anspruch 16, wobei die Wirtszelle *E*. *coli* ist.

18. Verfahren gemäß Anspruch 16, wobei der Vektor ein Vektor gemäß Anspruch 6 oder 7 ist.

19. Verfahren gemäß Anspruch 16, wobei der Vektor ein Vektor gemäß Anspruch 8 ist.

20. Verfahren gemäß Anspruch 16, wobei der Vektor ein Vektor gemäß Anspruch 9 oder 10 ist.

21. Verfahren gemäß Anspruch 16, wobei der Vektor ein Vektor gemäß Anspruch 11 oder 12 ist.

22. Verfahren gemäß Anspruch 16, wobei der Vektor ein Vektor gemäß Anspruch 13 ist.

23. Protein mit einer Peptidsequenz, die aus SEQ ID NO 14 und SEQ ID NO 22 der angefügten Sequenzliste ausgewählt ist.

24. Heterooligomer oder Gemisch, das sich aus wenigstens einem ersten und einem zweiten Protein zusammensetzt, wobei
- das erste Protein eine Sequenz aufweist, die aus SEQ ID NO 2, SEQ ID NO 10, SEQ ID NO 12, SEQ ID NO 14, SEQ ID NO 65, SEQ ID NO 67 und SEQ ID NO 69 der angefügten Sequenzliste ausgewählt ist, und das zweite Protein aus SEQ ID NO 22, SEQ ID NO 34 und SEQ ID NO 58 ausgewählt ist,
- oder das erste Protein eine Sequenz aufweist, die aus SEQ ID NO14, SEQ ID NO 65, SEQ ID NO 67 und SEQ ID NO 69 der angefügten Sequenzliste ausgewählt ist, und das zweite Protein die Sequenz SEQ ID NO 16 aufweist.

25. Verwendung eines Proteins gemäß Anspruch 23 oder eines Heterooligomers oder Gemischs gemäß Anspruch 24 zur Herstellung eines Arzneimittels.

26. Verwendung eines Proteins gemäß Anspruch 23 oder eines Heterooligomers oder Gemischs gemäß Anspruch 24 zur Herstellung eines Arzneimittels, das zur Behandlung oder zur Prophylaxe von Hepatitis C bestimmt ist.
